(19) Europäisches Patentamt / European Patent Office / Office européen des brevets

(11) **EP 2 235 539 B1**

(12) **FASCICULE DE BREVET EUROPEEN**

(45) Date de publication et mention de la délivrance du brevet: **31.08.2016 Bulletin 2016/35**

(21) Numéro de dépôt: **09721161.9**

(22) Date de dépôt: **12.01.2009**

(51) Int Cl.:
*G01N 33/68* *(2006.01)*    *C07K 16/18* *(2006.01)*

(86) Numéro de dépôt international:
**PCT/FR2009/000036**

(87) Numéro de publication internationale:
**WO 2009/112670 (17.09.2009 Gazette 2009/38)**

(54) **UTILISATION D'UNE PROTEINE OU D'UNE SEQUENCE PEPTIDIQUE NITREE POUR LA MISE EN ŒUVRE D'UNE METHODE DE DIAGNOSTIC**

VERWENDUNG EINER SEQUENZ VON PROTEINEN ODER NITRIERTEN PEPTIDEN ZUR IMPLEMENTIERUNG EINES DIAGNOSEVERFAHRENS

USE OF A PROTEIN OR NITRATED PEPTIDE SEQUENCE FOR IMPLEMENTING A DIAGNOSIS METHOD

(84) Etats contractants désignés:
**AT BE BG CH CY CZ DE DK EE ES FI FR GB GR HR HU IE IS IT LI LT LU LV MC MK MT NL NO PL PT RO SE SI SK TR**

(30) Priorité: **10.01.2008 FR 0800142**

(43) Date de publication de la demande:
**06.10.2010 Bulletin 2010/40**

(73) Titulaire: **Université Joseph Fourier 38041 Grenoble Cedex 09 (FR)**

(72) Inventeur: **BOTTARI, Serge F-38330 Biviers (FR)**

(74) Mandataire: **Grosset-Fournier, Chantal Catherine Grosset-Fournier & Demachy 54, rue Saint-Lazare 75009 Paris (FR)**

(56) Documents cités:
**WO-A-98/29452       WO-A-03/076946 US-A1- 2005 244 905**

• **CAVEDON ET AL.: "Nitrated albumin as a potential marker of nitrating stress in newborns : preliminary results" J MAT-FET NEONAT MED, vol. 19, no. suppl1, 2006, page 35, XP009104810**

• **JIAO KAISHENG ET AL: "Site-selective nitration of tyrosine in human serum albumin by peroxynitrite" ANALYTICAL BIOCHEMISTRY, vol. 293, no. 1, 1 juin 2001 (2001-06-01), pages 43-52, XP002493208 ISSN: 0003-2697**

• **YAO ZU YE ET AL: "ANTIBODIES THAT RECOGNIZE NITROTYROSINE" METHODS IN ENZYMOLOGY, ACADEMIC PRESS INC, SAN DIEGO, CA, US, vol. 269, 1 janvier 1996 (1996-01-01), pages 201-209, XP002041254 ISSN: 0076-6879**

• **BOTTARI S ET AL: "Nitration pattern of plasma proteins in newborns suffering from perpartal asphyxia : preliminary results." J.MAT-FET NEONAT MED, vol. 19, no. suppl.1, 2006, pages 34-35, XP009104811**

• **PRIEELS J P ET AL: "Nitration of tyrosyl residues in human alpha-lactalbumin. Effect on lactose synthase specifier activity." EUROPEAN JOURNAL OF BIOCHEMISTRY / FEBS 15 DEC 1975, vol. 60, no. 2, 15 décembre 1975 (1975-12-15), pages 533-539, XP9129005 ISSN: 0014-2956**

• **LILOVA A ET AL: "Topography of all tyrosine residues in subtilisin DY." BIOLOGICAL CHEMISTRY HOPPE-SEYLER SEP 1986, vol. 367, no. 9, septembre 1986 (1986-09), pages 861-870, XP9129012 ISSN: 0177-3593**

**(Cont. page suivante)**

- **YAMADA H ET AL: "Reaction of hen egg-white lysozyme with tetranitromethane: a new side reaction, oxidative bond cleavage at glycine 104, and sequential nitration of three tyrosine residues." JOURNAL OF BIOCHEMISTRY SEP 1990, vol. 108, no. 3, septembre 1990 (1990-09), pages 432-440, XP9129041 ISSN: 0021-924X**

Remarques:
Le dossier contient des informations techniques présentées postérieurement au dépôt de la demande et ne figurant pas dans le présent fascicule.

## Description

La présente invention a pour objet l'utilisation d'une protéine ou d'une séquence peptidique nitrée pour la mise en oeuvre d'une méthode de diagnostic.

La présente invention a plus particulièrement pour objet l'utilisation d'une protéine ou d'une séquence peptidique nitrée pour la mise en oeuvre du diagnostic de l'état de gravité et d'évolutivité de pathologies associées, entraînant ou dues au stress nitrant.

Le stress oxydant (ou stress oxydatif) est un type d'agression des constituants de la cellule dû aux espèces réactives oxygénées (ROS, Reactive Oxygen Species) et aux espèces réactives de l'azote (RNS, Reactive Nitrogen Species) oxydantes. Ces espèces sont, par définition, des radicaux libres. Par assimilation, le peroxyde d'hydrogène ($H_2O_2$) est considéré comme une ROS car, en présence de fer (sous forme ionique), il se dismute en deux radicaux hydroxyle (OH) (Réaction de Haber-Weiss).

La production de ROS et RNS est normale pour tous les organismes vivant en aérobie et ne constitue pas en soit une situation de stress oxydant parce que la cellule dispose d'un système complexe de détoxification contre les ROS comprenant des enzymes (superoxyde dismutase, catalase, glutathion péroxydase.....) et des petites molécules (vitamine E, vitamine C, glutathion,...). En situation physiologique, l'anion superoxyde ($O_2^-$) est produit essentiellement par les NADPH oxydases (NOX) et le monoxyde d'azote (NO) par la famille des NO synthases.

Le stress oxydant devient une situation pathologique dès que le système de protection est submergé par les ROS et RNS. Ceci peut être par exemple dû à :

- l'introduction dans la cellule de radicaux libres ou d'espèces réactives oxygénées (polluants chimiques pénétrant l'organisme via le système respiratoire, l'alimentation ou les muqueuses)
- une surproduction de ROS et RNS induite par l'hypoxie ou des processus de type ischémie-reperfusion qui sont à l'origine d'une partie des rejets des greffes ou à la présence de certains composés chimiques prooxydants.
- un dysfonctionnement de la chaîne respiratoire mitochondriale suite par exemple à une hypoxie, une hypoglycémie ou à des xénobiotiques agissant sur certains de ses complexes.
- une activation de la xanthine oxydase
- une augmentation de l'expression ou de l'activité de la NOS II suite par exemple au déclenchement d'une réaction inflammatoire.
- un défaut du système de protection, par exemple une mutation ou des xénobiotiques inactivant une des enzymes du système de protection ou une carence en une des vitamines antioxydantes (vitamines C et E).
- l'introduction dans la cellule ou dans un organe de molécules hautement réactives, par exemple nanoparticules (très petite et à surface spécifique très développée). Si ces nanoparticules sont nombreuses, les macrophages n'arrivent plus à les traiter et peuvent libérer leurs oxydants dans l'organisme en provoquant une réaction inflammatoire exacerbée.

Le stress oxydant a des conséquences fonctionnelles très différentes selon son intensité. Des travaux récents indiquent que le rapport ions superoxyde sur monoxyde d'azote ($O_2^-$/NO) est déterminant. En effet, tant que ce rapport $\leq 1$, l'$O_2^-$ réagit préférentiellement avec le NO, permettant l'apparition des espèces radicalaires azotées (RNS), nitrosonium ($NO^+$) et peroxynitrite ($ONOO^-$), qui induisent des modifications post-traductionnelles. Ces RNS induisent respectivement la nitrosation (R-Cys-SH → R-Cys-SNO) et la nitration (R-Tyr → R-Tyr-$NO_2$ des protéines. Contrairement aux modifications induites par les espèces ROS, celles induites par les espèces RNS sont réversibles.

Lorsque le rapport $O_2^-$/NO > 1, les ions $O_2^{·-}$, puis les radicaux $OH^{·-}$ « libres » induisent l'oxydation irréversible des protéines, lipides et acides nucléiques, qui peut être mesurée à l'aide de nombreux marqueurs plasmatiques.

Ces modifications sont par ailleurs le reflet d'un des mécanismes de défense cellulaire vis à vis du stress oxydant : la production de NO. On peut donc postuler qu'une fois la capacité cellulaire à « absorber » le $NO^+$ et l'$ONOO^-$ produits, ceux-ci, étant diffusibles au travers de la membrane plasmique, pourront respectivement nitroser et nitrer des protéines extracellulaires parmi lesquelles les protéines plasmatiques.

Les stress oxydant et nitrant sont des facteurs d'inflammation et de mutagenèse. Ils sont aussi considérés comme une des principales causes de cancer et joueraient un rôle dans les maladies neurodégénératives (Parkinson, Alzheimer, sclérose en plaques, SLA), tout comme dans plusieurs pathologies plus courantes telles que le diabète sucré de types 1 et 2, les maladies cardio-vasculaires, les accidents vasculaires cérébraux, l'arthrite rhumatoïde ou la cataracte.

A ce jour, il n'existe aucune méthode permettant de mettre en évidence le stress nitrant en dosant un marqueur circulant de façon spécifique et quantitative.

Khan et collaborateurs (Khan et al. Biochem J, 1998, 330, 795-801) divulguent une méthode permettant de doser les protéines nitrées sur les résidus tyrosines (test ELISA) en utilisant des anticorps reconnaissant les tyrosines nitrées de toutes les protéines.

Il est clairement stipulé que la méthode décrite par Khan et al. permet un dosage qualitatif des protéines nitrées, mais ceci ne permet pas de mesure précise et fiable des taux de nitration des protéines circulantes. En effet, Khan et al. divulguent l'utilisation d'anticorps capables de reconnaître toutes les protéines nitrées et non pas des protéines spécifiques.

L'art antérieur décrit des méthodes de détection de la quantité de NO associé aux protéines.

En particulier la demande WO/1998/029452 décrit des anticorps reconnaissant les tyrosines nitrées, en particulier les tyrosines nitrées des protéines transporteuses de NO, pour leur utilisation en tant qu'agents thérapeutiques pour soigner des pathologies associées à la nitrosylation et la nitration des protéines.

Egalement, la demande US 2005/244905 décrit une méthode de diagnostic du risque de maladies coronariennes chez les patients par la détection des taux de nitration sur tyrosine du fibrinogène. Cette méthode utilise un couple d'anticorps permettant ladite détection du fibrinogène nitré : un anticorps de capture détectant toutes les tyrosines nitrées, et un anticorps de détection spécifique du fibrinogène, nitré ou non.

Cavedon et al. décrit l'albumine nitrée comme un marqueur potentiel de stress nitrant chez les nouveau-nés (Cavedon et al., J. Mat-Fet Neonat Med, vol.19, p. 35, 2006).

D'autres exemples de méthode de détection de protéines nitrées sur tyrosines sont également décrits dans l'art antérieur.

Par exemple, la demande internationale WO 03/076946 décrit l'utilisation d'un « partenaire » immunologique capable de détecter un épitope spécifique contenant un acide aminé aromatique nitré. Plus particulièrement, WO 03/076946 décrit l'utilisation d'un anticorps spécifique reconnaissant un épitope du collagène de type II dans lequel une tyrosine est nitrée. L'anticorps décrit dans ce document est utilisé à des fins de diagnostic pour évaluer le taux d'oxydation des protéines, et plus particulièrement du collagène de type 2.2 dans le cadre des pathologies associées à un stress nitrant. Cependant à aucun moment il n'est décrit dans WO 03/076946 qu'il est possible de détecter l'état d'avancement ou d'évolutivité d'une pathologie associée au stress nitrant.

Bien que ces documents divulguent des anticorps reconnaissant des protéines nitrées il n'est rien dit concernant la production d'anticorps spécifiques dirigés contre des résidus nitrés, particulièrement les résidus tyrosine $Y^{138}$ et $Y^{411}$ de l'albumine, ou tout autre résidu de position définie dans des protéines circulantes.

La nitration de l'albumine a été décrite dans l'art antérieur. En particulier, Jiao et al. (Analytical. Biochem, 2001, 293, 43-52) divulguent les résidus nitrés de l'Albumine. Jiao et al. décrivent la nitration des résidus $Y^{138}$ et $Y^{411}$ de l'albumine, après *nitration in vitro* de l'albumine par du péroxynitrite, et identification des sites de nitration par spectrométrie de masse.

Malan, P. G., et al. (1970) Biochemistry 9(16), 3205-3214 et Sokolovsky, et al. (1966) Biochemistry 5(11), 3582-3589 décrivent quant à eux des méthodes de nitration *in vitro* des protéines utilisant du tétranitrométhane en tant qu'agent nitrant.

Un des aspects de l'invention est de fournir une méthode de diagnostic de l'état de gravité et d'évolutivité de pathologies dues ou associées au stress nitrant, ou provoquant.

L'un des autres aspects de l'invention est de fournir une méthode de dosage quantitatif *in vitro* des taux de nitration de protéines physiologiques.

Un autre aspect de l'invention est de permettre la détermination de la position des tyrosines nitrées dans les protéines physiologiques, au cours d'un stress nitrant.

La présente invention est illustrée par l'utilisation du dosage quantitatif, notamment *in vitro,* dans un échantillon biologique, du taux de nitration de résidus tyrosine d'une protéine ou d'une séquence peptidique physiologique déterminée nitrée, pour la mise en oeuvre d'une méthode de *diagnostic in vitro* de l'état de gravité et d'évolutivité d'une pathologie chronique ou aiguë associée au stress nitrant.

La présente invention concerne l'utilisation du dosage quantitatif, *in vitro,* dans un échantillon biologique, du taux de nitration de résidus tyrosine d'une protéine ou d'une séquence peptidique physiologique déterminée nitrée, pour la mise en oeuvre d'une méthode de diagnostic *in vitro* de l'état de gravité et d'évolutivité d'une pathologie chronique ou aiguë associée au stress nitrant,

ladite protéine ou séquence peptidique physiologique déterminée nitrée étant l'albumine nitrée,

ledit dosage quantitatif étant effectué à l'aide d'un anticorps spécifique, en particulier un anticorps monoclonal, reconnaissant spécifiquement

- soit le résidu tyrosine $Y^{138}$ nitrée de l'albumine,
- **soit** le résidu tyrosine $Y^{411}$ nitrée de l'albumine,

ladite pathologie chronique ou aiguë associée au stress nitrant appartenant au groupe suivant : les maladies inflammatoires, les maladies infectieuses, les maladies ischémiques et les maladies cardiovasculaires.

La présente invention repose sur la constatation inattendue de l'existence d'une corrélation entre la valeur du taux de nitration physiologique de protéines et l'état de gravité et d'évolutivité d'une pathologie associée au stress nitrant.

Dans l'invention, on entend par « dosage quantitatif du taux de nitration» l'action qui consiste à déterminer de manière précise la quantité de protéines déterminées nitrées. La quantification du taux de nitration de la protéine déterminée nitrée est effectuée par l'établissement du rapport entre la quantité de protéine déterminée nitrée et la quantité totale de ladite protéine déterminée, nitrée et non nitrée. Cette valeur est donc comprise entre 0 et 1.

Dans l'invention, il est appelé « protéine ou séquence peptidique physiologique déterminée », toute protéine, peptide, fragment de protéines ou de peptides, ou tout enchaînement d'au moins 2 acides aminés, connu dont la synthèse à lieu au sein d'un organisme vivant, généralement sans intervention extérieure humaine. Il est également entendu que les termes « protéine ou séquence peptidique physiologique déterminée » correspondent à « une protéine physiologique déterminée » ou à « une séquence peptidique physiologique déterminée ». Selon un mode de réalisation de l'invention, les protéines particulièrement concernées sont des protéines circulantes, retrouvées dans le sang, le liquide céphalorachidien ou les urines. Les autres protéines ne sont pas exclues de l'invention.

On entend également par protéine, dans ce qui suit, tout enchainement d'acides aminés présentant des propriétés antigéniques, et donc capable de permettre la réaction du système immunitaire et ainsi de générer des anticorps. Ces anticorps sont alors eux même capables de reconnaitre la dite protéine qui a permis leur synthèse.

Par « nitré », on définit dans l'invention la présence d'un groupement -$NO_2$ lié de façon covalente à une protéine ou une séquence peptidique. Aussi, par protéine nitrée on définit une protéine dont une ou plusieurs tyrosines possèdent sur leur groupement phénol un groupement -$NO_2$ en position 3. Ces groupements -$NO_2$ se fixent sur les protéines, *in vivo,* suite à la production de peroxynitrite pouvant réagir avec certains résidus tyrosine déterminés de protéines déterminées.

On entend dans ce qui suit par pathologie chronique toute affection de longue durée et évoluant généralement de façon péjorative et s'accompagnant fréquemment de complications sous la forme de pathologies associées pouvant être elles-mêmes aiguës ou chroniques. On entend par pathologie aiguë toute affection manifestée par des symptômes plus ou moins importants se terminant après une période relativement courte par la guérison ou la mort.

« L'état de gravité et d'évolutivité d'une pathologie » est défini dans l'invention comme une étape définie, caractérisée en ce qu'elle décrit un état particulier de ladite pathologie, ledit état pouvant correspondre de l'absence de symptômes caractéristiques de ladite pathologie à l'état le plus avancé, c'est-à-dire où tous les symptômes décrits jusqu'à présent sont cumulés. La caractérisation de l'état de gravité et d'évolutivité de la pathologie est basée sur les connaissances cliniques et physiopathologiques de ladite pathologie.

Il est défini dans l'invention une corrélation entre taux de nitration et stress nitrant. Par stress nitrant, on définit une surproduction de peroxynitrite ($ONOO^-$) par rapport à la quantité de peroxynitrite produite chez un individu déclaré sain. Cela signifie que plus la quantité de peroxynitrite est élevée dans l'organisme, plus les espèces azotées dérivées du peroxynitrite seront capable de nitrer les protéines. Aussi, plus le stress nitrant sera élevé, plus les protéines seront susceptibles d'être nitrées.

Selon un mode de réalisation avantageux de l'invention, le dosage quantitatif est effectué à l'aide d'au moins un anticorps spécifique reconnaissant une tyrosine nitrée physiologiquement de ladite protéine ou séquence peptidique physiologique déterminée nitrée.

Par « tyrosine nitrée », on entend dans l'invention la modification de résidu tyrosine après l'addition du groupe -$NO_2$ sur le cycle aromatique de la tyrosine. Le $NO_2$ ainsi présent sur le résidu tyrosine l'est de préférence en position 3. Les termes « tyrosine » et «résidu tyrosine » sont utilisé de manière uniforme dans l'invention pour désigner l'acide aminé en question.

Dans l'invention, l'expression « anticorps spécifique reconnaissant une tyrosine nitrée » désigne un anticorps reconnaissant une séquence d'acides aminés immunogène spécifique, ou particulière, dans laquelle se trouve un résidu tyrosine nitré. La reconnaissance est dite spécifique, ce qui signifie que la séquence d'acides aminés immunogène dans laquelle se trouve un résidu tyrosine non nitré n'est pas reconnue par le dit anticorps spécifique, et qu'une séquence d'acides aminés immunogène différente de la séquence en acide aminés immunogène spécifique, ou particulière, contenant une tyrosine, ou un résidu tyrosine, même nitré, ne sera par reconnue par ledit anticorps spécifique.

Selon une illustration de l'invention, la protéine physiologique ou séquence peptidique déterminée nitrée est préférentiellement une protéine circulante, notamment plasmatique, notamment choisie parmi les protéines suivantes : l'albumine, la préalbumine, la protéine de liaison à la vitamine D (Vitamin D Binding Protein ; VDBP), la transferrine, la céruloplasmine, la protéine de liaison au rétinol (Retinol Binding Protein ; RBP), l'insuline, l'hémoglobine, l'actine β, la protéine bande 3 du transporteur d'anion érythrocytaire, la chaine β de la spectrine érythrocytaire, le précurseur de la fibronectine, la chaine β du fibrinogène et la protéine de la membrane érythrocytaire bande 4.1.

Par « protéine circulante», il est défini dans l'invention des protéines qui sont retrouvées naturellement dans le sang, le plasma et éventuellement la lymphe, le liquide céphalorachidien, la salive ou l'urine.

Lesdites protéines: l'albumine, la préalbumine, la protéine de liaison à la vitamine D (Vitamin D Binding Protein ; VDBP), la transferrine, la céruloplasmine, la protéine de liaison au rétinol (Retinol Binding Protein ; RBP), l'insuline, l'hémoglobine, l'actine β, la protéine bande 3 du transporteur d'anion, la chaine β de la spectrine erythrocytaire, le précurseur de la fibronectine, la chaine β du fibrinogène et la protéine de la membrane érythrocytaire bande 4.1, décrites dans l'invention sont des protéines circulantes, présentes dans le sang des humains ou des animaux et purifiables ou productibles *in vitro* selon les techniques classiques connues de l'homme de métier.

La préalbumine décrite dans l'invention est également communément appelée transthyrétine.

Dans l'invention, l'actine β peut également être appelée β actine.

L'hémoglobine est une protéine caractérisée en ce qu'elle comprend 4 sous unités : deux sous unités d'hémoglobine α et deux sous unités d'hémoglobine β.

Il est également à noter que la protéine bande 3 du transporteur d'anion érythrocytaire correspond à l'antigène CD233.

Les protéines impliquées dans l'invention, sous leur forme non nitrée, sont représentées par les séquences suivantes :l'albumine est représentée par la séquence SEQ ID NO : 1, la transthyrétine est représentée par la séquence SEQ ID NO : 2, la VDBP par la séquence SEQ ID NO : 3, la transferrine par la séquence SEQ ID NO : 4, la céruloplasmine par la séquence SEQ ID NO : 5, la RBP par la séquence SEQ ID NO : 6, l'Insuline par la séquence SEQ ID NO : 7, l'hémoglobine α par la séquence SEQ ID NO : 8, l'hémoglobine β par la séquence SEQ ID NO: 9, l'actine β par la séquence SEQ ID NO: 10, la protéine bande 3 du transporteur d'anion érythrocytaire par la séquence SEQ ID NO : 11, la chaine β de la spectrine érythrocytaire par la séquence SEQ ID NO : 12, le précurseur de la fibronectine par la séquence SEQ ID NO : 13, la chaine P du fibrinogène par la séquence SEQ ID NO : 14 et la protéine de la membrane érythrocytaire bande 4.1 par la séquence SEQ ID NO : 15.

Dans l'invention, les protéines précédentes, sous leur forme non nitrée représentées par les séquences SEQ ID NO 1 à SEQ ID NO 15, sont également représentées par les variants ou isoformes des dites séquences, ou toute protéine présentant une identité de séquence d'au moins 90%, et plus particulièrement 100%, avec ladite protéine.

Selon une illustration de l'invention, lesdites pathologies chroniques ou aiguës associées au stress nitrant appartiennent au groupe suivant : les maladies inflammatoires et autoimmunitaires, les maladies infectieuses, les maladies neurodégénératives, les maladies hypoxiques et ischémiques, le diabète sucré de types 1 et 2, les maladies métaboliques, l'hyper- et l'hypothyroïdie, les maladies cardiovasculaires et respiratoires ainsi que le cancer.

Dans une illustration de l'invention, les pathologies chroniques ou aiguës associées au stress nitrant dans l'invention correspondent :

- aux maladies neurodégénératives, dont entre autre les maladies de Parkinson, d'Alzheimer, la sclérose latérale amyotrophique, la sclérose en plaques, la leucomalacie périventriculaire ou encore la maladie de Creutzfeld-Jacob,
- aux maladies ischémiques, dont entre autres les maladies coronariennes, l'infarctus du myocarde, les accidents vasculaires cérébraux, les chocs ou la prééclampsie et l'éclampsie,
- aux maladies liées à l'hypoxie, telles que les bronchopathies chroniques obstructives, l'asthme, l'emphysème, le tabagisme, les pneumopathies fibrosantes, le syndrome d'apnées du sommeil, l'hypoxie anté- ou néonatale ainsi que les encéphalopathies liées à l'asphyxie perpartale,
- aux diabètes de type 1 et 2, ainsi qu'à l'insulinoresistance, l'hypoglycémie néonatale, l'hypoglycémie survenant dans le cadre de diabètes mal ou non équilibrés ou traités ainsi que plus généralement toutes les complications du diabète telles et de façon non exclusive, la rétinopathie, la néphropathie, la neuropathie, l'artériopathie et la cardiopathie diabétiques,
- aux maladies cardiovasculaires dont entre autres l'athérosclerose, l'insuffisance et la décompensation cardiaque ou l'hypertension artérielle et l'hypertension artérielle pulmonaire, et
- aux complications chez des malades ayant subi une transplantation telle que les greffes de moelle, rénale, cardiaque, cardiopulmonaire et hépatique.
- Aux maladies inflammatoires d'origine infectieuse ou non ainsi qu'aux maladies autoimmunes telles la polyarthrite rhumatoïde, l'ostéoarthrite, la spondylarthrite ankylosante, la sclérodermie, le lupus érythémateux disséminé ou toute autre forme de lupus, le syndrome de Sjögren, le syndrome de Goodpasture, l'artérite temporale, la sarcoïdose, la sclérose en plaques, le purpura thrombocytopénique autoimmun, l'anémie hémolytique autoimmune, le pemphigus, la polymyosite, la fibromyalgie...

Dans une autre illustration de l'invention, les pathologies chroniques ou aiguës associées au stress nitrant correspondent aux pathologies précédentes d'où sont exclues l'hypoxie anté-ou néonatale, les encéphalopathies liées à l'asphyxie perpartale et l'hypoglycémie néonatale.

Autrement dit, une autre illustration de l'invention décrit des pathologies chroniques ou aiguës associées au stress nitrant dans l'invention correspondant :

- aux maladies neurodégénératives, dont entre autre les maladies de Parkinson, d'Alzheimer, la sclérose latérale amyotrophique, la sclérose en plaques, la leucomalacie périventriculaire ou encore la maladie de Creutzfeld-Jacob,
- aux maladies ischémiques, dont entre autres les maladies coronariennes, l'infarctus du myocarde, les accidents vasculaires cérébraux, les chocs ou la prééclampsie et l'éclampsie,
- aux maladies liées à l'hypoxie, telles que les bronchopathies chroniques obstructives, l'asthme, l'emphysème, le tabagisme, les pneumopathies fibrosantes et le syndrome d'apnées du sommeil,

- aux diabètes de type 1 et 2, ainsi que l'insulinoresistance précédent le diabète de type 2, l'hypoglycémie survenant dans le cadre de diabètes mal ou non équilibrés ou traités ainsi que plus généralement toutes les complications du diabète telles et de façon non exclusive, la rétinopathie, la néphropathie, la neuropathie, l'artériopathie et la cardiopathie diabétiques,
- aux maladies cardiovasculaires dont entre autres l'athérosclerose, l'insuffisance et la décompensation cardiaque ou l'hypertension artérielle et l'hypertension artérielle pulmonaire, et
- aux complications chez des malades ayant subi une transplantation telle que les greffes de moelle, rénale, cardiaque, cardiopulmonaire et hépatique.
- aux maladies inflammatoires d'origine infectieuse ou non ainsi qu'aux maladies autoimmunes telles la polyarthrite rhumatoïde, l'ostéoarthrite, la spondylarthrite ankylosante, la sclérodermie, le lupus érythémateux disséminé ou toute autre forme de lupus, le syndrome de Sjögren, le syndrome de Goodpasture, l'artérite temporale, la sarcoïdose, la sclérose en plaques, le purpura thrombocytopénique autoimmun, l'anémie hémolytique autoimmune, le pemphigus, la polymyosite, la fibromyalgie...

Selon un autre mode de réalisation avantageux de l'invention, ladite protéine ou séquence peptidique physiologique déterminée nitrée impliquée dans l'invention est l'albumine nitrée.

Selon une illustration de l'invention, le dosage quantitatif, *notamment in vitro,* mentionné précédemment, est effectué à l'aide d'un anticorps spécifique reconnaissant spécifiquement le résidu tyrosine $Y^{138}$ nitrée de l'albumine, en particulier un anticorps monoclonal.

Par « résidu tyrosine $Y^{138}$ », il est entendu dans l'invention qu'il s'agit de la tyrosine en position 138 dans l'albumine humaine. L'invention concerne également la tyrosine en position équivalente dans les albumines d'autres mammifères non humains.

Dans l'invention, le résidu $Y^{138}$ de l'albumine humaine (l'albumine étant représentée sous sa forme non nitrée par la séquence SEQ ID NO 1) est contenu, sous sa forme nitrée, dans le peptide de séquence SEQ ID NO 16.

Selon une illustration de l'invention, le dosage quantitatif, notamment *in vitro,* mentionné précédemment, est effectué à l'aide d'un anticorps spécifique reconnaissant spécifiquement le résidu tyrosine $Y^{411}$ nitrée de l'albumine, en particulier un anticorps monoclonal.

Par « résidu tyrosine $Y^{411}$ », il est entendu dans l'invention qu'il s'agit de la tyrosine en position 411 dans l'albumine humaine. L'invention concerne également la tyrosine en position équivalente dans les albumines d'autres mammifères non humains.

Dans l'invention, le résidu $Y^{411}$ de l'albumine humaine (l'albumine étant représentée sous sa forme non nitrée par la séquence SEQ ID NO 1) est contenu sous sa forme nitrée dans le peptide de séquence SEQ ID NO 17.

L'invention concerne également un anticorps reconnaissant spécifiquement l'albumine nitrée sur le résidu tyrosine $Y^{138}$, en particulier un anticorps monoclonal.

Selon un mode de réalisation préféré, l'invention décrit un anticorps monoclonal susmentionné, sécrété par l'hybridome déposé selon le traité de Budapest à la CNCM (Collection Nationale de Culture de Microorganismes, Institut Pasteur, 25, rue du Docteur Roux, 75724 Paris CEDEX 15, France) le 8 janvier 2009, sous le numéro d'accession CNCM I-4111.

Ledit anticorps monoclonal susmentionné est obtenu par l'immunisation de souris à l'aide du peptide SEQ ID NO 16, selon la procédure décrite dans la partie expérimentale ci-après. Ledit anticorps est également appelé ci-après anticorps 13H10-3G12-3A6 ou anticorps (clone) 13H10. Cet anticorps monoclonal est d'isotype IgG2b.

L'invention concerne également un anticorps reconnaissant spécifiquement l'albumine nitrée sur le résidu tyrosine $Y^{411}$, en particulier un anticorps monoclonal.

Selon un mode de réalisation préféré, l'invention décrit un anticorps monoclonal susmentionné, sécrété par l'hybridome déposé selon le traité de Budapest à la CNCM (Collection Nationale de Culture de Microorganismes, Institut Pasteur, 25, rue du Docteur Roux, 75724 Paris CEDEX 15, France) le 8 janvier 2009, sous le numéro d'accession CNCM I-4110.

Ledit anticorps monoclonal susmentionné est obtenu par l'immunisation de souris à l'aide du peptide SEQ ID NO 17, selon la procédure décrite dans la partie expérimentale ci-après. Ledit anticorps est également appelé ci-après anticorps 2F3-2E2 ou anticorps (clone) 2F3. Cet anticorps monoclonal est d'isotype IgG1.

Les anticorps de l'invention sont à la fois des anticorps polyclonaux et monoclonaux.

Les deux anticorps de l'invention sont plus particulièrement des anticorps monoclonaux. Le terme anticorps dans l'invention inclut tous les fragments dérivés des anticorps, en particulier les fragments des dits anticorps monoclonaux présentant substantiellement la même spécificité antigénique pour la protéine déterminée nitrée. Ces fragments comprennent des fragments d'anticorps (c'est-à-dire Fab, F(ab')2, CDRs, etc...), des anticorps polyfonctionnels, des anticorps monocaténaires (ScFv) etc... Les anticorps de l'invention peuvent être produits à l'aide de méthodes conventionnelles, comprenant l'immunisation d'un animal et la récupération des cellules spléniques de manière à produire des hybridomes par fusion cellulaire. Les anticorps de l'invention peuvent être utilisés avantageusement sous forme d'un mélange d'anticorps monoclonaux.

Les méthodes de production des anticorps monoclonaux sont connues de l'homme de métier. Elles comprennent généralement l'immunisation d'un animal non humain avec un antigène, suivie de la récupération des cellules de thymus de l'animal qui sont fusionnées avec des cellules immortalisées, généralement des cellules de myélome. Les hybridomes résultant produisent des anticorps monoclonaux.

Les anticorps avantageux de l'invention sont préparés par immunisation d'animaux non humains à l'aide de peptides spécifiques des séquences encadrant les tyrosines $Y^{138}$ et $Y^{411}$ nitrés de l'albumine substantiellement purs. Ces peptides ont les séquences suivantes :

- EETFLKK($Y^{138}$-NO$_2$)LYEIARR- comprenant la tyrosine $Y^{138}$, et représenté par la séquence SEQ ID NO : 16.
- LVR($Y^{411}$-NO$_2$)TKKV- comprenant la tyrosine $Y^{411}$, et représenté par la séquence SEQ ID NO : 17.

Les peptides ci-dessus sont nouveaux.

L'invention a également pour but de fournir l'hybridome déposé selon le traité de Budapest à la CNCM (Collection Nationale de Culture de Microorganismes, Institut Pasteur, 25, rue du Docteur Roux, 75724 Paris CEDEX 15, France) le 8 janvier 2009, sous le numéro d'accession CNCM I-4111.

De plus, l'invention décrit l'hybridome déposé selon le traité de Budapest à la CNCM (Collection Nationale de Culture de Microorganismes, Institut Pasteur, 25, rue du Docteur Roux, 75724 Paris CEDEX 15, France) le 8 janvier 2009, sous le numéro d'accession CNCM I-4110.

Ces deux hybridomes sont nouveaux. Les procédés d'obtention de ces deux hybridomes sont décrits dans les exemples ci-après.

L'invention est également illustrée par une méthode de *diagnostic in vitro* de l'état de gravité et d'évolutivité d'une pathologie chronique ou aiguë associée au stress nitrant dans un échantillon biologique d'un individu, comprenant :

- le dosage quantitatif, dans un échantillon biologique d'un individu, du taux de nitration d'une première protéine ou séquence peptidique physiologique déterminée nitrée,
- la quantification dudit taux de nitration de ladite première protéine ou séquence peptidique physiologique déterminée en comparant la concentration de ladite première protéine ou séquence peptidique physiologique déterminée nitrée avec la concentration totale de ladite première protéine ou séquence peptidique physiologique déterminée, nitrée et non nitrée, issus d'un échantillon biologique du même individu,
- la comparaison dudit taux de nitration de ladite première protéine ou séquence peptidique physiologique déterminée avec le taux de nitration d'une seconde protéine ou séquence peptidique physiologique déterminée issue d'un échantillon biologique d'un individu non affecté par ladite pathologie chronique ou aigüe,

  • ladite seconde protéine ou séquence peptidique physiologique déterminée étant un variant ou une isoforme, ou présentant une identité de séquence en acides aminés d'au moins 90%, et notamment 100%, avec la susdite première protéine ou séquence peptidique physiologique déterminée,
  • ladite première protéine ou séquence peptidique physiologique déterminée étant nitrée sur au moins un résidu tyrosine situé à une position équivalente dans la susdite seconde protéine ou séquence peptidique physiologique déterminée,

- la déduction, à partir de la comparaison effectuée à l'étape précédente, du degré de stress nitrant de l'individu pouvant correspondre à un état de gravité et d'évolutivité de la dite pathologie chronique ou aigüe.

L'invention concerne une méthode de *diagnostic in vitro* de l'état de gravité et d'évolutivité d'une pathologie chronique associée au stress nitrant dans un échantillon biologique d'un individu, comprenant :

- la détection d'un complexe immun résultant de la mise en contact d'au moins un anticorps reconnaissant spécifiquement un résidu tyrosine nitré de l'albumine physiologique avec l'albumine physiologique nitrée issue d'un échantillon biologique d'un individu, la dite détection du complexe immun permettant la détermination du taux de nitration de ladite albumine physiologique nitrée,
  ledit anticorps reconnaissant la nitration de l'albumine humaine sur

  - le résidu tyrosine $Y^{138}$, ou
  - le résidu tyrosine $Y^{411}$
  - la comparaison dudit taux de nitration de résidu tyrosine de l'albumine physiologique, avec les taux de nitrations de résidus tyrosine d'un ensemble de n albumines physiologiques dont la valeur du taux de nitration est connue et associée respectivement à un état déterminé de gravité et d'évolutivité de la dite pathologie chronique ou aigüe,

- la déduction, à partir de la comparaison effectuée à l'étape précédente, du degré de stress nitrant de l'individu pouvant correspondre à un état de gravité et d'évolutivité de la dite pathologie chronique ou aigüe, ladite pathologie chronique ou aiguë associée au stress nitrant appartenant au groupe suivant : les maladies inflammatoires, les maladies ischémiques et les maladies cardiovasculaires.

Cette méthode s'appuie sur la découverte par les inventeurs d'une méthode de nitration des protéines *in vitro* permettant la nitration sur des résidus tyrosines équivalents aux résidus nitrés physiologiquement.

La quantification du taux de nitration de la protéine déterminée nitrée est réalisée par l'établissement du rapport entre la quantité de protéine déterminée nitrée et la quantité totale de ladite protéine déterminée, nitrée et non nitrée. Cette valeur est donc idéalement comprise entre 0 et 1.

Par « première protéine physiologique » on entend dans ce qui suit toute protéine physiologique correspondant à la protéine physiologique étudiée, prélevée de l'échantillon biologique de l'individu en question, à savoir l'individu testé.

Par « seconde protéine physiologique » est définie une protéine présentant une identité de séquence d'au moins 90%, et plus particulièrement 100%, avec ladite première protéine déterminée, cette dite seconde protéine étant issue d'un échantillon biologique, de même nature ou de nature différente, d'un autre individu que l'individu d'où est issue la première protéine déterminée. En particulier l'individu duquel est issu la deuxième protéine déterminée nitrée correspond à un individu n'étant pas affecté par ladite pathologie associée au stress nitrant.

Par « variant » ou « isoforme » de la protéine déterminée, on définit toute protéine, peptide ou polypeptide encodés par un même gène, sauvage ou mutant, dont les séquences en acides aminés sont proches de celle de la protéine déterminée et présentant une identité de séquence d'au moins 90%, et plus particulièrement 100%, avec la protéine dont il est question et qui possède généralement la même fonction biologique que la dite protéine en question. Ne sont donc pas exclus des variants issus de mutations géniques et présentant des gains ou pertes de fonction. Ces variants ou isoformes peuvent être, par exemple, le résultat d'un épissage alternatif d'un même gène ou de l'expression de plusieurs gènes homologues dont les séquences ont divergé.

La première protéine physiologique et la seconde protéine physiologique peuvent être les mêmes protéines, exception faite de la nitration ou non d'un ou plusieurs résidus tyrosine.

Par « position équivalente » de tyrosine entre la première et la seconde protéine, on entend dans l'invention une position dans la séquence en acides aminés de la première protéine déterminée qui correspond à la position d'une tyrosine dans la seconde protéine déterminée telle que, si on aligne les deux protéines selon des procédures standard d'alignement de séquences (algorithme de Needleman-Wunsch, Algorithme de Smith-Waterman ...), ces deux résidus se retrouvent à la même position. De manière plus explicite, cela signifie que même si les deux protéines ne sont pas de même taille, à l'issue de l'alignement, les deux résidus se retrouveront à des positions identiques par rapport aux séquences environnantes.

La méthode également décrite dans l'invention consiste donc en un dosage du taux de nitration physiologique ou pathologique d'une première protéine physiologique déterminée de l'invention, à partir d'un échantillon biologique d'un individu. L'échantillon biologique avantageux dans l'invention peut être du sang ou un produit dérivé de celui-ci (plasma, sérum, globules, plaquettes). Les échantillons biologiques tels que l'urine, le liquide céphalorachidien, les tissus, les tumeurs, des cellules, les frottis sanguins, etc... peuvent également être impliqués dans l'invention. Le dosage consiste donc en l'évaluation de la nitration des protéines physiologiques nitrées. Une valeur finie du taux de nitration physiologique de ladite protéine déterminée est donc établie.

Le taux de nitration déterminé précédemment est comparé au taux de nitration de la seconde protéine déterminée issue d'un échantillon biologique d'un individu considéré comme n'étant pas atteint par l'une des pathologies de l'invention.

A ce jour, les taux de nitration normaux des protéines physiologiques ne sont pas connus de manière quantitative. Ils sont généralement estimés comme étant faibles (< 5%).

Les taux de nitration de la première et de la deuxième protéine physiologique déterminée sont alors comparés. Lorsque le taux de nitration de la première protéine nitrée est faible et comparable à celle d'individus sains, l'individu, dont l'échantillon biologique a permis d'isoler ladite première protéine physiologique déterminée, est considéré comme n'étant pas affecté par la pathologie chronique ou aiguë associée au stress nitrant dont il est question. L'individu, dont l'échantillon biologique a permis d'isoler ladite première protéine physiologique déterminée peut également être atteint par la pathologie chronique ou aiguë associée au stress nitrant en phase peu évolutive ou en rémission.

Si le taux de nitration de la première protéine nitrée est supérieur à zéro, l'individu est atteint par la pathologie chronique ou aiguë associée au stress nitrant dont il est question, ladite pathologie chronique ou aiguë pouvant être en phase évolutive ou en phase aigüe.

Dans un mode de réalisation avantageux de l'invention, le taux de nitration de la première protéine déterminée peut être comparé au taux de nitration d'une troisième protéine physiologique déterminée nitrée. Par troisième protéine physiologique, on définit une protéine présentant une identité de séquence d'au moins 90%, et plus particulièrement 100%, avec ladite première protéine déterminée, cette dite troisième protéine étant issue d'un échantillon biologique, de même nature ou de nature différente, d'un autre individu que l'individu d'où est issue la première protéine déterminée. En particulier, l'individu duquel est issue la troisième protéine déterminée nitrée correspond à un individu étant affecté par ladite pathologie associée au stress nitrant, et présentant la totalité des symptômes associés à cette pathologie.

Ainsi, de la comparaison du taux de nitration de la première protéine physiologique avec le taux de nitration de la seconde et/ou de la troisième protéine physiologique déterminée, il est possible de déterminer le degré de stress nitrant pouvant être corrélé à l'état de gravité et d'évolutivité de la pathologie, chronique ou aigüe, associée au stress nitrant chez le patient d'où est issue la première protéine physiologique.

L'invention est également illustrée par une méthode de diagnostic *in vitro* caractérisée par les étapes suivantes :

- le dosage quantitatif, dans un échantillon biologique d'un individu, du taux de nitration d'une première ou séquence peptidique protéine physiologique déterminée nitrée,
- la quantification dudit taux de nitration de ladite première protéine ou séquence peptidique physiologique déterminée en comparant la concentration de ladite première protéine ou séquence peptidique physiologique déterminée nitrée avec la concentration totale de ladite première protéine ou séquence peptidique physiologique déterminée, nitrée et non nitrée, issus d'un échantillon biologique du même individu,
- la comparaison dudit taux de nitration de résidus tyrosine de la première protéine ou séquence peptidique physiologique déterminée nitrée avec les taux de nitration d'un ensemble de n protéines ou séquences peptidiques physiologiques déterminées, la valeur du taux de nitration de chacune des susdites n protéines ou séquences peptidiques déterminées étant connues et associées respectivement à un état déterminé de gravité et d'évolutivité de la dite pathologie chronique ou aigüe,

  • lesdites n protéines ou séquences peptidiques physiologiques déterminées étant des variants ou des isoformes de la susdite première protéine ou séquence peptidique déterminée, ou présentant une identité de séquence en acides aminés d'au moins 90%, et notamment 100%, avec la susdite première protéine ou séquence peptidique physiologique déterminée,
  • lesdites n protéines ou séquences peptidiques physiologiques déterminées étant nitrées sur au moins un résidu tyrosine situé à une position équivalente au résidu tyrosine dans la susdite première protéine ou séquence peptidique physiologique déterminée,

- la déduction, à partir de la comparaison effectuée à l'étape précédente, du degré de stress nitrant de l'individu pouvant correspondre à un état de gravité et d'évolutivité de la dite pathologie chronique ou aigüe.

Les n protéines physiologiques déterminées de l'invention sont définies telles qu'elles présentent une homologie de séquence d'au moins 90%, et plus particulièrement 100% d'identité avec la première protéine déterminée ou qu'elle sont issues du même gène. Dans l'invention, n varie de 2 à 10, et avantageusement de 2 à 5.

Les dites n protéines sont issues de n échantillons biologiques différents. Le taux de nitration de chacune des n protéines déterminées est de valeur connue et est associé à un état particulier de ladite pathologie associée au stress nitrant.

La méthode également décrite dans l'invention consiste donc en un dosage du taux de nitration physiologique d'une première protéine physiologique déterminée de l'invention, à partir d'un échantillon biologique d'un individu.

Le taux de nitration déterminé précédemment est comparé au taux de nitration d'un ensemble de n protéines déterminées, chacune des n protéines déterminées étant issue d'un échantillon biologique différent, soit n échantillons biologiques. Les n échantillons peuvent provenir soit de n individus différents, soit du même individu mais dont des échantillons ont été prélevés aux cours des n stades de la pathologie associée au stress nitrant qu'il a développé.

Les n échantillons biologiques correspondent à un stade d'évolution différent de la pathologie associée au stress nitrant bien caractérisé, et quantifié.

Dans un des modes avantageux de l'invention, les n protéines déterminées sont définies de la manière suivante : une protéine déterminée correspond à une protéine issue d'un échantillon biologique d'un individu non atteint par la pathologie associée au stress nitrant, et les n-1 autres protéines déterminées correspondent à n-1 échantillons biologiques définissant n-1 états de gravité et d'évolutivité particuliers différents de la dite pathologie associée au stress nitrant.

Les taux de nitration de la première et de la deuxième protéine physiologique déterminée sont alors comparés.

Lorsque le taux de nitration de la première protéine nitrée correspond au taux de nitration de l'une des n protéines physiologiques déterminées l'individu est atteint d'un stress nitrant associée à la pathologie chronique ou aigüe dont il est question, au même degré que celui de l'individu dont est issu la protéine n physiologique déterminée nitrée.

Plus particulièrement, l'invention concerne une méthode de *diagnostic in vitro* dans laquelle le dosage quantitatif *in vitro* est effectué à l'aide d'au moins un anticorps, chaque anticorps reconnaissant spécifiquement un résidu tyrosine nitré d'une protéine ou séquence peptidique physiologique déterminée nitrée.

L'invention est illustrée par une méthode de diagnostic mentionnée précédemment, dans laquelle la protéine physiologique déterminée nitrée est préférentiellement une protéine circulante, notamment plasmatique, notamment choisie parmi : l'albumine, la préalbumine, la protéine de liaison à la vitamine D (Vitamin D Binding Protein ; VDBP), la transferrine, la céruloplasmine, la protéine de liaison au rétinol (Retinol Binding Protein ; RBP), l'insuline, l'hémoglobine, l'actine β, la protéine bande 3 du transporteur d'anion érythrocytaires, la chaine P de la spectrine erythrocytaire, le précurseur de la fibronectine, la chaine P du fibrinogène et la protéine de la membrane érythrocytaire bande 4.1.

De plus, l'invention est illustrée par une méthode de diagnostic, dans laquelle la pathologie chronique ou aiguë liée au stress nitrant appartient au groupe suivant : les maladies inflammatoires, les maladies infectieuses, les maladies neurodégénératives, les maladies hypoxiques et ischémiques, le diabète, les maladies métaboliques, les maladies cardiovasculaires et respiratoires ainsi que le cancer.

L'invention concerne une méthode de *diagnostic in vitro* telle que définie ci-dessus, dans laquelle le degré de stress nitrant de l'individu peut correspondre à un état de gravité et d'évolutivité des pathologies choisies parmi : les maladies inflammatoires, les maladies ischémiques, et les maladies cardiovasculaires.

L'invention est illustrée par une méthode de *diagnostic in vitro* de l'état de gravité et d'évolutivité d'une pathologie chronique ou aiguë associée au stress nitrant, dans un échantillon biologique, comprenant :

- la détection d'un complexe immun résultant de la mise en contact d'au moins un anticorps reconnaissant spécifiquement un résidu tyrosine nitré de l'albumine physiologique avec l'albumine physiologique nitrée issue d'un échantillon biologique d'un individu, la dite détection du complexe immun permettant la détermination du taux de nitration de ladite albumine physiologique nitrée,
- la comparaison dudit taux de nitration de résidu tyrosine de l'albumine physiologique, avec les taux de nitration de résidus tyrosine d'un ensemble de n albumines physiologiques dont la valeur du taux de nitration est connue et associée respectivement à un état déterminé de gravité et d'évolutivité de la dite pathologie chronique ou aigüe,
- la déduction, à partir de la comparaison effectuée à l'étape précédente, du degré de stress nitrant de l'individu pouvant correspondre à un état de gravité et d'évolutivité de la dite pathologie chronique ou aigüe.

Le « complexe immun » dont il est question ci-dessus (ou complexe antigène-anticorps) résulte de la combinaison d'un épitope (antigène) avec un anticorps dirigé contre cet épitope et seulement contre cet antigène. L'antigène impliqué dans l'invention correspond aux protéines déterminées nitrées citées précédemment.

La détection du complexe immun est effectuée à l'aide d'anticorps monoclonaux ou polyclonaux reconnaissant spécifiquement la protéine déterminée, de manière directe ou indirecte. Lors d'une détection directe, ledit anticorps permettant la détection est généralement couplé à des marqueurs. Les marqueurs peuvent être choisis parmi les radiomarqueurs, la biotine, des enzymes, des agents fluorescents, des particules magnétiques, etc...

Lors d'une détection indirecte, l'anticorps reconnaissant la protéine déterminée est lui même reconnu par un anticorps de détection couplé à l'un des marqueurs.

Les anticorps utilisés peuvent être mis en oeuvre sous forme soluble, ou immobilisés sur des supports, et plus particulièrement des billes, des plaques, des colonnes, etc...

La méthode décrite dans l'invention consiste en un dosage du taux de nitration physiologique d'une première protéine physiologique déterminée, à partir d'un échantillon biologique d'un individu.

Le taux de nitration déterminé précédemment est comparé au taux de nitration d'un ensemble de n protéines déterminées, chacune des n protéines déterminées étant issue d'un échantillon biologique différent, soit n échantillons biologiques. Les n échantillons peuvent provenir soit de n individus différents, soit du même individu mais dont des échantillons ont été prélevés aux cours des n stades de la pathologie associée au stress nitrant qu'il a développée.

Les n échantillons biologiques correspondent à un stade d'évolution différent de la pathologie associée au stress nitrant bien caractérisé, et quantifié.

Dans un des modes avantageux de l'invention, les n protéines déterminées sont définies de la manière suivante : une protéine déterminée correspond à une protéine issue d'un échantillon biologique d'un individu non atteint par la pathologie associée au stress nitrant, et les n-1 autres protéines déterminées correspondent à n-1 échantillons biologiques définissant n-1 états de gravité et d'évolutivité particuliers différents de la dite pathologie associée au stress nitrant.

Lorsque le taux de nitration de la première protéine nitrée correspond au taux de nitration de l'une des n protéines physiologiques déterminées, l'individu est atteint par la pathologie chronique ou aiguë associée au stress nitrant dont il est question, à un stade identique à celui de l'individu dont est issue la protéine n physiologique déterminée nitrée.

Plus particulièrement, l'invention concerne une méthode de *diagnostic in vitro,* dans laquelle ledit anticorps reconnaissant la nitration de l'albumine sur le résidu tyrosine $Y^{138}$.

Egalement, l'invention concerne une méthode de diagnostic *in vitro,* dans laquelle ledit anticorps reconnaissant la nitration de l'albumine sur le résidu tyrosine $Y^{411}$.

L'invention est illustrée par une méthode de diagnostic *in vitro* de l'état de gravité et d'évolutivité d'une pathologie chronique ou aiguë associée au stress nitrant, dans un échantillon biologique, comprenant :

- la détection, à l'aide d'un anticorps dirigé contre l'albumine, d'un complexe immun résultant dé la mise en contact d'au moins un anticorps spécifique, avec l'albumine physiologique nitrée issue d'un échantillon biologique d'un individu, la dite détection du complexe immun permettant la détermination du taux de nitration l'albumine physiologique nitrée,
- la comparaison dudit taux de nitration de résidu tyrosine de l'albumine physiologique nitrée, avec les taux de nitrations de résidus tyrosine d'un ensemble de n albumines physiologiques nitrées dont la valeur du taux de nitration de résidus tyrosine de l'albumine physiologique nitrée est connue et associée respectivement à un état de gravité et d'évolutivité de la dite pathologie chronique ou aigüe,
- la déduction, à partir de la comparaison effectuée à l'étape précédente, du degré de stress nitrant de l'individu pouvant être correspondre à un état de gravité et d'évolutivité de la dite pathologie chronique ou aigüe.

Ainsi, la méthode décrite dans l'invention consiste en un dosage du taux de nitration physiologique de l'albumine physiologique de l'invention, à partir d'un échantillon biologique d'un individu, en utilisant les anticorps spécifiques reconnaissant l'albumine nitrée de l'invention.

Les anticorps de l'invention permettent d'immobiliser spécifiquement l'albumine nitrée parmi une population de protéines, nitrées et non nitrées. Le complexe immun formé est alors détecté et la quantité de complexe est indicative du taux de nitration de l'albumine dans l'échantillon biologique.

Le taux de nitration déterminé précédemment est comparé au taux de nitration d'un ensemble de n albumines, chacune des n albumines étant issue d'un échantillon biologique différent. Les n échantillons peuvent provenir soit de n individus différents, soit du même individu mais dont des échantillons ont été prélevés aux cours des n stades de la pathologie associée au stress nitrant que le patient a développé.

Les n échantillons biologiques correspondent à des stades d'évolution différents de la pathologie associée au stress nitrant bien caractérisés, selon les critères cliniques de la pathologie, et le taux de nitration de l'albumine issus de ces n échantillons est quantifié.

Dans un des modes avantageux de l'invention, les n albumines déterminées sont définies de la manière suivante : une albumine déterminée correspond à une protéine issue d'un échantillon biologique d'un individu non atteint par la pathologie associée au stress nitrant, et les n-1 autres albumines déterminées correspondent à n-1 échantillons biologiques définissant n-1 états de gravité et d'évolutivité particuliers différents de la dite pathologie associée au stress nitrant.

L'invention est également illustrée par une méthode de *diagnostic in vitro* dans laquelle le degré de stress nitrant de l'individu peut correspondre à un état de gravité et d'évolutivité des pathologies choisies parmi: les maladies inflammatoires, les maladies infectieuses, les maladies neurodégénératives, les maladies ischémiques, le diabète, les maladies métaboliques, les maladies cardiovasculaires et respiratoires ainsi que le cancer.

Une méthode de diagnostic *in vitro* illustrant l'invention met particulièrement en oeuvre la détection du complexe immun à l'aide de techniques conventionnelles telles que l'ELISA (direct ou compétitif), l'immunohistochimie et l'immunocytochimie, l'immunoprécipitation, la néphélométrie, la turbidimétrie, le western blot ou toute autre méthode immunochimique ou radio-immunologique (RIA).

L'invention concerne une méthode de *diagnostic in vitro* telle que définie ci-dessus, dans laquelle la dite détection du complexe immun est réalisée par les techniques d'ELISA, d'immunohistochimie, d'immunocytochimie, d'immunoprécipitation, de western blot ou de radioimmunologie.

L'ELISA, le RIA, l'immunohistochimie et l'immunocytochimie, l'immunoprécipitation, la néphélométrie, la turbidimétrie, le western blot ou toute autre méthode immunochimique sont des techniques classiques connues de l'homme de métier.

Plus particulièrement, l'invention est illustrée par une méthode de diagnostic, préférentiellement *in vitro,* de l'état de gravité et d'évolutivité d'une pathologie chronique ou aiguë associée au stress nitrant dans un échantillon biologique d'un individu, tel que défini précédemment, dans laquelle le dosage quantitatif dans un échantillon biologique d'un individu, du taux de nitration d'une première protéine, notamment l'albumine, ou séquence peptidique physiologique déterminée nitrée, est réalisé à l'aide d'au moins un anticorps tel que défini précédemment, ledit anticorps étant soit fixé sur un support en tant qu'anticorps de capture, soit sert d'anticorps de détection spécifique.

En d'autres termes la méthode de diagnostic mentionnée précédemment est préférablement réalisée à l'aide d'un ELISA, dans lequel

- soit un anticorps spécifique de l'albumine nitrée ou non est immobilisé et sert d'anticorps de capture de l'albumine nitrée ou non, et les protéines immobilisées nitrées sont révélées a l'aide de l'anticorps monoclonal tel que défini ci-dessus ; éventuellement, les deux anticorps monoclonaux de l'invention peuvent être utilisés simultanément,
- soit un anticorps monoclonal, tel que défini ci-dessus, spécifique de l'albumine nitrée est immobilisé et sert d'anticorps de capture de l'albumine nitrée, et les protéines immobilisées nitrées sont révélées a l'aide d'un anticorps reconnaissant l'albumine nitrée ou non, éventuellement, les deux anticorps monoclonaux de l'invention peuvent être utilisés simultanément,

- soit un anticorps monoclonal tel que défini spécifique de l'albumine nitrée est immobilisé et sert d'anticorps de capture de l'albumine nitrée, et les protéines immobilisées nitrées sont révélées a l'aide d'un anticorps monoclonal tel que défini spécifique de l'albumine nitrée.

Dans la dernière catégorie, les couples possibles sont

- anticorps de capture : 2F3, anticorps de détection : 13H10, ou
- anticorps de capture : 13H10, anticorps de détection : 2F3.

Egalement, dans un autre mode de réalisation préféré, l'invention est illustrée par une méthode de diagnostic mentionnée précédemment qui correspond à un test de radioimmunologie (RIA) dans lequel :

- l'échantillon issu du patient, susceptible de contenir de l'albumine nitrée, est incubé avec une quantité déterminée, connue, d'albumine nitrée marquée à l'aide d'un traceur, ledit traceur étant l'iode radioactif ($^{125}$I, pour obtenir un mélange,
- le mélange précédent est mis en contact avec au moins un des anticorps spécifiques de l'albumine nitrée définis ci-dessus, afin de permettre la formation d'un complexe immun,
- le complexe immun formé entre ladite albumine nitrée marquée et au moins un des anticorps spécifiques de l'albumine nitrée obtenu à l'étape suivante est détecté et quantifié à l'aide d'un système de détection spécifique du marquage de ladite albumine nitrée, préférentiellement ladite détection étant réalisée à l'aide d'un compteur gamma, ou tout compteur permettant la détection de la désintégration des isotopes radioactifs,
- la déduction, à partir de la quantification précédente, de la quantité d'albumine nitrée initialement présente dans l'échantillon.

Dans le RIA, l'antigène à doser (l'albumine nitrée) entre en compétition avec l'antigène marqué (l'albumine nitrée marquée) pour la liaison à l'anticorps ; la totalité des sites anticorps disponibles est liée. On mesure la fraction liée qui diminue exponentiellement avec la concentration en antigène à doser. On peut procéder en phase liquide homogène ou en phase solide hétérogène ; dans ce dernier cas, la séparation des fractions libre et liée est facilitée.

Le principe du dosage RIA repose sur la compétition entre l'albumine nitrée marquée à l'iode 125 et l'albumine nitrée contenue dans les standards ou les échantillons à mesurer, vis-à-vis d'un nombre donné et limité de sites anticorps anti-albumine nitré spécifiques (Anticorps 13h10 et/ou 2F3) éventuellement fixés sur la phase solide (tubes revêtus, plaque de microtitration...).

A la fin de la période d'incubation, l'excès de traceur est aisément éliminé par une étape de lavage. La quantité d'albumine nitrée marquée liée à l'anticorps est inversement proportionnelle à la quantité d'albumine nitrée non marquée présente dans l'essai.

La présente invention est également illustrée par l'utilisation d'une protéine ou d'une séquence peptidique déterminée nitrée, nitrée sur au moins un résidu tyrosine, pour la mise en oeuvre d'une méthode de dosage quantitatif, notamment *in vitro,* du taux de nitration physiologique d'une protéine ou d'une séquence peptidique physiologique nitrée, ladite protéine ou séquence peptidique déterminée nitrée présentant une identité de séquence en acides aminés d'au moins 90%, et notamment 100%, avec la susdite protéine ou séquence peptidique physiologique déterminée nitrée, ladite protéine ou séquence peptidique déterminée nitrée présentant une nitration sur au moins un résidu tyrosine à une position équivalente à celle du résidu tyrosine de la susdite protéine ou séquence peptidique physiologique déterminée nitrée, la nitration de ladite protéine ou séquence peptidique physiologique déterminée étant corrélée au stress nitrant associé à des pathologies chroniques ou aigües.

L'invention est illustrée par l'utilisation d'une protéine ou d'une séquence peptidique déterminée nitrée, dans laquelle ladite protéine ou séquence peptidique déterminée nitrée permet de générer au moins un anticorps monoclonal ou polyclonal capable de reconnaître spécifiquement un résidu tyrosine nitré de la dite protéine ou séquence peptidique déterminée, lequel anticorps étant capable de reconnaître également spécifiquement ledit résidu tyrosine nitré à une position équivalente dans la protéine ou séquence peptidique physiologique déterminée nitrée.

L'invention est également illustrée par un procédé de préparation *in vitro* d'une protéine ou séquence peptidique déterminée nitrée sur des résidus tyrosine dans lesquelles les résidus tyrosine nitrés correspondent aux résidus tyrosine nitrés physiologiquement d'une protéine ou séquence peptidique physiologique déterminée nitrée dans des pathologies chroniques ou aigües associées au stress nitrant, ladite protéine ou séquence peptidique déterminée nitrée présentant une identité de séquence en acides aminés d'au moins 90%, et notamment 100%, avec la susdite protéine physiologique déterminée nitrée, comprenant :

- la nitration *in vitro* d'une protéine ou séquence peptidique déterminée par du tetranitrométhane dans un rapport molaire avec les tyrosines nitrables de la protéine ou séquence peptidique déterminée n'excédant pas 20 :1, dans un tampon aqueux de pH compris entre 7,5 et 8,5,
- l'identification *in vitro* des tyrosines nitrées de ladite protéine déterminée nitrée, en particulier par spectrométrie de masse, et
- éventuellement, la comparaison des tyrosines nitrées identifiées sur ladite protéine ou séquence peptidique déterminée avec les résidus tyrosine de la protéine ou séquence peptidique physiologique déterminée nitrée.

Il existe de nombreux agents nitrant des protéines, et particulièrement le péroxynitrite, le tétranitrométhane, le 3-morpholino-sydonimine, le sodium α-oxyhyponitrite, la spermine-NONOate et autres NONOates, le chlorure de nitryle (NO$_2$Cl), le nitroprussiate et la nitroglycérine. Ces agents nitrants ne sont pas les seuls. La précédente liste d'agents nitrant n'est pas exhaustive.

L'invention a pour avantage de fournir un procédé permettant de façon spécifique de nitrer *in vitro* des protéines sur des résidus tyrosines, résidus qui sont susceptibles d'être nitrés physiologiquement.

Contrairement à ce qui est décrit dans l'art antérieur, le procédé de l'invention utilise du tétranitrométhane dans des conditions de concentrations faibles qui peuvent être considérées comme mimant des conditions proches des conditions de nitration physiologiques. L'avantage de la nitration *in vitro* par du tétranitrométhane en milieu aqueux à pH faiblement alcalin par rapport au peroxynitrite consiste dans le fait qu'utilisé dans ces conditions le tétranitrométhane est beaucoup moins oxydant que le peroxynitrite ce qui évite de ce fait la formation de dérivés oxydés et produits d'oxydation des acides aminés, en particulier des cystéines, méthionines, tryptophanes et tyrosines, des protéines traitées. Cette méthode minimise également la formation de ponts disulfure ainsi que de dityrosines.

Le procédé de l'invention décrit donc une étape de nitration des protéines, où les quantités de tétranitrométhane sont dépendantes de la quantité de tyrosines susceptibles d'être nitrées contenues dans la protéine à nitrer. Pour adapter les concentrations de tétranitrométhane à utiliser dans le procédé de l'invention, il est donc nécessaire d'estimer ou de déterminer le nombre de tyrosines nitrables ou nitrées des protéines considérées.

Un des moyens de déterminer les tyrosines nitrables ou nitrées des protéines, est de réaliser une nitration *in vitro* des protéines avec du tétranitrométhane, à des rapports molaires croissants par rapport au nombre de résidus tyrosine présents dans la protéine. A l'issue de la nitration, la protéine est purifiée sur de l'anti-nitrotyrosine conjugué à l'agarose. Les résidus nitrés sont ensuite déterminés par spectrométrie de masse. Cette méthode permet d'avoir une information relative sur le nombre de tyrosines susceptibles d'être nitrées

Les figures suivantes illustrent l'invention :

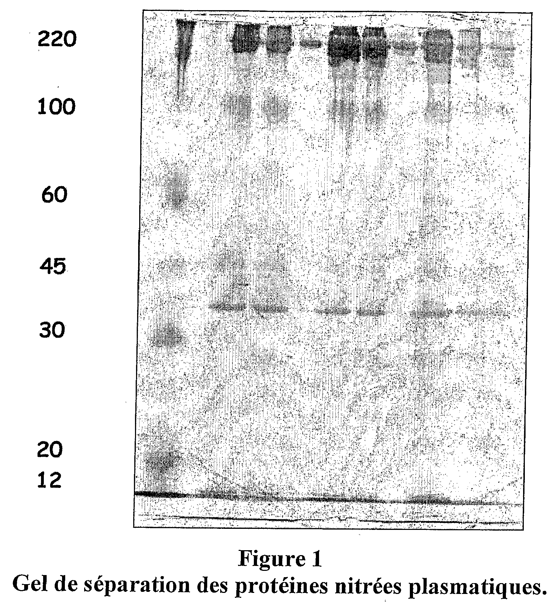

Figure 1 : La figure 1 représente un gel de séparation des protéines nitrées issues d'échantillons de plasma.

Les échantillons de plasma sont soumis à une immunoprécipitation par un anticorps anti-nitrotyrosine conjugué à l'agarose. Les protéines nitrées immunoprécipitées sont séparées par électrophorèse (SDS-PAGE) et révélées par coloration au bleu de Coomassie colloïdal. Les pistes correspondent à des immunoprécipités de protéines nitrées issues de plasma de différents patients.

Des marqueurs de poids moléculaire sont indiqués sur la partie gauche du gel.

Figures 2 : Les figures 2A, 2B, 2C, 2D, 2E, 2F, 2G et 2H représentent les spectres de masse des protéines nitrées prélevées sur le gel de séparation. Les spectres de masse des peptides sont obtenus à l'aide de la méthode MALDI-TOF.

La figure 2A correspond au spectre de masse correspondant aux peptides issus de l'albumine.
La figure 2B correspond au spectre de masse correspondant aux peptides issus de l'actine β.
La figure 2C correspond au spectre de masse correspondant aux peptides issus de la protéine de liaison à la vitamine D..
La figure 2D correspond au spectre de masse correspondant aux peptides permettant d'identifier la bande 3 de la protéine transporteuse d'anion érythrocytaire.
La figure 2E correspond au spectre de masse correspondant aux peptides issus de la chaine β de la spectrine érytrocytaire.
La figure 2F correspond au spectre de masse correspondant aux peptides issus de la chaine β du fibrinogène
La figure 2G correspond au spectre de masse correspondant aux peptides issus de la protéine de membrane érythrocytaire bande 4.1.
La figure 2H correspond au spectre de masse correspondant aux peptides issus du précurseur de la fibronectine.

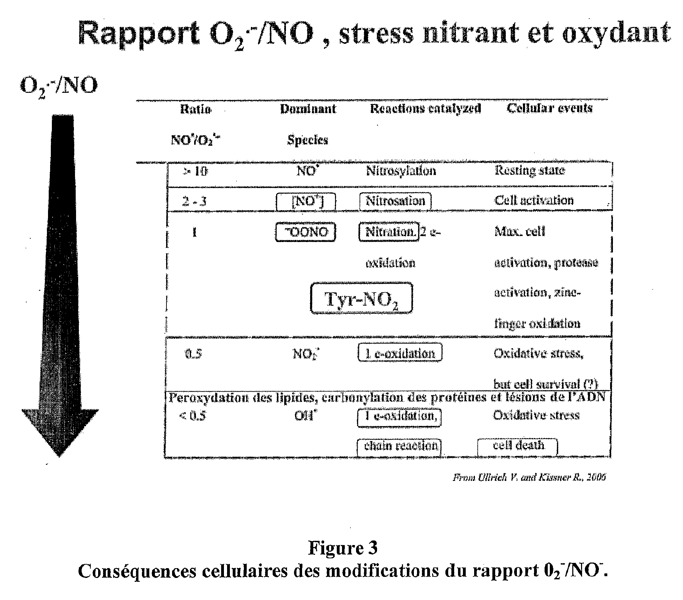

Figure 3 : La figure 3 schématise les conséquences cellulaires des modifications du rapport molaire O$_2$·$^-$ / NO·

**Figure 4 :** La figure 4 représente les réactions et modifications post-traductionnelles provoquées par les ROS et RNS formées en fonction du rapport molaire NO·/ $O_2$·⁻

**Figure 5 :** La figure 5 représente la courbe d'étalonnage du dosage ELISA de l'albumine nitrée. Ce dosage utilise un anticorps de capture polyclonal anti-nitrotyrosine et un anticorps de détection anti-albumine humaine.

**Figure 6 :** la figure 6 représente les corrélations entre les valeurs obtenues pour les dosages d'albumine nitrée, effectués en triple, dans 192 plasmas d'enfants nouveau-nés âgés de 0 à 5 jours.

**Figure 7 :** La figure 7 représente la courbe dose-réponse de détection de l'albumine nitrée dans un ELISA où l'anticorps 13H10 est utilisé en tant qu'anticorps de capture et un anticorps polyclonal anti-albumine humaine conjugué à la peroxydase (HRP) est utilisé en tant qu'anticorps de détection. L'axe des abscisses (log(x)) représente la concentration en albumine et l'axe des ordonnées représente la densité optique (DO) à 450 nm.

**Figure 8 :** La figure 8 représente là densité optique (DO) mesurée à 450 nm en fonction de la concentration en albumine nitrée dans un ELISA où l'anticorps 13H10 est utilisé en tant qu'anticorps de capture et un anticorps polyclonal anti-albumine humaine conjugué à la peroxydase (HRP) est utilisé en tant qu'anticorps de détection et de sérum humain. L'axe des abscisses (log(x)) représente la concentration en albumine nitrée et l'axe des ordonnées représente la densité optique (DO) à 450 nm.
Les quantités de sérum ajoutées sont également indiquées sur la figure.

**Figures 9 et 10 :** Les figures 9 et 10 représentent la densité optique (DO) mesurée à 450 nm en fonction de la concentration en albumine nitrée dans un ELISA où l'anticorps 13H10 est utilisé en tant qu'anticorps de capture et un anticorps polyclonal anti-albumine humaine conjugué à la peroxydase (HRP) est utilisé en tant qu'anticorps de détection et en présence de sérum humain réduit. L'axe des abscisses (log(x)) représente la concentration en albumine nitrée et l'axe des ordonnées représente la densité optique (DO) à 450 nm.
Les quantités de sérum ajoutées sont également indiquées sur les figures.

**Figure 11 :** La figure 11 représente la densité optique (DO) mesurée à 450 nm en fonction de la concentration en albumine nitrée dans un ELISA où l'anticorps 13H10 est utilisé en tant qu'anticorps de capture et un anticorps polyclonal anti-albumine humaine conjugué à la peroxydase (HRP) est utilisé en tant qu'anticorps de détection et en présence d'albumine humaine réduite. L'axe des abscisses (log(x)) représente la concentration en albumine nitrée et l'axe des ordonnées représente la densité optique (DO) à 450 nm.
Les concentrations d'albumine humaine réduite ajoutées sont également indiquées sur la figure.

**Figure 12 :** La figure 12 représente l'absorbance de en fonction de la concentration en albumine nitrée dans un ELISA où un anticorps polyclonal anti-albumine humaine est utilisé en tant qu'anticorps de capture et l'anticorps 13H10 conjugué à la peroxydase (HRP) est utilisé comme anticorps de détection. L'axe des abscisses (x) représente la concentration en albumine nitrée et l'axe des ordonnées représente la densité optique (DO) à 450 nm.
Les quantités d'anticorps polyclonal ajouté pour la détection sont é0galement indiquées sur la figure.

**Figure 13 :** La figure 13 représente l'absorbance de en fonction du facteur de dilution de l'anticorps 13H10 pour la détection de l'albumine nitrée dans des échantillons de sérum humains. L'axe des abscisses (x) représente le facteur de dilution de l'anticorps et l'axe des ordonnées représente la densité optique (DO) à 450 nm.

**Figures 14A et B :** Les figures 14A et B représentent la corrélation entre la lactacidémie artérielle et la concentration en albumine nitrée plasmatique durant les premières heures de la vie (Figure 14A) et à J1 (Figure 14B).

**Figures 15A-C :** Les figures 15 A-C représentent les concentrations en albumine nitrée (exprimées en médianes, $25^{eme}$- $75^{eme}$ percentiles et $10^{eme}$ - $90^{eme}$ percentiles) durant les premières heures de la vie (Figure 15A), à J1 (Figure 15 B) at et à J4 (Figure 15 C), correspondant au statut neurologique des nouveaux nés (normaux (NE 0) ou NE légère (NE 1) versus NE modérée (NE 2) à sévère (NE 3)).

**Figure 16:** La figure 16 représente la concentration en albumine nitre au jour J1 (exprimée en médiane, 25 - 75 percentiles and 10 - 90 percentiles) correspondant au statut neurologique des nouveaux nés (normaux (NE 0) ou NE légère (NE 1) versus NE modérée (NE 2) à sévère (NE 3)). L'astérisque représente une valeur de significativité p = 0.01

**EXEMPLES : PARTIE EXPERIMENTALE**

**Exemple 1** *Nitration in vitro* **de l'Albumine.**

**Matériels :**

Albumine humaine (> 98%, Fluka)
Tetranitrométhane (Aldrich)
Tris (Sigma)
Dimethylsulfoxyde (> 99.5%, Fluka)

**Méthode:**

L'albumine est dissoute dans une solution de Tris (50 mM, pH 8.0) à une concentration de 0.1 mM.

Le tetranitrométhane est dissous dans du dimethylsulfoxyde à une concentration de 800 mM et stocké à -20 °C.

Du tetranitrométhane dissous dans du dimethylsulfoxyde est ajouté à la solution d'albumine sous agitation pour obtenir une concentration finale de 2 mM.

Cette solution est incubée à 20 °C pendant 12 h sous légère agitation à l'obscurité.

Après nitration, la solution est congelée à -80°C et lyophilisée afin d'éliminer le tetranitrométhane résiduel et d'assurer une conservation optimale de l'albumine nitrée.

La méthode de nitration a été modifiée d'après Malan, P. G., et al. (1970) Biochemistry 9(16), 3205-3214 et Sokolovsky, et al. (1966) Biochemistry 5(11), 3582-3589.

**Exemple 2 : Nitration** *in vitro* **de l'Insuline.**

**Matériels :**

Insuline bovine (I 1882, Sigma, ≥25 USP units/mg)
Tetranitrométhane (Aldrich)
Tris (Sigma)
Dimethylsulfoxyde (> 99.5%, Fluka)

**Méthode:**

L'insuline est dissoute dans une solution de Tris (50 mM, pH 8.0) à une concentration de 1 mM.

Le tetranitrométhane est dissous dans de l'éthanol absolu à une concentration de 800 mM et stocké à -20 °C.

Du tetranitrométhane dissous dans du dimethylsulfoxyde est ajouté à la solution d'insuline sous agitation pour obtenir une concentration finale de 20 mM.

Cette solution est incubée à 20 °C pendant 12 h sous légère agitation à l'obscurité (adapté de Morris et al. Biochemistry, 1970, 9(20) 3930-3937 et Carpenter et al. Biochemistry, 1980, 19(25) 5926-5931.

Après nitration, la solution est congelée à -80°C et lyophilisée afin d'éliminer le tetranitrométhane résiduel et d'assurer une conservation optimale de l'insuline nitrée.

**Exemple 3 : Nitration** *in vitro* **de L'Hémoglobine**

**Matériels :**

Hémoglobine bovine (H2500 Sigma)
Tetranitrométhane (Aldrich)
Tris (Sigma)
Dimethylsulfoxyde (> 99.5%, Fluka)

**Méthode:**

L'hémoglobine est dissoute dans du tampon phosphate 75 mM/carbonate 25 mM, pH 7.5 saturé en $CO_2$, à une concentration de 0.1 mM.

Le tetranitrométhane est dissous dans de l'éthanol absolu à une concentration de 800 mM et stocké à -20 °C.

Du tetranitrométhane dissous dans du dimethylsulfoxyde est ajouté à la solution d'hémoglobine sous agitation pour obtenir une concentration finale de 4 mM.

Cette solution est incubée à 20 °C pendant 12 h sous légère agitation à l'obscurité.

Après nitration, la solution est congelée à -80°C et lyophilisée afin d'éliminer le tetranitrométhane résiduel et d'assurer une conservation optimale de l'hémoglobine nitrée.

La méthode de nitration a été modifiée d'après Pietraforte, D.,et al. (2003) Amino Acids 25(3-4), 341-350, Pietraforte, D., et al. (2001) Biochemistry 40(50), 15300-15309 et Minetti, M., et al. (2000) Biochemistry 39(22), 6689-6697.

**Exemple 4 : Mise en évidence de protéines plasmatiques nitrées**

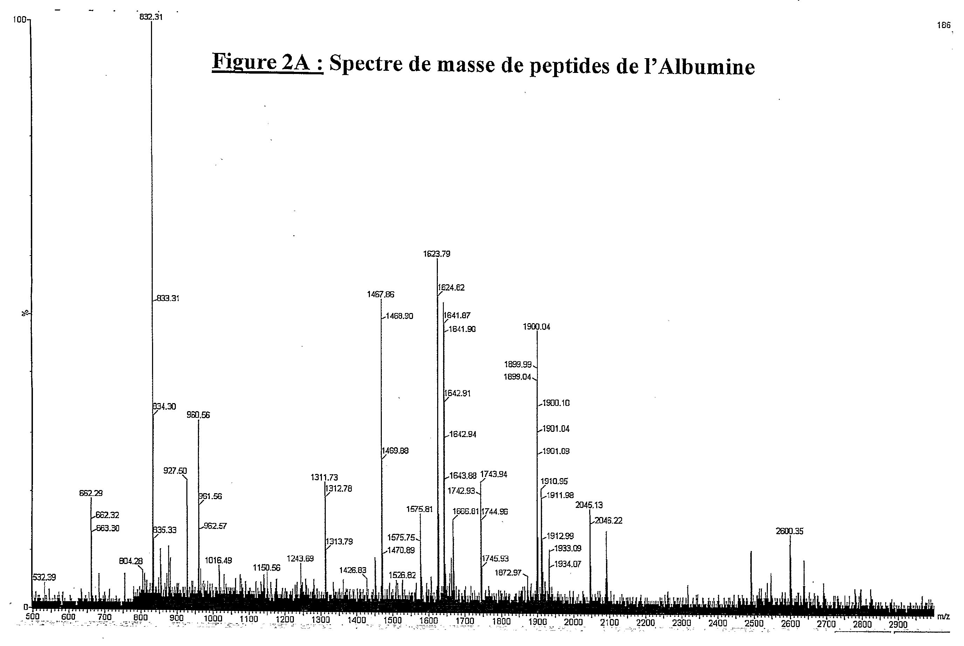

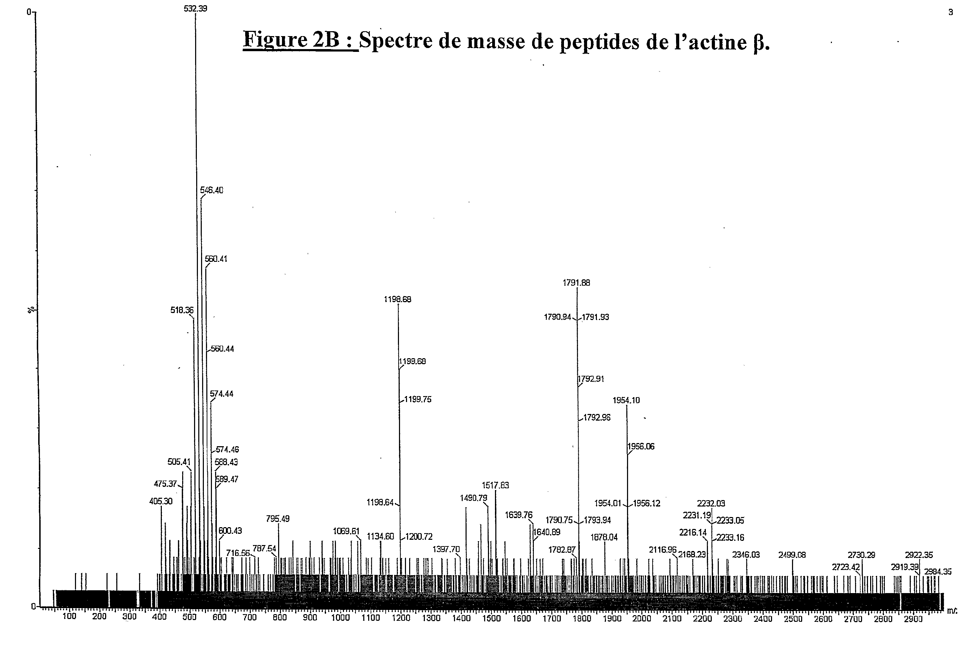

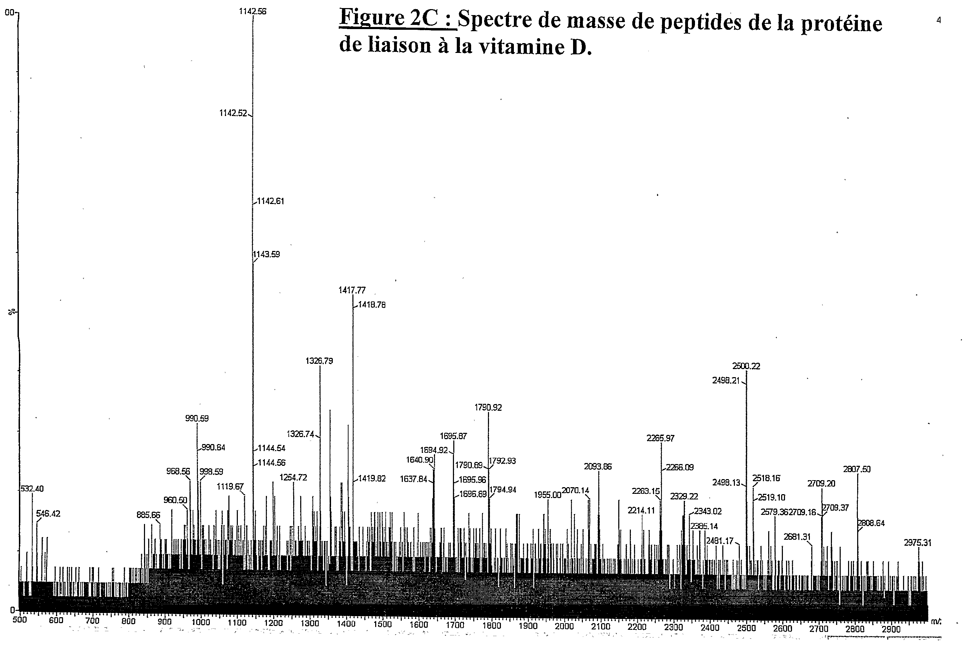

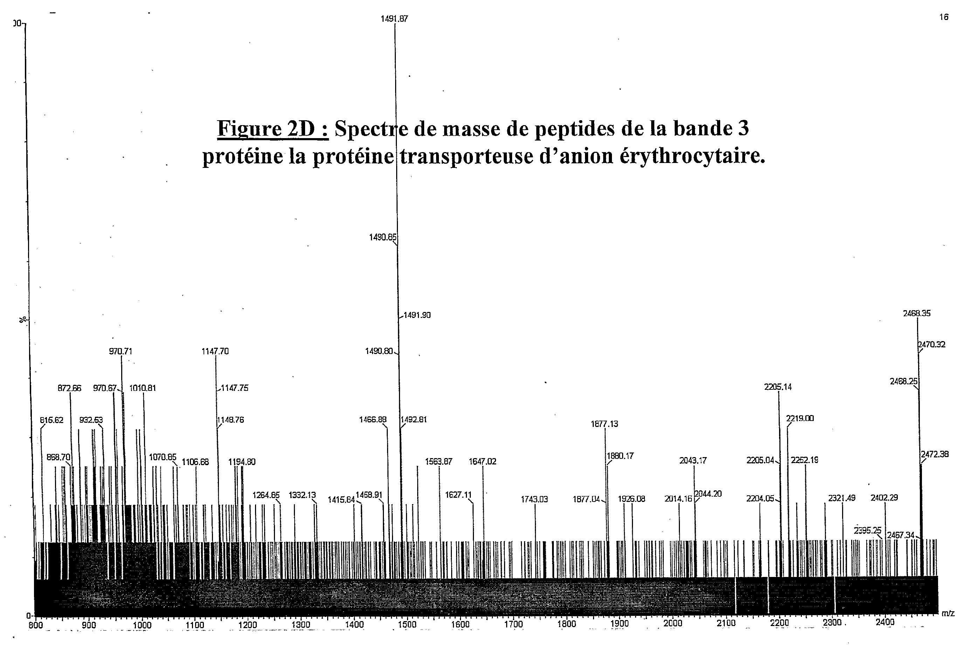

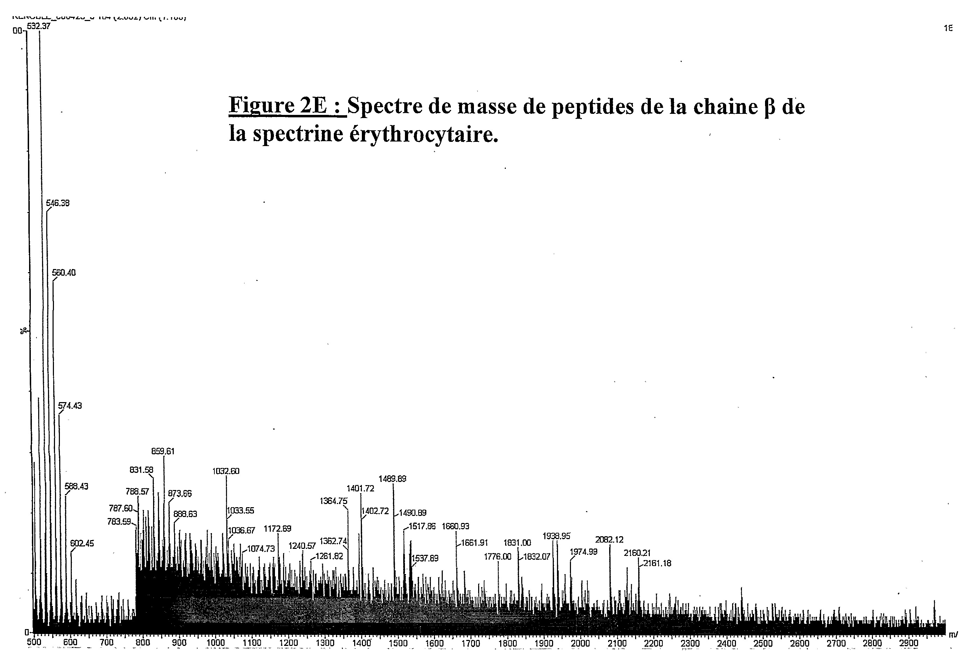

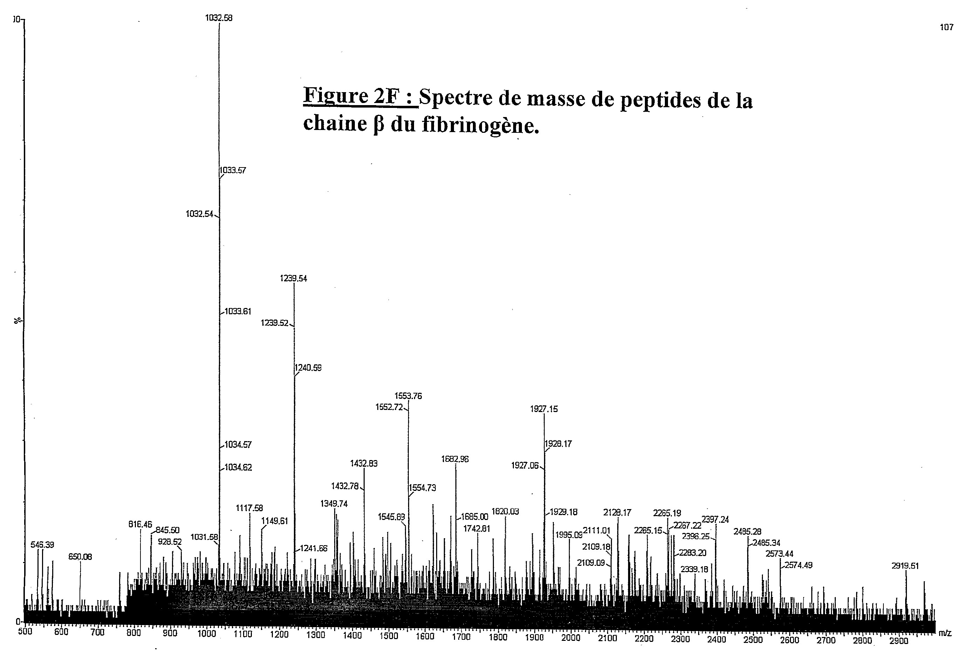

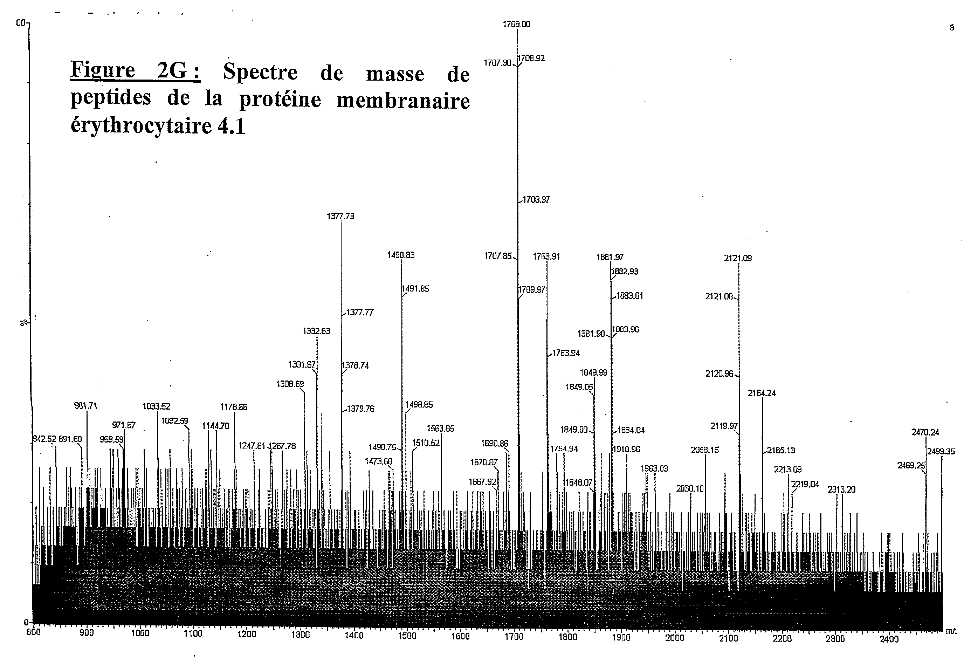

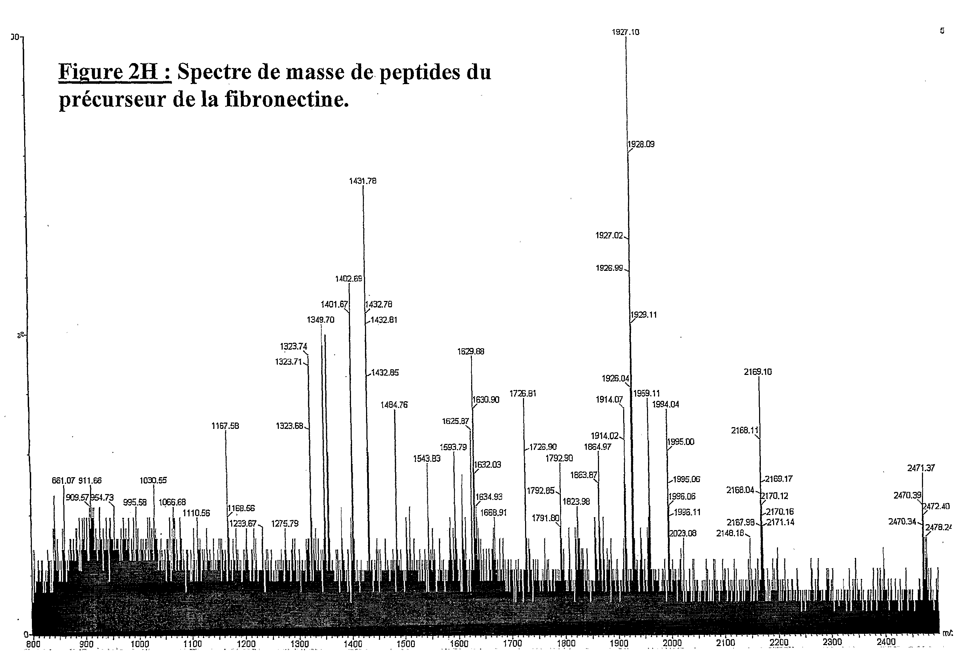

Les figures 1 et 2 illustrent cet exemple

**Matériels :**

Plasma humain
Anti-nitrotyrosine polyclonal purifié par affinité sur colonne de 3-nitrotyrosine
CarboLink Gel et AminoLink Gel (Pierce)

**Echantillons de plasma**

Les échantillons de sang ont été collectés sur tube EDTA dans le cadre d'une étude approuvée par le Comité d'Ethique concerné, soit dans le cadre du bilan d'individus sains soit dans le cadre de bilans diagnostiques chez des individus ayant souffert d'asphyxie.

Les échantillons ont été conservés dans de la glace et centrifugés à 4°C dans l'heure. Les plasmas obtenus ont été stockés à -20°C avant leur analyse. Tous les échantillons ont été analysés moins de 6 mois après leur prélèvement.

**Immunoprécipitation des protéines nitrées**

L'anticorps anti-nitrotyrosine polyclonal de lapin obtenu par immunisation avec de la KLH nitrée, est purifié par affinité sur une colonne de 3-nitrotyrosine couplée à l'agarose (AminoLink) par le groupement aminé.

L'anticorps purifié est ensuite couplé de façon covalente à de l'agarose par l'intermédiaire de ses résidus glucidiques oxydés (carbonyles) avec les groupements hydrazides greffés sur l'agarose (CarboLink). Les liaisons hydrazones ainsi formées sont stabilisées par réduction au cyanoborohydrure de sodium ($NaCNBH_3$).

Les échantillons de plasma sont incubés pendant 14 h à 4°C sous agitation légère avec les billes d'anti-nitrotyrosine-agarose et lavées ensuite 6 fois avec du PBS/triton X-100 0,1 %. Les billes sont reprises dans un volume équivalent de tampon de Laemmli 2X, chauffées 5 min à 60°C et chargées sur un gel SDS polyacrylamide de 10% d'une Épaisseur de 1,0 mm. Des marqueurs de poids moléculaire sont chargés afin de pouvoir estimer la masse des protéines détectées.

A la fin de la migration, le gel est fixé et coloré au Bleu de Coomassie colloïdal (cf. figure 1).

**Identification des protéines présentes sur le gel**

Les bandes visualisées par coloration au Bleu de Coomassie colloïdal sont excisées et congelées à -80°C.

Elles sont ensuite digérées à la trypsine et analysées par spectrométrie de masse (MALDI-QTOF). Les spectres de masse permettant l'identification des peptides des protéines identifiées sont représentés par les figures 2A, 2B, 2C, 2D, 2E, 2F, 2G et 2H.

Dans les échantillons plasmatiques, les inventeurs ont trouvé l'albumine, la protéine de liaison à la vitamine D (Vitamin D Binding Protein ; VDBP), l'actine β, la protéine bande 3 du transporteur d'anion érythrocytaire, la chaine P de la spectrine érythrocytaire, le précurseur de la fibronectine + la chaine β du fibrinogène et la protéine de la membrane érythrocytaire bande 4.1.

**Interprétation**

Les résultats permettent la visualisation et l'identification de ces protéines nitrées, sous la forme de bandes colorées, dans le plasma de ces sujets. On voit que l'intensité des bandes varie selon les sujets, indiquant un taux de nitration variable des protéines d'un sujet à l'autre. Parmi les protéines visibles sur le gel, les suivantes ont pu être identifiées par spectrométrie de masse : l'albumine, la protéine de liaison à la vitamine D (Vitamin D Binding Protein ; VDBP), l'actine β, la protéine bande 3 du transporteur d'anion érythrocytaire, la chaine β de la spectrine érythrocytaire, le précurseur de la fibronectine + la chaine β du fibrinogène et la protéine de la membrane érythrocytaire bande 4.1.

**Exemple 5 : Evaluation de la nitration de l'albumine chez des patients atteints d'insuffisance cardiaque.**

**Introduction :**

Il est démontré que l'insuffisance cardiaque (IC) s'accompagne d'une augmentation du stress oxydant et que l'augmentation de ce stress est corrélée avec le stade de la maladie [Sorescu, D. and K.K. Griendling, Reactive oxygen species, mitochondria, and NAD(P)H oxidases in the development and progression of heart failure. Congest Heart Fail, 2002. 8(3): p. 132-40*; White, M., et al., Increased systemic inflammation and oxidative stress in patients with worsening congestive heart failure: improvement after short-term inotropic support. Clin Sci (Lond), 2006. 110(4): p. 483-9; Mallat, Z., et al., Elevated levels of 8-iso-prostaglandin F2alpha in pericardial fluid of patients with heart failure: a potential role for in vivo oxidant stress in ventricular dilatation and progression to heart failure. Circulation, 1998. 97(16): p. 1536-9; Dhalla, A.K., M.F. Hill, and P.K. Singal, Role of oxidative stress in transition of hypertrophy to heart failure. J Am Coll Cardiol, 1996. 28(2): p. 506-14; Ferrari, R., et al., Oxidative stress during myocardial ischaemia and heart failure. Eur Heart J, 1998. 19 Suppl B: p. B2-11*]. Il semble évident que l'hypoperfusion et l'hypoxie relative des tissus, résultant de l'IC, induit un stress oxydant. Cependant, l'idée selon laquelle une augmentation du stress oxydant pourrait être à l'origine de la progression de l'IC, ou du moins y contribuer, gagne du terrain. Ce stress oxydant pourrait, comme pour d'autres maladies chroniques, être d'origine inflammatoire systémique [ Cotter, G., et al., Acute heart failure: a novel approach to its pathogenesis and treatment. Eur J Heart Fail, 2002. 4(3): p. 227-34*; Mann, D.L., Inflammatory mediators and the failing heart: past, present, and the foreseeable future. Circ Res, 2002. 91(11): p. 988-98; Yndestad, A., et al., Systemic inflammation in heart failure--the whys and wherefores. Heart Fail Rev, 2006. 11(1): p. 83-92; Tousoulis, D., et al., Statins in heart failure. Beyond the lipid lowering effect. Int J Cardiol, 2007. 115(2): p. 144-50*].

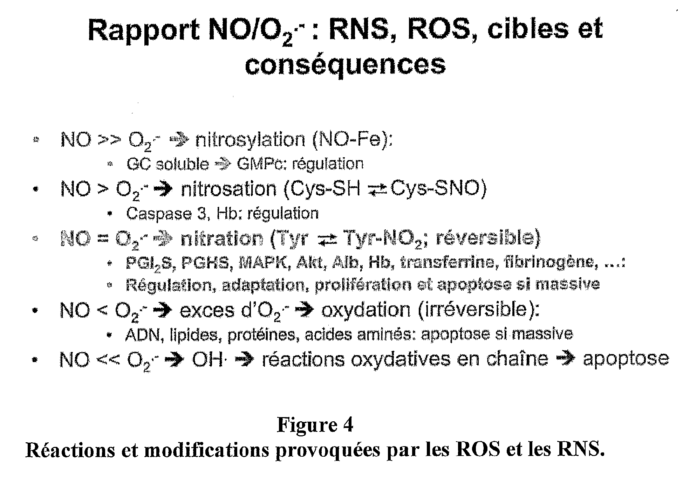

La nitrosation induite par le $NO^+$ et la nitration provoquée par le $ONOO^-$ sont donc des évènements qui surviennent avant l'oxydation. Ces modifications entraînent, comme nous l'avons montré pour les MAP kinases et PKB/Akt, des changements fonctionnels au niveau d'enzymes et de protéines structurales et de ce fait des effets métaboliques et trophiques [ Frein, D., et al., Redox regulation: a new challenge for pharmacology. Biochem Pharmacol, 2005. 70(6): p. 811-23; Ullrich, V. and R. Kissner, Redox signaling: bioinorganic chemistry at its best. J Inorg Biochem, 2006. 100(12): p. 2079-86; Pinzar, E., et al., Angiotensin II induces tyrosine nitration and activation of ERK1/2 in vascular smooth muscle cells. FEBS Lett, 2005. 579(22): p. 5100-4; Lokuta, A.J., et al., Increased nitration of sarcoplasmic reticulum Ca2+-ATPase in human heart failure. Circulation, 2005. 111(8): p. 988-95] (figure 4).

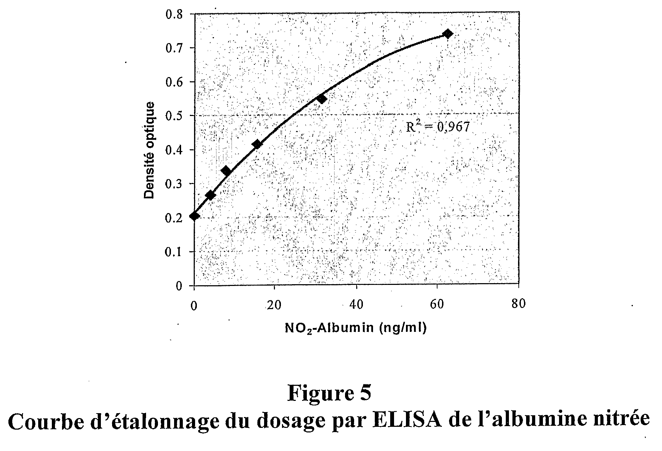

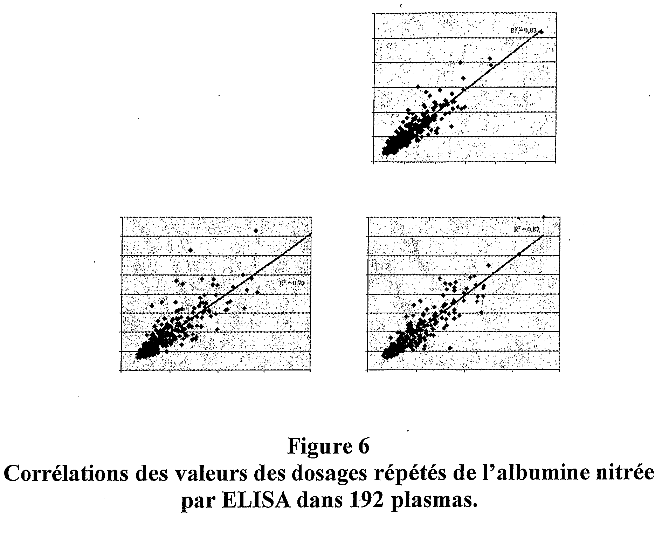

Les deux autres marqueurs disponibles pour le stress nitrant, la nitrotyrosine plasmatique et le NHPA urinaire n'étant pas fiables *[*Ryberg, H. and K Caidahl, Chromatographic and mass spectrometric methods for quantitative determination of 3-nitrotyrosine in biological samples and their application to human samples. J Chromatogr B Analyt Technol Biomed Life Sci, 2007; Pannala, A.S., et al., pH-dependent nitration of para-hydroxyphenylacetic acid in the stomach. Free Radic Biol Med, 2006. 41(6): p. 896-901*]*, l'albumine nitrée semble donc constituer un nouveau marqueur spécifique et unique de ce stress et pourrait à ce titre refléter la capacité de l'organisme à faire face au stress oxydant auquel il est confronté. Afin de vérifier cette hypothèse, un nouveau marqueur plasmatique potentiel de nitration est proposé, l'albumine nitrée dont le dosage par ELISA a été mis au point (figures 5 et 6).

La population d'intérêt dans le cadre de cette étude est celle des insuffisants cardiaques représentatifs de la population générale des deux régions participant au projet : Auvergne et Rhône-Alpes.

Nous disposons pour cela d'une part de l'accès aux patients ambulatoires de la cohorte RESIC38 (environ 450 patients) et d'autre part à ceux des hôpitaux de jour (Grenoble, Lyon, Clermont-Ferrand) pour couvrir les différents stades de l'insuffisance cardiaque tels que définis par la NYHA (New York Heart Association, accessible sur http://www.resic38.org/doc/Documents/Classification-nyha.pdf). Les sujets témoins sains seront recrutés par l'intermédiaire du CIC de Grenoble. Il nous semble essentiel de faire porter cette étude sur une population représentative qui comportera de ce fait de nombreux patients âgés et atteints de co-morbidités, mais dont les résultats seront de ce fait applicables en pratique médicale courante.

Jusqu'à présent, seuls deux marqueurs biologiques pronostiques ont été validés dans l'insuffisance cardiaque: le BNP ou son précurseur le proNT-BNP ainsi que la troponine T. D'autres paramètres biologiques, en particulier des marqueurs de l'inflammation ont été étudiés récemment et semblent indiquer l'existence d'un syndrome inflammatoire dans l'insuffisance cardiaque [Yndestad, A., et al., Systemic inflammation in heart failure--the whys and wherefores. Heart Fail Rev, 2006. 11(1): p. 83-92]. L'hypothèse de l'existence d'un syndrome inflammatoire chronique associé à un certain nombre de facteurs de risques et de pathologies chroniques systémiques a fait l'objet d'un commentaire dans *Lancet* en 2007 [Fabbri, L.M. and K.F. Rabe, From COPD to chronic systemic inflammatory syndrome? Lancet, 2007. 370(9589): p. 797-9]. Parmi ces pathologies figure l'insuffisance cardiaque. Sur la base de cette hypothèse et de résultats obtenus avec des médicaments ayant des propriétés anti-inflammatoires, les auteurs suggèrent que la prise en charge de cette composante inflammatoire chronique pourrait contribuer à l'amélioration de pathologies telles le syndrome métabolique, la BPCO (bronchopathie chronique obstructive), et l'insuffisance cardiaque ainsi que des co-morbidités fréquentes chez les patients atteints.

Pour valider cette hypothèse, il faut tout d'abord en démontrer l'existence qui repose pour l'instant essentiellement sur l'élévation de la CRP (protéine C-réactive): Compte tenu du nombre de facteurs connus ayant des effets pro ou anti-inflammatoires directs ou indirects, des variations d'expression de leurs récepteurs cellulaires et solubles, une étude détaillée de ceux-ci à grande échelle semble difficilement réalisable et en tirer des conclusions quant à la résultante globale de leurs interactions impossible. Une autre approche consisterait donc à étudier des marqueurs de points de convergence des voies activées ou inhibées par l'ensemble de ces facteurs afin d'obtenir une indication précise de l'existence ou non d'un état inflammatoire. L'augmentation de la concentration d'un certain nombre de protéines plasmatiques tels la CRP (protéine C-réactive), l'orosomucoïde et le fibrinogène reflètent l'existence d'un état inflammatoire mais cette élévation est généralement transitoire même en cas de persistance de l'inflammation. Seule la CRP peut rester détectable à l'aide de techniques de dosage ultra-sensibles.

Le déclenchement d'une réponse inflammatoire par des agents non spécifiques tels les lipopolysaccharides provoque un stress oxydatif au niveau cellulaire. Ce stress oxydatif correspond à la formation de « radicaux libres » de l'oxygène et de l'azote qui induisent des modifications fonctionnelles réversibles ou non selon leur nature, des macromolécules. Ce sont ces modifications qui, surtout si elles sont irréversibles, peuvent entraîner des altérations du métabolisme, voir la mort cellulaire et de ce fait des lésions tissulaires. Comme indiqué plus haut, nombre de ces modifications ont été identifiées et leurs conséquences sont maintenant souvent connues. Il est de ce fait important de pouvoir d'une part identifier la nature des radicaux libres générés et surtout de déterminer si l'organisme a pu y faire face grâce à ses systèmes de défense naturels.

Comme dit précédemment, la production de peroxynitrite se situe à la croisée des chemins puisqu'elle est la dernière étape permettant la « captation » des ions superoxyde et qu'il est le dernier radical libre donnant lieu à des modifications post-traductionnelles réversibles (nitration des tyrosines). On peut imaginer que cette étape de nitration, réversible, des protéines constitue un « tampon » entraînant certes des adaptations ou des perturbations temporaires, mais permettant aussi de protéger l'ADN chromatinien d'oxydation et de nitration qui induiraient l'apoptose.

Le peroxynitrite et ses précurseurs étant extrêmement diffusibles, il semble logique de penser qu'une fois les mécanismes de captation et de dégradation de ceux-ci saturés, il y aura extravasation vers le milieu extracellulaire et de ce fait nitration de protéines de ce milieu, donc entre autres dans le plasma. Cette situation correspondrait à un stress nitrant « décompensé ».

Cette hypothèse est renforcée par nos premières études cliniques réalisées dans l'asphyxie perpartale et l'hypoglycémie néonatale dont il a été montré par ailleurs par d'autres qu'elles entraînent un nitration importante des protéines cellulaires en particulier au niveau cérébral[Groenendaal, F., et al., Nitrotyrosine in brain tissue of neonates after perinatal asphyxia. Arch Dis Child Fetal Neonatal Ed, 2006. 91 (6): p. F429-33*;* Groenendaal, F., et al., Nitrotyrosine in Human Neonatal Spinal Cord after Perinatal Asphyxia. Neonatology, 2007. 93(1): p. 1-6*;* Suh, S.W., et al., Hypoglycemic neuronal death and cognitive impairment are prevented by poly(ADP-ribose) polymerase inhibitors administered after hypoglycemia. J Neurosci, 2003. 23(33): p. 10681-90].

Il semble donc bien que cette nitration cellulaire « décompensée » s'accompagne d'une augmentation de la nitration de l'albumine plasmatique qui constitue un marqueur de la saturation des capacités d'absorption d'ions superoxyde et de peroxynitrite par la cellule. L'association d'une nitration intense au déclenchement de l'apoptose ayant été décrite par plusieurs auteurs[Ischiropoulos, H. and J.S. Beckman, Oxidative stress and nitration in neurodegeneration: cause, effect, or association? J Clin Invest, 2003. 111(2): p. 163-9*;* Carreras, M.C. and J.J. Poderoso, Mitochondrial nitric oxide in the signaling of cell integrated responses. Am J Physiol Cell Physiol, 2007. 292(5): p. C1569-80]*,* il semble justifié d'étudier ce paramètre dans toute pathologie s'accompagnant d'une part d'un stress oxydatif augmenté et d'autre part d'un remodelage tissulaire lié à une augmentation de l'apoptose.

**Objectifs**

**Objectif principal :**

Evaluer l'intérêt pronostique du dosage de l'albumine nitrée plasmatique par ELISA dans l'insuffisance cardiaque à l'aide d'un critère combiné comportant la mortalité et le passage à une classe NYHA supérieure.

**Critère de jugement principal :**

Il s'agit d'un critère combiné comportant la mortalité et le passage à une classe NYHA supérieure à 2 ans.

Ce paramètre est évalué en fonction des données démographiques (âge, sexe), addictions passées et actuelles (tabac, alcool), anthropométriques (taille, poids, index de masse corporelle, rapport taille/hanches, voire composition corporelle), de la classification NYHA et des paramètres cliniques classiques de l'insuffisance cardiaque (fraction d'éjection ventriculaire gauche, tension artérielle moyenne, fréquence cardiaque, fibrillation auriculaire, cardiomyopathie ischémique, données de l'épreuve d'effort cardio-respiratoires comme la $VO_2$ et les équivalents respiratoires du $CO_2$ et de l'$O_2$ si disponible dans les 6 mois précédant l'inclusion, de l'étiologie et du type de l'insuffisance cardiaque (systolique ou diastolique), des co-morbidités éventuelles (diabète, BPCO, maladies inflammatoires chroniques), des traitements préexistants ou instaurés (diurétiques, inhibiteurs de l'enzyme de conversion, antagonistes de l'angiotensine, ß-bloquants, statines, aspirine, défibrillateurs implantables, réhabilitation à l'effort), et de leur durée ainsi que de l'évolution de l'IC chez les sujets suivis Le phénotypage et le suivi de la cohorte RESIC 38 (environ 450 patients inclus) servent de référence.

Le recrutement des patients de la cohorte RESIC 38 est complété par l'hôpital de jour afin de disposer de malades des différents stades NYHA. Le recrutement des témoins se fait par l'intermédiaire du CIC de Grenoble.

L'évaluation est faite de façon comparative par rapport à une série de marqueurs pronostiques connus et dans une certaine mesure validés dans l'IC (troponine T, NT-pro-BNP, hématocrite, adiponectine), du stress oxydant (8-isoprostaglandine F2α, GSH/GSSG, vitamine E,et albumine carbonylée) et de l'inflammation (CRP ultrasensible, IL-6, récepteur soluble de l'IL-6 et IL-10)[ White, M., et al., Increased systemic inflammation and oxidative stress in patients with worsening congestive heart failure: improvement after short-term inotropic support. Clin Sci (Lond), 2006. 110(4): p. 483-9*;* Polidori, M.C., et al., Increased F2 isoprostane plasma levels in patients with congestive heart failure are correlated with antioxidant status and disease severity. J Card Fail, 2004. 10(4): p. 334-8*;* Adamopoulos, S., J.T. Parissis, and D.T. Kremastinos, A glossary of circulating cytokines in chronic heart failure. Eur JHeart Fail, 2001. 3(5): p. 517-26*;* Anand, I.S., et al., C-reactive protein in heart failure: prognostic value and the effect of valsartan. Circulation, 2005. 112(10): p. 1428-34*;* de Denus, S., C. Pharand, and D.R. Williamson, Brain natriuretic peptide in the management of heart failure: the versatile neurohormone. Chest, 2004. 125(2): p. 652-68*;* Kistorp, C., et al., N-terminal pro-brain natriuretic peptide, C-reactive protein, and urinary albumin levels as predictors of mortality and cardiovascular events in older adults. Jama, 2005. 293(13): p. 1609-16*;* Moskowitz, R. and M Kukin, Oxidative stress and congestive heart failure. Congest Heart Fail, 1999. 5(4): p. 153-163*;* Mariani, E., et al., Oxidative stress in brain aging, neurodegenerative and vascular diseases: an overview. J Chromatogr B Analyt Technol Biomed Life Sci, 2005. 827(1): p. 65-75*;* Hall, C., Essential biochemistry and physiology of (NT-pro)BNP. Eur J Heart Fail, 2004. 6(3): p. 257-60*;* Antman, E.M., Decision making with cardiac troponin tests. N Engl J Med, 2002. 346(26): p. 2079-82*;* Kistorp, C., et al., Plasma adiponectin, body mass index, and mortality in patients with chronic heart failure. Circulation, 2005. 112(12): p. 1756-62*].*

**Objectif 2 :**

Evaluation de la valeur prédictive de l'albumine nitrée plasmatique sur la survenue de co-morbités.

**Critères de jugements :**

Corrélation entre la concentration de l'albumine nitrée plasmatique dosée lors de l'inclusion et à 1 an et la survenue de pathologies associées (syndrome métabolique, BPCO, diabète, athérosclérose,..).

**Objectif 3 :**

Evaluation de la valeur et le cas échéant de la sensibilité de l'albumine nitrée plasmatique comme marqueur d'un état inflammatoire chronique.

**Critères de jugements :**

Corrélation entre la concentration de l'albumine nitrée plasmatique et des paramètres inflammatoires reconnus : CRP ultra sensible, IL-6 et son récepteur soluble, IL-10.

**Objectif 4 :**

Evaluation de la valeur et le cas échéant de la sensibilité de l'albumine nitrée plasmatique comme marqueur du stress oxydatif « intermédiaire ».

**Critères de jugements :**

Corrélation entre la concentration de l'albumine nitrée plasmatique et des paramètres du stress oxydatif correspondant à différents stades de celui-ci :

- GSH/GSSG: oxydation des thiols : stade "précoce"
- 8-isoprostaglandine $F_{2\alpha}$ et albumine carbonylée: peroxydation des lipides et oxydation des protéines : stade "avancé"
- vitamine E : anti-oxydants

**Procédures d'investigation menées et différences par rapport à la prise en charge habituelle**

En dehors de la prise de sang à l'inclusion et à 1 an, il n'y a pas de procédures supplémentaires par rapport à la prise en charge habituelle des patients.

**CARACTERISTIQUES DES SUJETS**

**Recrutement**

Les patients sont recrutés lors des consultations spécialisées de cardiologie. Ils sont alors sollicités pour participation à l'étude.

Le recrutement des patients de la cohorte RESIC 38 est complété par l'hôpital de jour afin de disposer de malades des différents stades de la classification de la NYHA. Le recrutement des témoins se fait par l'intermédiaire du CIC de Grenoble.

Sont proposés pour l'étude les sujets répondant à chacun des critères suivants :

- Age compris entre 18 et 90 ans
- Personne affiliée à la sécurité sociale ou bénéficiaire d'un tel régime
- Insuffisants cardiaques de classe NYHA II à IV

Ne peuvent pas être inclus les sujets répondant à au moins un des critères suivants :

- Pathologie non reliée avec l'insuffisance cardiaque (ex : maladie neuro-musculaire évoluée, cancer métastatique de la prostate) et engageant le pronostique vital dans les 6 mois.
- Femme enceinte, parturiente, mère qui allaite
- Personne privée de liberté par décision judiciaire ou administrative, personne faisant l'objet d'une mesure de protection légale
- Personne inapte à répondre aux questions

**VARIABLES MESURÉES ET MÉTHODES DE MESURE**

- Age, sexe
- Addictions passées et actuelles (tabac, alcool)
- Etiologie de la cardiopathie
- Classe NYHA
- Antécédents, co-morbidités
- Traitements en cours
- Tension artérielle, pouls
- Périmètre de marche de 6 min
- Indice de masse corporelle
- Tour de taille, hanches et bassin
- Hospitalisations en rapport avec l'insuffisance cardiaque

***Paramètres para-cliniques***

Les examens suivants doivent dater de moins de trois mois par rapport à la date prévue pour les différentes visites.

- Echo-doppler cardiaque et carotidien
- ECG
- Spirométrie (VEMS, CV) et gaz du sang à l'air ambiant
- Dexascann pour mesurer la composition corporelle

*Paramètres biologiques*

- Paramètres pronostiques "reconnus":

    - NT-pro-BNP: immunochimie
    - Troponine T: immunochimie
    - Hématocrite: optique
    - Adiponectine: ELISA

- Stress nitrant :

    - Albumine plasmatique nitrée: ELISA
    - Albumine plasmatique: immunochimie

- Inflammation :

    - CRP ultrasensible: immunochimie
    - IL-6 et IL-6sr: ELISA
    - IL-10: ELISA

- Stress oxydatif :

    - 8-isoprostaglandine F2☐ urinaire: ELISA
    - GSH/GSSG: enzymatique
    - Albumine carbonylée: ELISA (mise au point en cours)
    - Vitamine E: HPLC

- Etat nutritionnel :

    - Protéines plasmatiques totales: colorimétrie
    - Albumine plasmatique: immunochimie
    - Préalbumine (TTY): immunochimie

- Sérothèque : excédents de sérum et plasma aliquotés et stockés à -80°C
- Urinothèque : 50 ml d'urines seront aliquotés et stockés à -80°C

## ANALYSE STATISTIQUE DES PARAMÈTRES MESURÉS

*Calcul du nombre de sujets*

Le calcul du nombre de sujets est basé sur la comparaison non paramétrique de la mortalité entre deux groupes ; il est obtenu à partir de la formule suivante :

$$n=\frac{nA[1-SA(t)]+nB[1-SB(t)]}{2-SA(t)-SB(t)}$$

(Source : "Analyse statistique des données de survie" Catherine Hill, et coll. - Médecine-sciences Flammarion)
La "survie sans aggravation" est définie par l'absence d'événement (décès, passage à une classe NYHA supérieure à 2 ans.)
Compte tenu d'une amélioration attendue du critère de jugement principal ("survie sans aggravation ") de 25% entre les deux groupes, le nombre de sujet à inclure est de 48 par groupe, soit 192 sujets pour les 4 quartiles, avec un risque alpha de 5% et une puissance de 80%, permettant de mettre en évidence un odds-ratio de 1,7 entre quartiles.

### *Stratégie d'analyse des données*

### Seuil Statistique et conditions d'applications :

Lors d'un test, le seuil statistique ($\alpha$) retenu pour considérer une différence comme statistiquement significative sera p inférieur ou égal à 0,05.

Toutefois, pour parer à la perte de puissance qu'introduisent les comparaisons multiples sur des paramètres non indépendants, on met en oeuvre la méthode corrective de Bonferroni avec comme seuil statistique $\alpha' = \alpha / k$.

On met en oeuvre le test de Shapiro-Wilks pour démontrer la normalité des paramètres.

On met en oeuvre le test de Levene pour démontrer l'homogénéité des variances.

Lorsque que les conditions d'application des tests paramétriques ne seront pas satisfaites, des tests non paramétriques pourront être réalisés.

Pour démontrer les liaisons entre paramètres quantitatifs, le test de corrélation de Pearson est mis en oeuvre après vérification de la normalité des paramètres.

Pour démontrer les liaisons entre paramètres qualitatifs, le test du chi-deux est mis en oeuvre.

### Variables Quantitatives

Les paramètres quantitatifs pour lesquels la normalité a été admise sont décrits par la moyenne $\pm$ écart-type, l'intervalle de confiance à 95% ainsi que les $5^{ème}$ et $95^{ème}$ percentiles. Ils sont exprimés en médiane, minimum, maximum et percentiles à $5^{ème}$ et $95^{ème}$ lorsque la normalité aura été rejetée.

### Variables Qualitatives

Les paramètres qualitatifs sont exprimés en effectif et pourcentage.

Les variables catégorielles sont résumées par des statistiques descriptives à chaque temps de l'évaluation et dans chaque groupe : Effectif et fréquence.

### Populations d'analyse

Une analyse statistique globale est réalisée sur l'ensemble de la population.

### Analyse du critère principal

### - *Objectif 1:*

Evaluer l'intérêt pronostique du dosage de l'albumine nitrée plasmatique par ELISA dans l'insuffisance cardiaque à l'aide d'un critère combiné comportant la mortalité et le passage à une classe NYHA supérieure.

### - *Paramètres :*

Le critère de censure retenu est le critère combiné suivant : le décès, le passage à une classe NYHA supérieure à 2 ans.

Le critère de jugement est évalué lors des visites de suivi des patients au sein de chaque centre. Une confirmation de la mortalité est prévue à l'issue de l'étude par l'envoi de courrier aux mairies de naissance de tous les patients.

Bien qu'il s'agisse d'un paramètre quantitatif continu, la durée de survie est décrite par sa médiane et son intervalle de confiance à 95% ainsi que les quartiles, afin de tenir compte de sa distribution asymétrique.

### - *Test:*

1.1) On teste l'intérêt du paramètre (albumine nitrée plasmatique) à l'aide du modèle de Cox en univarié, après vérification des conditions d'application.

1.2) Dans un second temps, le modèle est ajusté sur les facteurs démographiques (age, sexe, BMI) et sur les facteurs pronostics de l'insuffisance cardiaque reconnus ; c'est à dire : la classe fonctionnelle NYHA, le score de test de marche, le niveau de fraction d'éjection (+ ou- de 45 %), le taux de NT-pro BNP plasmatique.

1.3) La méthode de Kaplan-Meier est mise en oeuvre pour estimer les courbes de survie de chaque quartile pour les facteurs significatifs dans le modèle de Cox ajusté.

La comparaison des courbes de survie est réalisée à l'aide du test du Log-Rank ajusté sur l'ensemble des strates (facteurs pronostiques connus).

**Résultats**

Le but de cette étude était de tester l'hypothèse d'une augmentation de la production de peroxynitrite chez des enfants asphyxiés et de vérifier si la concentration en albumine nitrée est corrélée au statut clinique de ces enfants. Pour cela, l'albumine nitrée a été dosée dans 114 échantillons de plasma collectés dans les premières heures de la vie (PHV), au jour 1 (J1) et au jour 4 (J4) chez 48 enfants nés à terme et ayant subi une asphyxie périnatale. Le dosage de l'albumine nitrée a été corrélé avec l'avancement de pathologies néonatales neurologiques (encéphalopathie) et systémiques (coagulopathie et lésions rénales, hépatiques et cardiaques). 17 patients ont développé une encéphalopathie modérée à sévère. Les caractéristiques principales des patients sont représentées dans le tableau I suivant.

Tableau I : Caractéristiques cliniques et biochimiques des patients étudiés. Les variables sont exprimées en moyenne $\pm$ écart type). IPPV: pression de ventilation positive intermittente.

| Valeur/Nombre | |
|---|---|
| Variables des premières heures | |
| pH de l'artère ombilicale | $7,08 \pm 0,16$ |
| Déficit en base de l'artère ombilicale | $10,1 \pm 6,6$ |
| Score Apgar 5 minutes | $3,0 \pm 1,9$ |
| IPPV à la naissance (n) | 41 |
| Premier pH artériel | $7,22 \pm 0,16$ |
| Premier déficit en base artérielle (mmol/1) | $13,3 \pm 4,3$ |
| Premier lactate artériel (mmol/1) | $11,4 \pm 4,9$ |
| Mesures cliniques | |
| Encéphalopathies (n) | 27 |
| Normal (pas d'encéphalopathie) | 21 |
| légères | 10 |
| modérées | 10 |
| sévères | 7 |
| Lésions cardiaques (n) | 12 |
| Lésions rénales(n) | 8 |
| Lésions hépatiques(n) | 11 |
| Coagulopathie (n) | 11 |

**Corrélations entre la concentration plasmatique en albumine nitrée et les variables continues**.

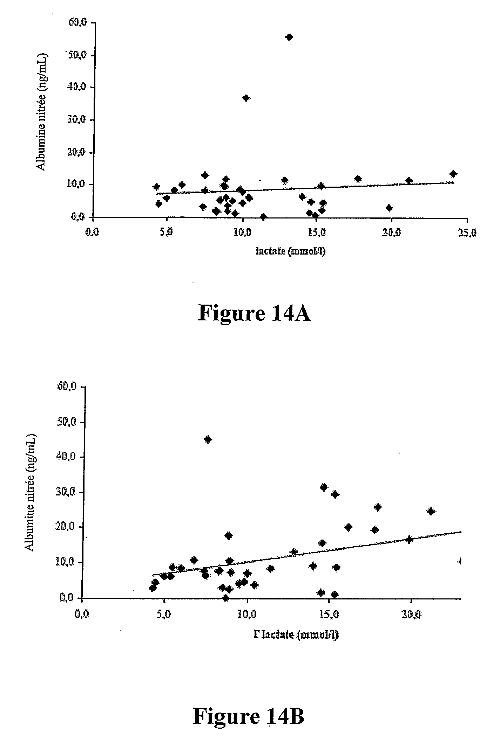

L'albumine nitre PHV est seulement corrélée avec la créatinine à J1 (r = 0.38, p<0.05). L'albumine nitrée à J1 est inversement corrélée aux scores d'Apgar (r = -0.34, -0.47 et -0.33 pour les scores d'Apgar à 1, 5 and 10 minutes, respectivement, p<0.05) et avec le pH PHV (r = -0.41, p = 0.01), et directement corrélé avec la lactacidémie PHV (r = 0.47, p<0.01, figures 14A et 14B). Aucune corrélation significative n'a été trouvée entre les concentrations en albumine nitrée et les concentrations en albumine.

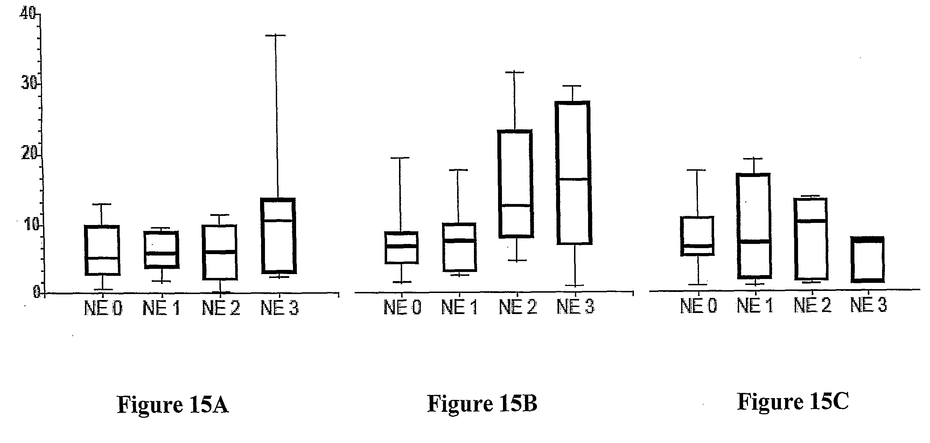

Les concentrations en albumine nitrée correspondant aux données cliniques sont présentées dans le tableau I. La médiane de concentration en albumine nitrée (25-75eme percentiles) à J1 augmente avec la sévérité de l'encéphalopathie néonatale (NE) : 7.0 (5.3-8.6) ng/ml chez des enfants ne développant pas de NE, 8.3 (4.7-10.4) ng/ml chez des enfants avec une NE légère, 13.1 (8.4-20.3) ng/ml dans le cas de NE légère and 16.7 (13.1-24.8) ng/ml dans le cas d'une NE sévère ($\chi^2$ = 7.23, p<0.05, figures 15A-C).

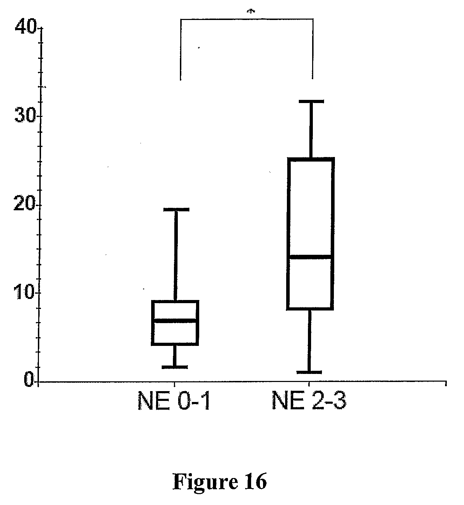

Les différences en concentrations d'albumine nitrée entre les 4 sous- groupes de NE atteignent un taux de significativité seulement quand la correction de Bonferroni n'est pas appliquée aux différences en albumine nitrée a J1 entre la NE modérée et pas de NE (z-score: 2.2) et entre NE sévère et pas de NE (z-score: 1.98). L'albumine nitrée plasmatique Plasma à J1 était significativement plus élevée chez les nouveau-nés souffrant de forme modérée de NE que chez des nouveau-nés souffrant de forme légère de NE ou sans NE (médiane et 25-75eme percentiles: 14.4 and 8.4-23.7 ng/ml versus 7.3 and 4.4-9.2 ng/ml, respectivement, $\chi^2$ = 6.14, p = 0.01, figure 16).

Les taux d'albumine nitrée au jour 1 sont augmentés chez les patients qui développent une forme modérée ou sévère de NE comparée à celle des patients ayant un profil neurologique normal ou qui développent une NE légère (médiane: 14.4 ng/ml versus 7.3 ng/ml, respectivement, p = 0.01).

A l'opposé, la concentration en albumine nitrée à J1 n'est pas associée à des complications systémiques. Les concentrations en albumine nitrée aux premières heures de la vie et à J4 ne diffèrent pas en ce qui concerne les données neurologiques néonatales.

## Conclusion:

Les résultats indiquent qu'un stress nitrant significatif apparait au cours des asphyxies périnatales sévères.

De manière surprenante, l'albumine nitrée plasmatique apparait comme étant un bon marqueur de l'évolution neurologique des enfants à terme présentant une asphyxie périnatale.

### Exemple 6 : Obtention, criblage et caractérisation des hybridomes.

### 1- Immunisation

5 souris femelles OF1 Charles River (18-20g) ont été immunisées par injection intraveineuse et sous-cutanée d'un mélange de peptides SEQ ID NO 16 et SEQ ID NO 17, les deux peptides étant couplés à l'ovalbumine en présence d'adjuvant complet de Freund. 3 souris (souris 1 à 3) ont reçu 5O$\mu$g du mélange des peptides et 2 souris (souris 4 et 5) ont reçu 10$\mu$g du mélange des peptides.

3 semaines après la première injection, les souris ont été réinjectées avec le mélange des deux peptides en présence d'adjuvant incomplet de Freund (1er rappel).

3 semaines après la seconde injection, les souris ont été réinjectées avec le mélange des deux peptides en présence d'adjuvant incomplet de Freund (deuxième rappel).

1 mois après la première injection, des échantillons de sérum des souris injectées a été prélevé, et lesdits sérums ont été testés pour la présence d'anticorps dirigés contre l'albumine nitrée.

Seuls les sérum des souris 2 et 5 présentaient des anticorps reconnaissant l'albumine nitrée, et ont ainsi été conservés.

10 jours après le test du sérum, la souris 2 a reçu un rappel (boost) intra-péritonéal et intraveineux de 20 $\mu$g du mélange des peptides. Les rates ont été prélevées 3 jours après le boost.

1 mois après le test du sérum, la souris 5 a reçu un rappel (boost) intra-péritonéal et intraveineux de 10 $\mu$g du mélange des peptides. Les rates ont été prélevées 3 jours après le boost.

### 2- Fusion cellulaire

Les souris 2 et 5 ont été saignées et leur rate a été prélevée stérilement avec du DMEM (Dulbecco's Modified Eagle's Medium). Les rates ont été broyées et filtrées sur grille.

En parallèle, la cavité intraperitonéale des souris est lavée avec du DMEM, et le DMEM comprenant des macrophages est récupéré. Les macrophages ont été comptés sur lame de Malassez afin de préparer une solution à 104 macrophages / ml en milieu DMEM HAT SVF 20% ATB (DMEM, 4mM glutamine, HAT (hypoxantine 100$\mu$M, aminoptérine 0,4 $\mu$M, thymidine 16 $\mu$M), 20% sérum de veau foetal décomplémenté, 1% antibiotiques (Pénicilline/Streptomycine)).

Les splénocytes obtenus ont alors été lavés trois fois avec du DMEM
En parallèle, des cellules de myélome de souris BalB/c Sp2/O Ag14 (ATCC n° CRL 1581) sont également lavées 3 fois dans du DMEM

Les splénocytes et les cellules de myélome ont été mélangés avec un rapport splénocytes/myélome de 5/1 et centrifugés à 244g pendant 7 minutes.

Le surnageant a été éliminé et 1 ml de PEG (solution à 40% de polyéthylène glycol, PM 1500 chauffée à 37°C) a été ajouté.

Les cellules ont été centrifugées à 800 rpm (108 g) pendant 12 minutes, 10 ml de milieu DMEM HAT SVF 20% (DMEM, 4mM glutamine, HAT (hypoxantine 100$\mu$M, aminoptérine 0,4 $\mu$M, thymidine 16 $\mu$M), 20% sérum de veau foetal décomplémenté) a été ajouté lentement.

Les cellules ont été centrifugées à 1200 rpm (244 g) pendant 7 minutes, le surnageant a été éliminé et sur le culot a été ajouté un volume v de milieu DMEM HAT SVF 20% tel que :

v (en ml) = nb de splénocytes / 107 (soit 107cellules/ml)

Le tube a été laissé à température ambiante pendant 1 heure avant d'être retourné délicatement pour remettre les cellules en suspension.

Dans des plaques de 96 puits, 100 $\mu$l/puits de la solution à 104 macrophages / ml ont été ajoutés puis 100$\mu$l par puits de cellules fusionnées ont été ajoutés aux dilutions suivantes : dilution 1/10 : 3 plaques à 105 splénocytes par puits

dilution 1/20 : 5 plaques (2x 50ml) à 5 x104 splénocytes par puits
dilution 1/40 : 2 plaques à 2,5 x104 splénocytes par puits

Les plaques ont été placées à l'étuve à 37°C, 5%CO2 pendant 10 jours.

### 3- Sélection des hybridomes

Après 10 jours de culture des produits de fusion, deux tests de sélection sont réalisés :

- Test 1 : Les puits dont les cellules sont arrivées à confluence sont analysés :

  - pour les fusions issues des splénocytes de la souris 2, 164 puits ont été analysés,
  - pour les fusions issues des splénocytes de la souris 5, 407 puits ont été analysés.

  100 $\mu$l de surnageant de chacun de ces puits ont été prélevés et les surnageants ont été testés par un test ELISA pour la détection de l'anticorps recherché (cf. Criblage des surnageants des hybridomes anti-albumine nitrée).
  Après le test ELISA, les cellules sélectionnées (sécrétant des anticorps dirigés contre l'albumine nitrée) ont été passées dans 0,4 ml de milieu en puits d'une plaque de 24 puits. Lorsque les cellules commençaient à se multiplier (24 à 48 heures), 1mL du milieu DMEM HAT SVF 15% HCF 1% ATB (DMEM, 4mM glutamine, HAT (hypoxantine 100$\mu$M, aminoptérine 0,4 $\mu$M, thymidine 16 $\mu$M), 15% sérum de veau foetal décomplémenté, 1% HCF (hybridoma cloning factor macrophage-like origin), 1% antibiotiques (Pénicilline/Streptomycine)) a été ajouté.

- Test 2 : Les puits dont les cellules sont arrivées à confluence sont analysés :

  - pour les fusions issues des splénocytes de la souris 2, 1 puits de plaque 24 puits a été analysé,
  - pour les fusions issues des splénocytes de la souris 5, 6 puits de plaque 24 puits ont été analysés.

  100 $\mu$1 de surnageant de chacun de ces puits ont été prélevés et les surnageants ont été testés par un test ELISA pour la détection de l'anticorps recherché (cf. Criblage des surnageants des hybridomes anti-albumine nitrée).

### 4- Criblage des surnageants des hybridomes anti-albumine nitrée

Antigènes (Ag) utilisés: Albumine nitrée, albumine, KLH-peptide SEQ ID NO 16 ou KLH-peptide SEQ ID NO 17.

| ETAPES | CONDITIONS |
|---|---|
| <u>Coating</u> (adsorption de l'Ag.)<br>    Concentration de l'Ag<br>    Tampon | Plaque 96puits (Maxisorp, Nunc)<br>1 $\mu$g/ml<br>PBS |

(suite)

| ETAPES | CONDITIONS |
|---|---|
| Volume / puits<br>Incubation | 50 μl<br>1 nuit à température ambiante |
| Lavage : x1 | PBS - 0,05% (v/v) Tween 20 |
| *Saturation*<br>Tampon<br>Volume / puits<br>Incubation | PBS-lait 2,5% (p/v)<br>150 μl<br>1h à 25°C |
| Lavage: x1 | PBS - 0,05% (v/v) Tween 20 |
| *Anticorps à tester*<br>Surnageant de culture pur<br>Volume / puits<br>Incubation | 50 μl<br>2h à 25°C |
| Lavage : x3 | PBS - 0,05% (v/v) Tween 20 |
| *Anticorps secondaire*<br>(conjugué peroxydase)<br>Dilution<br>Tampon<br>Volume /puits<br>Incubation | Anti-IgG et IgM (115-036-044, Jackson)<br>1/10 000<br>PBS-0,05% (v/v) Tween 20-0,5% (p/v) BSA<br>50 μl<br>1h à 25°C |
| Lavage : x3 | PBS - 0,05% (v/v) Tween 20 |
| *Révélation*<br>Réactif<br>Volume /puits<br>Incubation | Tetramethylbenzidine (50-76-05, KPL, Inc.)<br>50 μl<br>10 min |
| *Arrêt de la réaction*<br>*Volume* | $H_2SO_4$ 1M ( S1526, Sigma)<br>50 μl |

**5- Isotypage des anticorps**

L'isotype des anticorps a été déterminé en utilisant le kit SouthernBiotech SBA Clonotyping System/HRP (Cliniscience, Montrouge, France) de la façon suivante :

**1. Coating des plaques**

Diluer l'anticorps anti-immunoglobulines de souris à la concentration de 5 μg/ml. Déposer 50 μl par puits et incuber 1 heure à 37°C ou 16 heures à température ambiante.

**2. Lavage**

Rincer 1 fois avec 200μl/puit de PBS-Tween20 0,05%%(v/v).

**3. Blocage**

Ajouter dans chaque puits 150 μl de PBS-Lait 2,5%(p/v) et incuber 1 heure à 37°C.

**4. Lavage**

Rincer 1 fois avec le tampon PBS-Tween20 0,05%(v/v).

**5. Préparation des échantillons d'anticorps à tester**

Diluer les surnageants de culture de l'hybridome au 1/10 en PBS-Tween20 0,05%(v/v)-BSA 0,5%(p/v). Déposer 50 $\mu$l par puits et incuber 2 heures à température ambiante.

**6. Lavage**

Rincer 3 fois avec le tampon PBS-Tween20 0,05%(v/v).

**7. Anticorps secondaire**

Déposer 50 $\mu$l par puits d'anticorps anti-souris IgA, IgG1, IgG2a, IgG2b, IgG3 ou IgM conjugués à la peroxidase (HRP) (dilués au 1/2000 en PBS-Tween20 0,05%(v/v)-BSA 0,5%(p/v)) et incuber 1 heure à température ambiante.

**8. Lavage**

Rincer 3 fois avec le tampon PBS-Tween20 0,05%(v/v).

**9. Réaction avec le substrat**

Déposer 50 $\mu$l par puits de Tetramethylbenzidine (KPL, Inc.) et incuber la plaque 10 minutes à température ambiante.

**10. Arrêt de la réaction**

Ajouter 50 $\mu$l de $H_2SO_4$ dans chaque puits et lire l'absorbance à 450 nm, au lecteur de microplaques (Dynex).

**6- Résultats**

7 hybridomes ont été conservés : 2F3, 11G6, 12F5, 12H3, 13H8, 13H10 et 15F8 en raison de leur excellente affinité pour les peptides correspondant aux séquences nitrées de l'albumine humaine (SEQ ID NO 16 et SEQ ID NO 17) et l'Alb-NO$_2$, comme indiqué dans le tableau II ci-après.

Tableau II : Densité optique et isotype des 7 clones sélectionnés.

| | **DO sur Alb-N02** | DO sur SEQ ID NO17 | DO sur SEQ ID NO 16 | Isotype |
|---|---|---|---|---|
| <u>**2F3**</u> | **1,172** | **2,211** | 0,275 | IgG 1 |
| 11G6 | **1,355** | 0,081 | 0,081 | IgM |
| 12F5 | 0,061 | 0,232 | 0,052 | IgM, IgG1, IgG2b |
| 12H3 | 0,066 | 1,006 | 0,056 | IgG1 |
| 13H8 | 0,104 | 0,063 | 1,837 | IgG1, IgM |
| <u>**13H10**</u> | **1,449** | 0,059 | **2,562** | IgG2b |
| <u>**15F8**</u> | **1,461** | 0,064 | **2,225** | IgA, IgG2b |

Des tests de spécificité sur accrochage (coating) sur albumine KLH nitrée, insuline nitrée et hémoglobine nitrée ont également été réalisés. Tous les anticorps sont négatifs sur albumine, KLH nitrée, insuline nitrée et hémoglobine nitrée.

Les clones 2F3 et 13H10 ont été conservés et clonés en réalisant des dilutions limites dans des plaques 96 puits contenant 10, 5, 3, 1 ou 5 cellules par puits en moyenne.

Les produits du clonage ont été congelé dans du milieu Milieu DMEM HT SVF 15% HCF 1% ATB (2) : DMEM, 4mM glutamine, HAT (hypoxantine 100$\mu$M, thymidine 16 $\mu$M), 15% sérum de veau foetal décomplémenté, 1% HEF (hybridoma enhancing suplement), 1% antibiotiques (Pénicilline/Streptomycine) complété avec 10% DMSO (Diméthylsulfoxide)

**Exemple 7: Validation de l'utilisation du mAb anti-alb-NO$_2$-Tyr$^{138}$ (clone 13H10) comme anticorps de capture en ELISA pour le dosage de l'albumine nitrée**

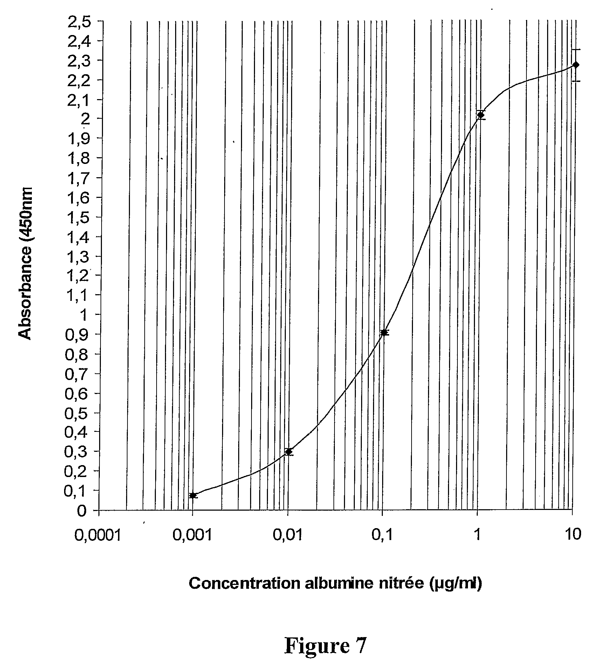

Dans cette conformation d'ELISA, l'anticorps monoclonal anti-alb-NO2-Tyr138 (clone 13H10) est utilisé pour la capture et un anticorps anti-albumine polyclonal conjugué à la peroxydase est utilisé pour la détection. Les plaques sont révélées par du TMB (3,3',5,5'-tetramethylbenzidine), un substrat chromogène de la péroxidase dont la couleur vire au bleu en présence de peroxyde d'hydrogène et dont la couleur devient jaune en présence d'acide sulfurique (arrêt de la réaction). La réaction peut être quantifiée par détection à 450 nm.
Les résultats obtenus montrent que l'anticorps monoclonal anti-alb-NO2-Tyr138 peut être utilisé comme anticorps de capture en ELISA sandwich (figure 7).
La courbe dose-réponse indique une limite de détection de l'albumine nitrée de 1 ng/ml.

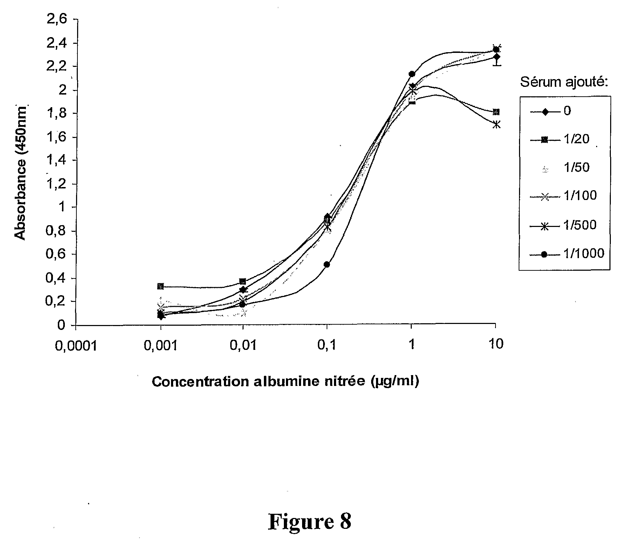

**Exemple 8 : Effet du sérum sur l'ELISA d'albumine nitrée utilisant le mAb anti-alb-NO$_2$Tyr$^{138}$ (clone 13H10) en capture (figure 8)**

Dans cette conformation d'ELISA, l'anticorps monoclonal anti-alb-NO$_2$-Tyr$^{138}$ est utilisé pour la capture et un anticorps anti-albumine polyclonal conjugué à la peroxydase est utilisé pour la détection. Les plaques sont révélées par du TMB.
Un mélange (pool) de sérum humain natif à différentes dilutions est ajouté à la gamme d'albumine nitrée. Les courbes indiquent que le sérum dilué au 1:20 n'interfère de façon significative avec le dosage de l'albumine nitré que pour des concentrations de celle-ci < 10 ng/ml. Aux dilutions plus importantes, le sérum ne montre aucune interférence significative. On notera que ce mélange de sérum contient par nature également de l'albumine nitrée « endogène ».

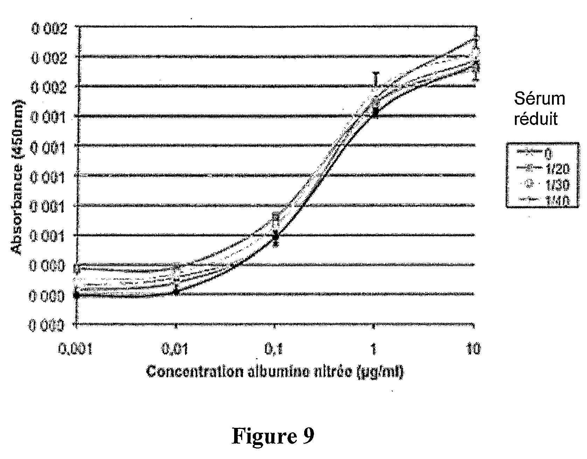

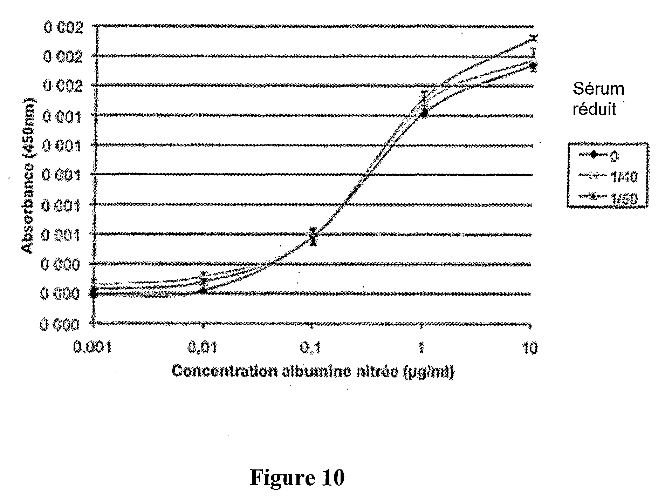

**Exemple 9 : Effet du sérum réduit sur l'ELISA d'albumine nitrée utilisant le mAb anti-alb-NO$_2$-Tyr$^{138}$ (clone 13H10) en capture (figure 9 et 10)**

Dans cette conformation d'ELISA, l'anticorps monoclonal anti-alb-NO2-Tyr138 est utilisé pour la capture et un anticorps anti-albumine polyclonal conjugué à la peroxydase est utilisé pour la détection. Les plaques sont révélées par du TMB.
Afin de s'affranchir de l'interférence de l'albumine nitrée endogène, le sérum à été réduit à l'aide d'hydrosulfite de sodium (0,1 M, pH 9.0, 1 h). Ce traitement réduit les résidus nitrotyrosine en aminotyrosines non reconnues par l'anticorps.

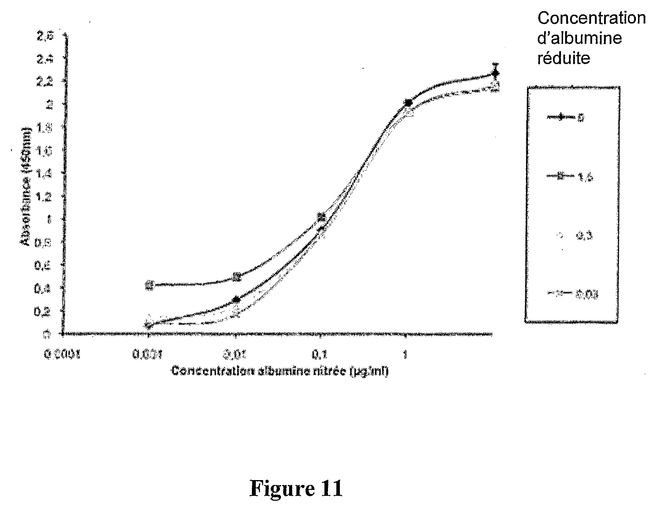

**Exemple 10 : Effet de l'albumine réduite sur l'ELISA d'albumine nitrée utilisant le mAb anti-alb-NO$_2$-Tyr$^{138}$ (clone 13H10) en capture (Figure 11)**

Dans cette conformation d'ELISA, l'anticorps monoclonal anti-alb-NO2-Tyr$^{138}$ est utilisé pour la capture et un anticorps anti-albumine polyclonal conjugué à la peroxydase est utilisé pour la détection. Les plaques sont révélées par du TMB.
De l'albumine humaine sérique réduite à l'aide d'hydrosulfite de sodium (0,1 M, pH 9.0, 1 h) est ajoutée à différentes concentrations à la gamme d'albumine nitrée.
On note que l'albumine réduite n'interfère qu'à des concentrations ≥ 150.000 fois celles de l'albumine nitrée.

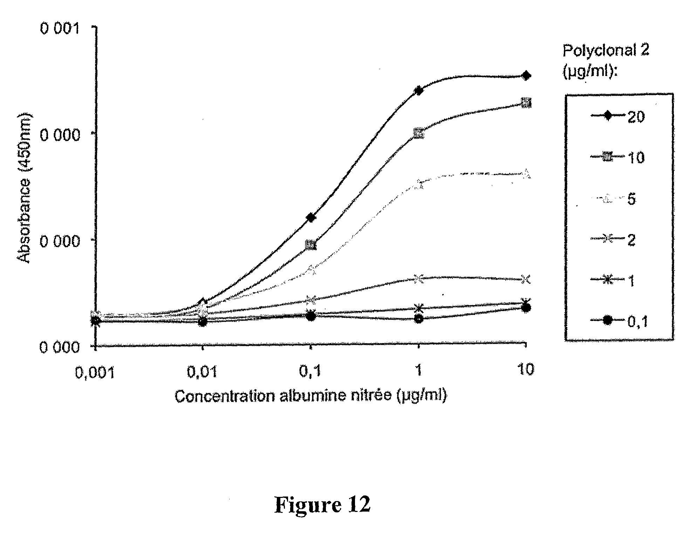

**Exemple 11 : Validation de l'utilisation du mAb anti-alb-NO2-Tyr138 (clone 13H10) comme anticorps de détection en ELISA pour le dosage de l'albumine nitrée plasmatique (Figure 12)**

Dans cette conformation d'ELISA, un anticorps anti-albumine polyclonal est utilisé pour la capture et l'anticorps monoclonal anti-alb-NO2-Tyr138 conjugué à la peroxydase est utilisé pour la détection. Les plaques sont révélées par du TMB.
Les résultats obtenus indiquent que l'anticorps monoclonal anti-alb-NO2-Tyr138 peut être utilisé comme anticorps de détection en ELISA sandwich.

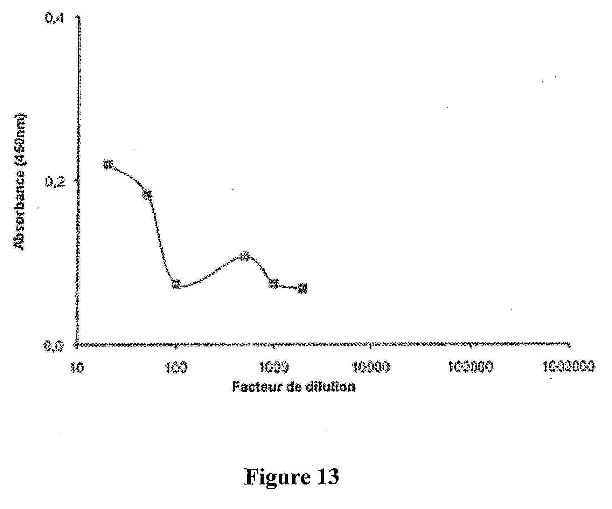

**Exemple 12 : Validation de l'utilisation du mAb anti-alb-NO2-Tyr138 (clone 13H10) en ELISA pour le dosage de l'albumine nitrée dans des sérums humains (Figure 13)**

Dans cette conformation d'ELISA, l'anticorps monoclonal anti-alb-NO2-Tyr138 est utilisé pour la capture et un anticorps anti-albumine polyclonal conjugué à la peroxydase est utilisé pour la détection. Les plaques sont révélées par du TMB.

Un mélange de sérums humains est dosé à différentes dilutions dans du PBS. Les résultats indiquent que l'albumine nitrée peut être dosée dans le sérum jusqu'à une dilution de celui-ci à 1:50.

### Exemple 13 : Anticorps dirigé contre la séquence LVRY-NO$_2$TQKAPQ qui comprend la

### nitrotyrosine en position 411 de l'albumine

Des résultats similaires à ceux décrits ci-dessus ont été obtenus avec l'anticorps monoclonal 2F3 dirigé contre la séquence LVR(Y-NO$_2$)TQKAPQ qui comprend la nitrotyrosine en position 411.

En d'autres termes, cet anticorps montre la même spécificité et affinité envers l'albumine nitrée et fonctionne en tant qu'anticorps de capture ou de détection.

### Conclusions

Les résultats obtenus indiquent que les anticorps monoclonaux dirigés contre les séquences nitrées connues de l'albumine sérique humaine comprenant respectivement la Tyr[138] et la Tyr[411]:

- o reconnaissent de façon spécifique ces deux séquences au sein de l'albumine humaine nitrée native
- o ne reconnaissent pas l'albumine humaine non nitrée
- o ne reconnaissent pas d'autres peptides ou protéines nitrées telles que l'insuline, l'hémoglobine et la KLH
- o sont utilisables pour le dosage de l'albumine humaine nitrée par ELISA
- o sont utilisables pour le dosage de l'albumine humaine sérique nitrée par ELISA dans le sérum humain
- o peuvent être utilisés en capture ou en détection en ELISA

**Revendications**

1. Utilisation du dosage quantitatif, *in vitro,* dans un échantillon biologique, du taux de nitration de résidus tyrosine d'une protéine ou d'une séquence peptidique physiologique déterminée nitrée, pour la mise en oeuvre d'une méthode de diagnostic *in vitro* de l'état de gravité et d'évolutivité d'une pathologie chronique ou aigüe associée au stress nitrant, ladite protéine ou séquence peptidique physiologique déterminée nitrée étant l'albumine nitrée, ledit dosage quantitatif étant effectué à l'aide d'un anticorps spécifique, en particulier un anticorps monoclonal, reconnaissant spécifiquement

   - soit le résidu tyrosine $Y^{138}$ nitrée de l'albumine,
   - soit le résidu tyrosine $Y^{411}$ nitrée de l'albumine,

   ladite pathologie chronique ou aigüe associée au stress nitrant appartenant au groupe suivant : les maladies inflammatoires, les maladies infectieuses, les maladies ischémiques et les maladies cardiovasculaires.

2. Anticorps reconnaissant spécifiquement l'albumine nitrée sur le résidu tyrosine $Y^{138}$, en particulier un anticorps monoclonal.

3. Anticorps monoclonal selon la revendication 2, sécrété par l'hybridome déposé à la CNCM (Collection Nationale de Culture de Microorganismes, Institut Pasteur, Paris, France) le 8 janvier 2009, sous le numéro d'accession CNCM I-4111.

4. Anticorps reconnaissant spécifiquement l'albumine nitrée sur le résidu tyrosine $Y^{411}$, en particulier un anticorps monoclonal.

5. Anticorps monoclonal selon la revendication 4, sécrété par l'hybridome déposé à la CNCM (Collection Nationale de Culture de Microorganismes, Institut Pasteur, Paris, France) le 8 janvier 2009, sous le numéro d'accession CNCM I-4110.

6. Hybridome déposé à la CNCM (Collection Nationale de Culture de Microorganismes, Institut Pasteur, Paris, France) le 8 janvier 2009, sous le numéro d'accession CNCM I-4111.

7. Hybridome déposé à la CNCM (Collection Nationale de Culture de Microorganismes, Institut Pasteur, Paris, France) le 8 janvier 2009, sous le numéro d'accession CNCM I-4110.

8. Méthode de diagnostic *in vitro* de l'état de gravité et d'évolutivité d'une pathologie chronique associée au stress nitrant dans un échantillon biologique d'un individu, comprenant :

   - la détection d'un complexe immun résultant de la mise en contact d'au moins un anticorps reconnaissant spécifiquement un résidu tyrosine nitré de l'albumine physiologique avec l'albumine physiologique nitrée issue d'un échantillon biologique d'un individu, la dite détection du complexe immun permettant la détermination du taux de nitration de ladite albumine physiologique nitrée, ledit anticorps reconnaissant la nitration de l'albumine humaine sur

     • le résidu tyrosine $Y^{138}$, ou
     • le résidu tyrosine $Y^{411}$

   - la comparaison dudit taux de nitration de résidu tyrosine de l'albumine physiologique, avec les taux de nitration de résidus tyrosine d'un ensemble de n albumines physiologiques dont la valeur du taux de nitration est connue et associée respectivement à un état déterminé de gravité et d'évolutivité de la dite pathologie chronique ou aigüe,
   - la déduction, à partir de la comparaison effectuée à l'étape précédente, du degré de stress nitrant de l'individu pouvant correspondre à un état de gravité et d'évolutivité de la dite pathologie chronique ou aigüe,

   ladite pathologie chronique ou aigüe associée au stress nitrant appartenant au groupe suivant : les maladies inflammatoires, les maladies infectieuses, les maladies ischémiques et les maladies cardiovasculaires.

9. Méthode de diagnostic *in vitro* selon la revendication 8, dans laquelle le degré de stress nitrant de l'individu peut correspondre à un état de gravité et d'évolutivité des pathologies choisies parmi: les maladies inflammatoires, les maladies infectieuses, les maladies ischémiques, et les maladies cardiovasculaires.

10. Méthode de diagnostic *in vitro* selon la revendication 8 ou 9, dans laquelle la dite détection du complexe immun est réalisée par les techniques d'ELISA, d'immunohistochimie, d'immunocytochimie, d'immunoprécipitation, de western blot ou de radioimmunologie.


**Patentansprüche**

1. Verwendung einer quantitativen Bestimmung, *in vitro* in einer biologischen Probe, der Nitrierungsrate von Tyrosinresten eines Proteins oder einer bestimmten nitrierten physiologischen Peptidsequenz, zur Implementierung eines *In-vitro*-Verfahrens zur Diagnose der Schwere und des Progressionsgrads einer chronischen oder akuten Pathologie, die mit Nitrostress assoziiert ist,
   wobei es sich bei dem Protein oder der bestimmten physiologischen nitrierten Peptidsequenz um nitriertes Albumin handelt,
   wobei die quantitative Bestimmung mithilfe eines spezifischen Antikörpers durchgeführt wird, insbesondere eines monoklonalen Antikörpers, der

   - entweder den nitrierten Tyrosinrest $Y^{138}$ des Albumins,
   - oder den nitrierten Tyrosinrest $Y^{422}$ des Albumins spezifisch erkennt,

   wobei die chronische oder akute Pathologie, die mit Nitrostress assoziiert ist, zur folgenden Gruppe gehört: Entzündungskrankheiten, Infektionskrankheiten,
   ischämische Krankheiten und kardiovaskuläre Krankheiten.

2. Antikörper, der das nitrierte Albumin auf dem Tyrosinrest $Y^{138}$ spezifisch erkennt, insbesondere ein monoklonaler Antikörper.

3. Monoklonaler Antikörper nach Anspruch 2, der von dem Hybridom sezerniert wird, das am 8. Januar 2009 unter der Zugangsnummer CNCM I-4111 bei der CNCM (Collection Nationale de Culture de Microorganismes, Institut Pasteur, Paris, Frankreich) hinterlegt wurde.

4. Antikörper, der das nitrierte Albumin auf dem Tyrosinrest $X^{411}$ spezifisch erkennt, insbesondere ein monoklonaler Antikörper.

5. Monoklonaler Antikörper nach Anspruch 4, der von dem Hybridom sezerniert wird, das am 8. Januar 2009 unter der Zugangsnummer CNCM I-4110 bei der CNCM (Collection Nationale de Culture de Microorganismes, Institut Pasteur, Paris, Frankreich) hinterlegt wurde.

6. Hybridom, das am 8. Januar 2009 unter der Zugangsnummer CNCM 1-4111 bei der CNCM (Collection Nationale de Culture de Microorganismes, Institut Pasteur, Paris, Frankreich) hinterlegt wurde.

7. Hybridom, das am 8. Januar 2009 unter der Zugangsnummer CNCM 1-4110 bei der CNCM (Collection Nationale de Culture de Microorganismes, Institut Pasteur, Paris, Frankreich) hinterlegt wurde.

8. *In-vitro*-Verfahren zur Diagnose der Schwere und des Progressionsgrads einer chronischen Pathologie, die mit Nitrostress assoziiert ist, in einer biologischen Probe eines Individuums, umfassend:

   - den Nachweis eines Immunkomplexes, der aus dem Inkontaktbringen mindestens eines Antikörpers, der einen nitrierten Tyrosinrest des physiologischen Albumins spezifisch erkennt, mit dem nitrierten physiologischen Albumin aus einer biologischen Probe eines Individuums resultiert, wobei der Nachweis des Immunkomplexes die Feststellung der Nitrierungsrate des nitrierten physiologischen Albumins ermöglicht,
   wobei der Antikörper die Nitrierung von humanem Albumin auf

   • dem Tyrosinrest $Y^{138}$, oder
   • dem Tyrosinrest $Y^{411}$ erkennt,

   - wobei der Vergleich des Nitrierungsrate des Tyrosinrests des physiologischen Albumins mit der Nitrierungsrate des Tyrosinrests einer Gesamtheit von n physiologischen Albuminen, von denen der Wert der Nitrierungsrate bekannt ist und jeweils mit einer bestimmten Schwere und einem bestimmten Progressionsgrad der chronischen oder akuten Pathologie assoziiert ist,
- wobei die Ableitung aus dem im vorhergehenden Schritt durchgeführten Vergleich des Grads an Nitrostress des Individuums einem Schwere- und Progressionsgrad der chronischen oder akuten Pathologie entsprechen kann,

wobei die chronische oder akute Pathologie, die mit Nitrostress assoziiert ist, zur folgenden Gruppe gehört: Entzündungskrankheiten, Infektionskrankheiten,
ischämische Krankheiten und kardiovaskuläre Krankheiten.

9. *In-vitro*-Verfahren zur Diagnose nach Anspruch 8, wobei der Grad an Nitrostress des Individuums einer Schwere und einem Progressionsgrad der Pathologien entsprechen kann, ausgewählt aus: Entzündungskrankheiten, Infektionskrankheiten, ischämischen Krankheiten und kardiovaskulären Krankheiten.

10. In-vitro-Diagnoseverfahren nach Anspruch 8 oder 9, wobei der Nachweis des Immunkomplexes mit den Techniken ELISA, Immunohistochemie, Immunzytochemie, Immunfällung, Western-blot oder Radioimmunologie durchgeführt wird.

**Claims**

1. Use of *in vitro* quantitative assay, in a biological sample, of the degree of nitration of tyrosine residues of a given nitrated protein or physiological peptide sequence, for the implementation of an *in vitro* method of diagnosis of the state of severity and progressiveness of a chronic or acute pathology associated with nitrating stress, said given nitrated protein or physiological peptide sequence being nitrated albumin, said quantitative assay being carried out by means of a specific antibody, in particular a monoclonal antibody, specifically recognizing

   - either the nitrated $Y^{138}$ tyrosine residue of albumin,
   - or the nitrated $Y^{411}$ tyrosine residue of albumin,

   said chronic or acute pathology associated with nitrating stress belonging to the following group: inflammatory diseases, infectious diseases, ischaemic diseases and cardiovascular diseases.

2. Antibody specifically recognizing the albumin nitrated on the $Y^{138}$ tyrosine residue, in particular a monoclonal antibody.

3. Monoclonal antibody according to claim 2, secreted by the hybridoma deposited at the CNCM (Collection Nationale de Culture de Microorganismes, Institut Pasteur, Paris, France) on 8th January 2009, under the accession number CNCM I-4111.

4. Antibody specifically recognizing the albumin nitrated on the $Y^{411}$ tyrosine residue, in particular a monoclonal antibody.

5. Monoclonal antibody according to claim 4, secreted by the hybridoma deposited at the CNCM (Collection Nationale de Culture de Microorganismes, Institut Pasteur, Paris, France) on 8th January 2009, under the accession number CNCM I-41110.

6. Hybridoma deposited at the CNCM (Collection Nationale de Culture de Microorganismes, Institut Pasteur, Paris, France) on 8th January 2009, under the accession number CNCM I-4111.

7. Hybridoma deposited at the CNCM (Collection Nationale de Culture de Microorganismes, Institut Pasteur, Paris, France) on 8th January 2009, under the accession number CNCM I-4110.

8. *In vitro* method of diagnosis of the state of severity and progressiveness of a chronic pathology associated with nitrating stress in a biological sample from an individual, comprising:

   - the detection of an immune complex resulting from the contact of at least one antibody specifically recognizing a nitrated tyrosine residue of the physiological albumin with the nitrated physiological albumin from a biological sample of an individual, said detection of the immune complex permitting the determination of the degree of nitration of said nitrated physiological albumin,
said antibody recognizing the nitration of human albumin on

- the $Y^{138}$ tyrosine residue, or
- the $Y^{411}$ tyrosine residue

- the comparison of said degree of nitration of the tyrosine residue of physiological albumin, with nitration degrees of tyrosine residues of a set of n physiological albumin whose degree of nitration value is known and associated respectively to a determinate state of severity and progressiveness of said chronic or acute pathology,
- the deduction from the comparison made at the precedent stage, of the degree of nitrating stress of the individual that can correspond to a state of severity and progressiveness of said chronic or acute pathology,

said chronic or acute pathology associated with nitrating stress belonging to the following group: inflammatory diseases, infectious diseases, ischaemic diseases and cardiovascular diseases.

9. *In vitro* method of diagnosis according to claim 8, wherein the degree of nitrating stress of the individual can correspond to a state of severity and progressiveness of pathologies chosen from among: inflammatory diseases, infectious diseases, ischemic diseases and cardiovascular diseases.

10. *In vitro* method of diagnosis according to claim 8 or 9, wherein said detection of the immune complex is completed by ELISA, immunohistochemistry, immunocytochemistry, immunoprecipitation, western blot or radioimmunology techniques.

**Figure 1**
Gel de séparation des protéines nitrées plasmatiques.

EP 2 235 539 B1

Figure 2A : Spectre de masse de peptides de l'Albumine

EP 2 235 539 B1

**Figure 2B : Spectre de masse de peptides de l'actine β.**

Figure 2C : Spectre de masse de peptides de la protéine de liaison à la vitamine D.

Figure 2D : Spectre de masse de peptides de la bande 3 protéine la protéine transporteuse d'anion érythrocytaire.

**Figure 2E :** Spectre de masse de peptides de la chaine β de la spectrine érythrocytaire.

EP 2 235 539 B1

**Figure 2F :** Spectre de masse de peptides de la chaine β du fibrinogène.

**Figure 2G : Spectre de masse de peptides de la protéine membranaire érythrocytaire 4.1**

EP 2 235 539 B1

Figure 2H : Spectre de masse de peptides du précurseur de la fibronectine.

# Rapport O$_2$·/NO , stress nitrant et oxydant

O$_2$·/NO

| Ratio NO·/O$_2$·‾ | Dominant Species | Reactions catalyzed | Cellular events |
|---|---|---|---|
| > 10 | NO· | Nitrosylation | Resting state |
| 2 - 3 | [NO$^+$] | Nitrosation | Cell activation |
| 1 | ‾OONO | Nitration. 2 e‾ oxidation  Tyr-NO$_2$ | Max. cell activation, protease activation, zinc-finger oxidation |
| 0.5 | NO$_2$· | 1 e-oxidation | Oxidative stress, but cell survival (?) |
| Peroxydation des lipides, carbonylation des protéines et lésions de l'ADN | | | |
| < 0.5 | OH· | 1 e-oxidation, chain reaction | Oxidative stress  cell death |

*From Ullrich V. and Kissner R., 2006*

**Figure 3**
**Conséquences cellulaires des modifications du rapport 0$_2$‾/NO·.**

# Rapport NO/$O_2^-$ : RNS, ROS, cibles et conséquences

- NO >> $O_2^-$ → nitrosylation (NO-Fe):
  - GC soluble → GMPc: régulation
- NO > $O_2^-$ → nitrosation (Cys-SH ⇌ Cys-SNO)
  - Caspase 3, Hb: régulation
- NO = $O_2^-$ → nitration (Tyr ⇌ Tyr-$NO_2$; réversible)
  - $PGI_2S$, PGHS, MAPK, Akt, Alb, Hb, transferrine, fibrinogène, ...:
  - Régulation, adaptation, prolifération et apoptose si massive
- NO < $O_2^-$ → exces d'$O_2^-$ → oxydation (irréversible):
  - ADN, lipides, protéines, acides aminés: apoptose si massive
- NO << $O_2^-$ → OH· → réactions oxydatives en chaîne → apoptose

## Figure 4
**Réactions et modifications provoquées par les ROS et les RNS.**

**Figure 5**

**Courbe d'étalonnage du dosage par ELISA de l'albumine nitrée**

**Figure 6**
**Corrélations des valeurs des dosages répétés de l'albumine nitrée
par ELISA dans 192 plasmas.**

**Figure 7**

**Figure 8**

**Figure 9**

**Figure 10**

Figure 11

**Figure 12**

**Figure 13**

**Figure 14A**

**Figure 14B**

Figure 15A

Figure 15B

Figure 15C

Figure 16

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 1998029452 A
- US 2005244905 A

- WO 03076946 A
- FR 0800142

**Littérature non-brevet citée dans la description**

- **KHAN et al.** *Biochem J,* 1998, vol. 330, 795-801
- **CAVEDON et al.** *J. Mat-Fet Neonat Med,* 2006, vol. 19, 35
- **JIAO et al.** *Analytical. Biochem,* 2001, vol. 293, 43-52
- **MALAN, P. G. et al.** *Biochemistry,* 1970, vol. 9 (16), 3205-3214
- **SOKOLOVSKY et al.** *Biochemistry,* 1966, vol. 5 (11), 3582-3589
- **DE MORRIS et al.** *Biochemistry,* 1970, vol. 9 (20), 3930-3937
- **CARPENTER et al.** *Biochemistry,* 1980, vol. 19 (25), 5926-5931
- **PIETRAFORTE, D.** *Amino Acids,* 2003, vol. 25 (3-4), 341-350
- **PIETRAFORTE, D. et al.** *Biochemistry,* 2001, vol. 40 (50), 15300-15309
- **MINETTI, M. et al.** *Biochemistry,* 2000, vol. 39 (22), 6689-6697
- **SORESCU, D. ; K.K. GRIENDLING.** Reactive oxygen species, mitochondria, and NAD(P)H oxidases in the development and progression of heart failure. *Congest Heart Fail,* 2002, vol. 8 (3), 132-40
- **WHITE, M. et al.** ncreased systemic inflammation and oxidative stress in patients with worsening congestive heart failure: improvement after short-term inotropic support. *Clin Sci (Lond),* 2006, vol. 110 (4), 483-9
- **MALLAT, Z. et al.** Elevated levels of 8-iso-prostaglandin F2alpha in pericardial fluid of patients with heart failure: a potential role for in vivo oxidant stress in ventricular dilatation and progression to heart failure. *Circulation,* 1998, vol. 97 (16), 1536-9
- **DHALLA, A.K. ; M.F. HILL ; P.K. SINGAL.** Role of oxidative stress in transition of hypertrophy to heart failure. *J Am Coll Cardiol,* 1996, vol. 28 (2), 506-14
- **FERRARI, R. et al.** Oxidative stress during myocardial ischaemia and heart failure. *Eur Heart J,* 1998, (19), B2-11

- **COTTER, G., et al.** Acute heart failure: a novel approach to its pathogenesis and treatment. *Eur J Heart Fail,* 2002, vol. 4 (3), 227-34
- **MANN, D.L.** Inflammatory mediators and the failing heart: past, present, and the foreseeable future. *Circ Res,* 2002, vol. 91 (11), 988-98
- **YNDESTAD, A. et al.** Systemic inflammation in heart failure--the whys and wherefores. *Heart Fail Rev,* 2006, vol. 11 (1), 83-92
- **TOUSOULIS, D. et al.** Statins in heart failure. Beyond the lipid lowering effect. *Int J Cardiol,* 2007, vol. 115 (2), 144-50
- **FREIN, D. et al.** Redox regulation: a new challenge for pharmacology. *Biochem Pharmacol,* 2005, vol. 70 (6), 811-23
- **ULLRICH, V. ; R. KISSNER.** Redox signaling: bioinorganic chemistry at its best. *J Inorg Biochem,* 2006, vol. 100 (12), 2079-86
- **PINZAR, E. et al.** Angiotensin II induces tyrosine nitration and activation of ERK1/2 in vascular smooth muscle cells. *FEBS Lett,* 2005, vol. 579 (22), 5100-4
- **LOKUTA, A.J. et al.** Increased nitration of sarcoplasmic reticulum Ca2+- ATPase in human heart failure. *Circulation,* 2005, vol. 111 (8), 988-95
- **RYBERG, H. ; K CAIDAHL.** Chromatographic and mass spectrometric methods for quantitative determination of 3-nitrotyrosine in biological samples and their application to human samples. *J Chromatogr B Analyt Technol Biomed Life Sci,* 2007
- **PANNALA, A.S. et al.** pH-dependent nitration of para-hydroxyphenylacetic acid in the stomach. *Free Radic Biol Med,* 2006, vol. 41 (6), 896-901
- **FABBRI, L.M. ; K.F. RABE.** From COPD to chronic systemic inflammatory syndrome?. *Lancet,* 2007, vol. 370 (9589), 797-9
- **GROENENDAAL, F. et al.** Nitrotyrosine in brain tissue of neonates after perinatal asphyxia. *Arch Dis Child Fetal Neonatal Ed,* 2006, vol. 91 (6), F429-33
- **GROENENDAAL, F. et al.** Nitrotyrosine in Human Neonatal Spinal Cord after Perinatal Asphyxia. *Neonatology,* 2007, vol. 93 (1), 1-6

- **SUH, S.W. et al.** Hypoglycemic neuronal death and cognitive impairment are prevented by poly(ADP-ribose) polymerase inhibitors administered after hypoglycemia. *J Neurosci,* 2003, vol. 23 (33), 10681-90
- **ISCHIROPOULOS, H. ; J.S. BECKMAN.** Oxidative stress and nitration in neurodegeneration: cause, effect, or association?. *J Clin Invest,* 2003, vol. 111 (2), 163-9
- **CARRERAS, M.C. ; J.J. PODEROSO.** Mitochondrial nitric oxide in the signaling of cell integrated responses. *Am J Physiol Cell Physiol,* 2007, vol. 292 (5), C1569-80
- **WHITE, M. et al.** Increased systemic inflammation and oxidative stress in patients with worsening congestive heart failure: improvement after short-term inotropic support. *Clin Sci (Lond),* 2006, vol. 110 (4), 483-9
- **POLIDORI, M.C. et al.** Increased F2 isoprostane plasma levels in patients with congestive heart failure are correlated with antioxidant status and disease severity. *J Card Fail,* 2004, vol. 10 (4), 334-8
- **ADAMOPOULOS, S. ; J.T. PARISSIS ; D.T. KRE-MASTINOS.** A glossary of circulating cytokines in chronic heart failure. *Eur JHeart Fail,* 2001, vol. 3 (5), 517-26

- **ANAND, I.S. et al.** C-reactive protein in heart failure: prognostic value and the effect of valsartan. *Circulation,* 2005, vol. 112 (10), 1428-34
- **DE DENUS, S., ; C. PHARAND ; D.R. WILLIAM-SON.** Brain natriuretic peptide in the management of heart failure: the versatile neurohormone. *Chest,* 2004, vol. 125 (2), 652-68
- **KISTORP, C. et al.** N-terminal pro-brain natriuretic peptide, C-reactive protein, and urinary albumin levels as predictors of mortality and cardiovascular events in older adults. *Jama,* 2005, vol. 293 (13), 1609-16
- **MOSKOWITZ, R. ; M KUKIN.** Oxidative stress and congestive heart failure. *Congest Heart Fail,* 1999, vol. 5 (4), 153-163
- **MARIANI, E. et al.** Oxidative stress in brain aging, neurodegenerative and vascular diseases: an overview. *J Chromatogr B Analyt Technol Biomed Life Sci,* 2005, vol. 827 (1), 65-75
- **HALL, C.** Essential biochemistry and physiology of (NT-pro)BNP. *Eur J Heart Fail,* 2004, vol. 6 (3), 257-60
- **ANTMAN, E.M.** Decision making with cardiac troponin tests. *N Engl J Med,* 2002, vol. 346 (26), 2079-82
- **KISTORP, C. et al.** Plasma adiponectin, body mass index, and mortality in patients with chronic heart failure. *Circulation,* 2005, vol. 112 (12), 1756-62

SEQUENCE LISTING

<110> Université Joseph Fourier BOTTARI, Serge

<120> UTILISATION D'UNE PROTEINE OU D'UNE SEQUENCE PEPTIDIQUE NITREE POUR LA MISE OEUVRE D'UNE METHODE DIAGNOSTIC

<130> WOB 07 CR UJF ALBN

<150> FR 08/00142
<151> 2008-01-10

<160> 11

<170> PatentIn version 3.5

<210> 1
<211> 609
<212> PRT
<213> Homo sapiens

<400> 1

Met Lys Trp Val Thr Phe Ile Ser Leu Leu Phe Leu Phe Ser Ser Ala
1                   5                   10                  15

Tyr Ser Arg Gly Val Phe Arg Arg Asp Ala His Lys Ser Glu Val Ala
                20                  25                  30

His Arg Phe Lys Asp Leu Gly Glu Glu Asn Phe Lys Ala Leu Val Leu
            35                  40                  45

Ile Ala Phe Ala Gln Tyr Leu Gln Gln Cys Pro Phe Glu Asp His Val
        50                  55                  60

Lys Leu Val Asn Glu Val Thr Glu Phe Ala Lys Thr Cys Val Ala Asp
65                  70                  75                  80

Glu Ser Ala Glu Asn Cys Asp Lys Ser Leu His Thr Leu Phe Gly Asp
                85                  90                  95

Lys Leu Cys Thr Val Ala Thr Leu Arg Glu Thr Tyr Gly Glu Met Ala
            100                 105                 110

Asp Cys Cys Ala Lys Gln Glu Pro Glu Arg Asn Glu Cys Phe Leu Gln
        115                 120                 125

His Lys Asp Asp Asn Pro Asn Leu Pro Arg Leu Val Arg Pro Glu Val
    130                 135                 140

Asp Val Met Cys Thr Ala Phe His Asp Asn Glu Glu Thr Phe Leu Lys

<pre>
        145                  150                  155                  160

      Lys Tyr Leu Tyr Glu Ile Ala Arg Arg His Pro Tyr Phe Tyr Ala Pro
                      165                  170                  175

      Glu Leu Leu Phe Phe Ala Lys Arg Tyr Lys Ala Ala Phe Thr Glu Cys
                  180                  185                  190

      Cys Gln Ala Ala Asp Lys Ala Ala Cys Leu Leu Pro Lys Leu Asp Glu
                  195                  200                  205

      Leu Arg Asp Glu Gly Lys Ala Ser Ser Ala Lys Gln Arg Leu Lys Cys
          210                  215                  220

      Ala Ser Leu Gln Lys Phe Gly Glu Arg Ala Phe Lys Ala Trp Ala Val
      225                  230                  235                  240

      Ala Arg Leu Ser Gln Arg Phe Pro Lys Ala Glu Phe Ala Glu Val Ser
                      245                  250                  255

      Lys Leu Val Thr Asp Leu Thr Lys Val His Thr Glu Cys Cys His Gly
                  260                  265                  270

      Asp Leu Leu Glu Cys Ala Asp Asp Arg Ala Asp Leu Ala Lys Tyr Ile
              275                  280                  285

      Cys Glu Asn Gln Asp Ser Ile Ser Ser Lys Leu Lys Glu Cys Cys Glu
              290                  295                  300

      Lys Pro Leu Leu Glu Lys Ser His Cys Ile Ala Glu Val Glu Asn Asp
      305                  310                  315                  320

      Glu Met Pro Ala Asp Leu Pro Ser Leu Ala Ala Asp Phe Val Glu Ser
                      325                  330                  335

      Lys Asp Val Cys Lys Asn Tyr Ala Glu Ala Lys Asp Val Phe Leu Gly
                  340                  345                  350

      Met Phe Leu Tyr Glu Tyr Ala Arg Arg His Pro Asp Tyr Ser Val Val
              355                  360                  365

      Leu Leu Leu Arg Leu Ala Lys Thr Tyr Glu Thr Thr Leu Glu Lys Cys
          370                  375                  380

      Cys Ala Ala Ala Asp Pro His Glu Cys Tyr Ala Lys Val Phe Asp Glu
      385                  390                  395                  400
</pre>

32

```
Phe Lys Pro Leu Val Glu Glu Pro Gln Asn Leu Ile Lys Gln Asn Cys
            405                 410                 415

Glu Leu Phe Glu Gln Leu Gly Glu Tyr Lys Phe Gln Asn Ala Leu Leu
            420                 425                 430

Val Arg Tyr Thr Lys Lys Val Pro Gln Val Ser Thr Pro Thr Leu Val
            435                 440                 445

Glu Val Ser Arg Asn Leu Gly Lys Val Gly Ser Lys Cys Cys Lys His
        450                 455                 460

Pro Glu Ala Lys Arg Met Pro Cys Ala Glu Asp Tyr Leu Ser Val Val
465                 470                 475                 480

Leu Asn Gln Leu Cys Val Leu His Glu Lys Thr Pro Val Ser Asp Arg
                485                 490                 495

Val Thr Lys Cys Cys Thr Glu Ser Leu Val Asn Arg Arg Pro Cys Phe
                500                 505                 510

Ser Ala Leu Glu Val Asp Glu Thr Tyr Val Pro Lys Glu Phe Asn Ala
            515                 520                 525

Glu Thr Phe Thr Phe His Ala Asp Ile Cys Thr Leu Ser Glu Lys Glu
        530                 535                 540

Arg Gln Ile Lys Lys Gln Thr Ala Leu Val Glu Leu Val Lys His Lys
545                 550                 555                 560

Pro Lys Ala Thr Lys Glu Gln Leu Lys Ala Val Met Asp Asp Phe Ala
            565                 570                 575

Ala Phe Val Glu Lys Cys Cys Lys Ala Asp Asp Lys Glu Thr Cys Phe
            580                 585                 590

Ala Glu Glu Gly Lys Lys Leu Val Ala Ala Ser Gln Ala Ala Leu Gly
        595                 600                 605

Leu
```

<210> 2
<211> 147
<212> PRT
<213> Homo sapiens

<400> 2

```
Met Ala Ser His Arg Leu Leu Leu Leu Cys Leu Ala Gly Leu Val Phe
1               5                   10                  15

Val Ser Glu Ala Gly Pro Thr Gly Thr Gly Glu Ser Lys Cys Pro Leu
            20                  25                  30

Met Val Lys Val Leu Asp Ala Val Arg Gly Ser Pro Ala Ile Asn Val
        35                  40                  45

Ala Val His Val Phe Arg Lys Ala Ala Asp Asp Thr Trp Glu Pro Phe
    50                  55                  60

Ala Ser Gly Lys Thr Ser Glu Ser Gly Glu Leu His Gly Leu Thr Thr
65                  70                  75                  80

Glu Glu Glu Phe Val Glu Gly Ile Tyr Lys Val Glu Ile Asp Thr Lys
                85                  90                  95

Ser Tyr Trp Lys Ala Leu Gly Ile Ser Pro Phe His Glu His Ala Glu
            100                 105                 110

Val Val Phe Thr Ala Asn Asp Ser Gly Pro Arg Arg Tyr Thr Ile Ala
        115                 120                 125

Ala Leu Leu Ser Pro Tyr Ser Tyr Ser Thr Thr Ala Val Val Thr Asn
    130                 135                 140

Pro Lys Glu
145
```

<210> 3
<211> 474
<212> PRT
<213> Homo sapiens

<400> 3

```
Met Lys Arg Val Leu Val Leu Leu Leu Ala Val Ala Phe Gly His Ala
1               5                   10                  15

Leu Glu Arg Gly Arg Asp Tyr Glu Lys Asn Lys Val Cys Lys Glu Phe
            20                  25                  30

Ser His Leu Gly Lys Glu Asp Phe Thr Ser Leu Ser Leu Val Leu Tyr
        35                  40                  45
```

```
Ser Arg Lys Phe Pro Ser Gly Thr Phe Glu Gln Val Ser Gln Leu Val
    50                  55                  60

Lys Glu Val Val Ser Leu Thr Glu Ala Cys Cys Ala Glu Gly Ala Asp
65                  70                  75                  80

Pro Asp Cys Tyr Asp Thr Arg Thr Ser Ala Leu Ser Ala Lys Ser Cys
                85                  90                  95

Glu Ser Asn Ser Pro Phe Pro Val His Pro Gly Thr Ala Glu Cys Cys
            100                 105                 110

Thr Lys Glu Gly Leu Glu Arg Lys Leu Cys Met Ala Ala Leu Lys His
        115                 120                 125

Gln Pro Gln Glu Phe Pro Thr Tyr Val Glu Pro Thr Asn Asp Glu Ile
    130                 135                 140

Cys Glu Ala Phe Arg Lys Asp Pro Lys Glu Tyr Ala Asn Gln Phe Met
145                 150                 155                 160

Trp Glu Tyr Ser Thr Asn Tyr Gly Gln Ala Pro Leu Ser Leu Leu Val
            165                 170                 175

Ser Tyr Thr Lys Ser Tyr Leu Ser Met Val Gly Ser Cys Cys Thr Ser
        180                 185                 190

Ala Ser Pro Thr Val Cys Phe Leu Lys Glu Arg Leu Gln Leu Lys His
    195                 200                 205

Leu Ser Leu Leu Thr Thr Leu Ser Asn Arg Val Cys Ser Gln Tyr Ala
    210                 215                 220

Ala Tyr Gly Glu Lys Lys Ser Arg Leu Ser Asn Leu Ile Lys Leu Ala
225                 230                 235                 240

Gln Lys Val Pro Thr Ala Asp Leu Glu Asp Val Leu Pro Leu Ala Glu
            245                 250                 255

Asp Ile Thr Asn Ile Leu Ser Lys Cys Cys Glu Ser Ala Ser Glu Asp
            260                 265                 270

Cys Met Ala Lys Glu Leu Pro Glu His Thr Val Lys Leu Cys Asp Asn
        275                 280                 285
```

35

```
Leu Ser Thr Lys Asn Ser Lys Phe Glu Asp Cys Cys Gln Glu Lys Thr
    290             295             300

Ala Met Asp Val Phe Val Cys Thr Tyr Phe Met Pro Ala Ala Gln Leu
305             310             315             320

Pro Glu Leu Pro Asp Val Glu Leu Pro Thr Asn Lys Asp Val Cys Asp
                325             330             335

Pro Gly Asn Thr Lys Val Met Asp Lys Tyr Thr Phe Glu Leu Ser Arg
            340             345             350

Arg Thr His Leu Pro Glu Val Phe Leu Ser Lys Val Leu Glu Pro Thr
        355             360             365

Leu Lys Ser Leu Gly Glu Cys Cys Asp Val Glu Asp Ser Thr Thr Cys
    370             375             380

Phe Asn Ala Lys Gly Pro Leu Leu Lys Lys Glu Leu Ser Ser Phe Ile
385             390             395             400

Asp Lys Gly Gln Glu Leu Cys Ala Asp Tyr Ser Glu Asn Thr Phe Thr
            405             410             415

Glu Tyr Lys Lys Lys Leu Ala Glu Arg Leu Lys Ala Lys Leu Pro Asp
            420             425             430

Ala Thr Pro Lys Glu Leu Ala Lys Leu Val Asn Lys Arg Ser Asp Phe
        435             440             445

Ala Ser Asn Cys Cys Ser Ile Asn Ser Pro Pro Leu Tyr Cys Asp Ser
    450             455             460

Glu Ile Asp Ala Glu Leu Lys Asn Ile Leu
465             470
```

<210> 4
<211> 698
<212> PRT
<213> Homo sapiens

<400> 4

```
Met Arg Leu Ala Val Gly Ala Leu Leu Val Cys Ala Val Leu Gly Leu
1               5               10                  15

Cys Leu Ala Val Pro Asp Lys Thr Val Arg Trp Cys Ala Val Ser Glu
```

<pre>
                    20                        25                        30

    His Glu Ala Thr Lys Cys Gln Ser Phe Arg Asp His Met Lys Ser Val
            35                    40                    45

    Ile Pro Ser Asp Gly Pro Ser Val Ala Cys Val Lys Lys Ala Ser Tyr
        50                    55                    60

    Leu Asp Cys Ile Arg Ala Ile Ala Ala Asn Glu Ala Asp Ala Val Thr
    65                    70                    75                    80

    Leu Asp Ala Gly Leu Val Tyr Asp Ala Tyr Leu Ala Pro Asn Asn Leu
                    85                    90                    95

    Lys Pro Val Val Ala Glu Phe Tyr Gly Ser Lys Glu Asp Pro Gln Thr
                    100                   105                   110

    Phe Tyr Tyr Ala Val Ala Val Val Lys Lys Asp Ser Gly Phe Gln Met
            115                   120                   125

    Asn Gln Leu Arg Gly Lys Lys Ser Cys His Thr Gly Leu Gly Arg Ser
        130                   135                   140

    Ala Gly Trp Asn Ile Pro Ile Gly Leu Leu Tyr Cys Asp Leu Pro Glu
    145                   150                   155                   160

    Pro Arg Lys Pro Leu Glu Lys Ala Val Ala Asn Phe Phe Ser Gly Ser
                    165                   170                   175

    Cys Ala Pro Cys Ala Asp Gly Thr Asp Phe Pro Gln Leu Cys Gln Leu
            180                   185                   190

    Cys Pro Gly Cys Gly Cys Ser Thr Leu Asn Gln Tyr Phe Gly Tyr Ser
            195                   200                   205

    Gly Ala Phe Lys Cys Leu Lys Asp Gly Ala Gly Asp Val Ala Phe Val
        210                   215                   220

    Lys His Ser Thr Ile Phe Glu Asn Leu Ala Asn Lys Ala Asp Arg Asp
    225                   230                   235                   240

    Gln Tyr Glu Leu Leu Cys Leu Asp Asn Thr Arg Lys Pro Val Asp Glu
                    245                   250                   255

    Tyr Lys Asp Cys His Leu Ala Gln Val Pro Ser His Thr Val Val Ala
                    260                   265                   270
</pre>

Arg Ser Met Gly Gly Lys Glu Asp Leu Ile Trp Glu Leu Leu Asn Gln
275 280 285

Ala Gln Glu His Phe Gly Lys Asp Lys Ser Lys Glu Phe Gln Leu Phe
290 295 300

Ser Ser Pro His Gly Lys Asp Leu Leu Phe Lys Asp Ser Ala His Gly
305 310 315 320

Phe Leu Lys Val Pro Pro Arg Met Asp Ala Lys Met Tyr Leu Gly Tyr
325 330 335

Glu Tyr Val Thr Ala Ile Arg Asn Leu Arg Glu Gly Thr Cys Pro Glu
340 345 350

Ala Pro Thr Asp Glu Cys Lys Pro Val Lys Trp Cys Ala Leu Ser His
355 360 365

His Glu Arg Leu Lys Cys Asp Glu Trp Ser Val Asn Ser Val Gly Lys
370 375 380

Ile Glu Cys Val Ser Ala Glu Thr Thr Glu Asp Cys Ile Ala Lys Ile
385 390 395 400

Met Asn Gly Glu Ala Asp Ala Met Ser Leu Asp Gly Gly Phe Val Tyr
405 410 415

Ile Ala Gly Lys Cys Gly Leu Val Pro Val Leu Ala Glu Asn Tyr Asn
420 425 430

Lys Ser Asp Asn Cys Glu Asp Thr Pro Glu Ala Gly Tyr Phe Ala Val
435 440 445

Ala Val Val Lys Lys Ser Ala Ser Asp Leu Thr Trp Asp Asn Leu Lys
450 455 460

Gly Lys Lys Ser Cys His Thr Ala Val Gly Arg Thr Ala Gly Trp Asn
465 470 475 480

Ile Pro Met Gly Leu Leu Tyr Asn Lys Ile Asn His Cys Arg Phe Asp
485 490 495

Glu Phe Phe Ser Glu Gly Cys Ala Pro Gly Ser Lys Lys Asp Ser Ser
500 505 510

```
Leu Cys Lys Leu Cys Met Gly Ser Gly Leu Asn Leu Cys Glu Pro Asn
        515                 520             525

Asn Lys Glu Gly Tyr Tyr Gly Tyr Thr Gly Ala Phe Arg Cys Leu Val
        530                 535             540

Glu Lys Gly Asp Val Ala Phe Val Lys His Gln Thr Val Pro Gln Asn
545                 550             555                 560

Thr Gly Gly Lys Asn Pro Asp Pro Trp Ala Lys Asn Leu Asn Glu Lys
                565             570             575

Asp Tyr Glu Leu Leu Cys Leu Asp Gly Thr Arg Lys Pro Val Glu Glu
            580             585             590

Tyr Ala Asn Cys His Leu Ala Arg Ala Pro Asn His Ala Val Val Thr
        595             600             605

Arg Lys Asp Lys Glu Ala Cys Val His Lys Ile Leu Arg Gln Gln Gln
        610             615             620

His Leu Phe Gly Ser Asn Val Thr Asp Cys Ser Gly Asn Phe Cys Leu
625             630             635             640

Phe Arg Ser Glu Thr Lys Asp Leu Leu Phe Arg Asp Asp Thr Val Cys
            645             650             655

Leu Ala Lys Leu His Asp Arg Asn Thr Tyr Glu Lys Tyr Leu Gly Glu
            660             665             670

Glu Tyr Val Lys Ala Val Gly Asn Leu Arg Lys Cys Ser Thr Ser Ser
        675             680             685

Leu Leu Glu Ala Cys Thr Phe Arg Arg Pro
        690             695
```

<210> 5
<211> 1006
<212> PRT
<213> Homo sapiens

<400> 5

```
Met Lys Ile Leu Ile Leu Gly Ile Phe Leu Phe Leu Cys Ser Thr Pro
1               5                   10                  15

Ala Trp Ala Lys Glu Lys His Tyr Tyr Ile Gly Ile Ile Glu Thr Thr
```

|  |  |  |  | 20 |  |  |  | 25 |  |  |  | 30 |  |
|---|---|---|---|---|---|---|---|---|---|---|---|---|---|

Trp Asp Tyr Ala Ser Asp His Gly Glu Lys Lys Leu Ile Ser Val Asp
          35                  40                  45

Thr Glu His Ser Asn Ile Tyr Leu Gln Asn Gly Pro Asp Arg Ile Gly
    50                  55                  60

Arg Leu Tyr Lys Lys Ala Leu Tyr Leu Gln Tyr Thr Asp Glu Thr Phe
65                  70                  75                  80

Arg Thr Thr Ile Glu Lys Pro Val Trp Leu Gly Phe Leu Gly Pro Ile
              85                  90                  95

Ile Lys Ala Glu Thr Gly Asp Lys Val Tyr Val His Leu Lys Asn Leu
              100                 105                 110

Ala Ser Arg Pro Tyr Thr Phe His Ser His Gly Ile Thr Tyr Tyr Lys
        115                 120                 125

Glu His Glu Gly Ala Ile Tyr Pro Asp Asn Thr Thr Asp Phe Gln Arg
    130                 135                 140

Ala Asp Asp Lys Val Tyr Pro Gly Glu Gln Tyr Thr Tyr Met Leu Leu
145                 150                 155                 160

Ala Thr Glu Glu Gln Ser Pro Gly Glu Gly Asp Gly Asn Cys Val Thr
              165                 170                 175

Arg Ile Tyr His Ser His Ile Asp Ala Pro Lys Asp Ile Ala Ser Gly
          180                 185                 190

Leu Ile Gly Pro Leu Ile Ile Cys Lys Lys Asp Ser Leu Asp Lys Glu
          195                 200                 205

Lys Glu Lys His Ile Asp Arg Glu Phe Val Val Met Phe Ser Val Val
    210                 215                 220

Asp Glu Asn Phe Ser Trp Tyr Leu Glu Asp Asn Ile Lys Thr Tyr Cys
225                 230                 235                 240

Ser Glu Pro Glu Lys Val Asp Lys Asp Asn Glu Asp Phe Gln Glu Ser
              245                 250                 255

Asn Arg Met Tyr Ser Val Asn Gly Tyr Thr Phe Gly Ser Leu Pro Gly
          260                 265                 270

```
Leu Ser Met Cys Ala Glu Asp Arg Val Lys Trp Tyr Leu Phe Gly Met
        275                 280                 285

Gly Asn Glu Val Asp Val His Ala Ala Phe Phe His Gly Gln Ala Leu
        290                 295                 300

Thr Asn Lys Asn Tyr Arg Ile Asp Thr Ile Asn Leu Phe Pro Ala Thr
305                 310                 315                 320

Leu Phe Asp Ala Tyr Met Val Ala Gln Asn Pro Gly Glu Trp Met Leu
                325                 330                 335

Ser Cys Gln Asn Leu Asn His Leu Lys Ala Gly Leu Gln Ala Phe Phe
                340                 345                 350

Gln Val Gln Glu Cys Asn Lys Ser Ser Ser Lys Asp Asn Ile Arg Gly
            355                 360                 365

Lys His Val Arg His Tyr Tyr Ile Ala Ala Glu Glu Ile Ile Trp Asn
    370                 375                 380

Tyr Ala Pro Ser Gly Ile Asp Ile Phe Thr Lys Glu Asn Leu Thr Ala
385                 390                 395                 400

Pro Gly Ser Asp Ser Ala Val Phe Phe Glu Gln Gly Thr Thr Arg Ile
                405                 410                 415

Gly Gly Ser Tyr Lys Lys Leu Val Tyr Arg Glu Tyr Thr Asp Ala Ser
            420                 425                 430

Phe Thr Asn Arg Lys Glu Arg Gly Pro Glu Glu Glu His Leu Gly Ile
        435                 440                 445

Leu Gly Pro Val Ile Trp Ala Glu Val Gly Asp Thr Ile Arg Val Thr
    450                 455                 460

Phe His Asn Lys Gly Ala Tyr Pro Leu Ser Ile Glu Pro Ile Gly Val
465                 470                 475                 480

Arg Phe Asn Lys Asn Asn Glu Gly Thr Tyr Tyr Ser Pro Asn Tyr Asn
                485                 490                 495

Pro Gln Ser Arg Ser Val Pro Pro Ser Ala Ser His Val Ala Pro Thr
            500                 505                 510
```

```
Glu Thr Phe Thr Tyr Glu Trp Thr Val Pro Lys Glu Val Gly Pro Thr
        515                 520                 525

Asn Ala Asp Pro Val Cys Leu Ala Lys Met Tyr Tyr Ser Ala Val Asp
        530                 535                 540

Pro Thr Lys Asp Ile Phe Thr Gly Leu Ile Gly Pro Met Lys Ile Cys
545                 550                 555                 560

Lys Lys Gly Ser Leu His Ala Asn Gly Arg Gln Lys Asp Val Asp Lys
                565                 570                 575

Glu Phe Tyr Leu Phe Pro Thr Val Phe Asp Glu Asn Glu Ser Leu Leu
        580                 585                 590

Leu Glu Asp Asn Ile Arg Met Phe Thr Thr Ala Pro Asp Gln Val Asp
        595                 600                 605

Lys Glu Asp Glu Asp Phe Gln Glu Ser Asn Lys Met His Ser Met Asn
        610                 615                 620

Gly Phe Met Tyr Gly Asn Gln Pro Gly Leu Thr Met Cys Lys Gly Asp
625                 630                 635                 640

Ser Val Val Trp Tyr Leu Phe Ser Ala Gly Asn Glu Ala Asp Val His
                645                 650                 655

Gly Ile Tyr Phe Ser Gly Asn Thr Tyr Leu Trp Arg Gly Glu Arg Arg
                660                 665                 670

Asp Thr Ala Asn Leu Phe Pro Gln Thr Ser Leu Thr Leu His Met Trp
        675                 680                 685

Pro Asp Thr Glu Gly Thr Phe Asn Val Glu Cys Leu Thr Thr Asp His
        690                 695                 700

Tyr Thr Gly Gly Met Lys Gln Lys Tyr Thr Val Asn Gln Cys Arg Arg
705                 710                 715                 720

Gln Ser Glu Asp Ser Thr Phe Tyr Leu Gly Glu Arg Thr Tyr Tyr Ile
                725                 730                 735

Ala Ala Val Glu Val Glu Trp Asp Tyr Ser Pro Gln Arg Glu Trp Glu
                740                 745                 750
```

44

Lys Glu Leu His His Leu Gln Glu Gln Asn Val Ser Asn Ala Phe Leu
                755                    760                765

Asp Lys Gly Glu Phe Tyr Ile Gly Ser Lys Tyr Lys Lys Val Val Tyr
        770                    775                780

Arg Gln Tyr Thr Asp Ser Thr Phe Arg Val Pro Val Glu Arg Lys Ala
785                    790                795                800

Glu Glu Glu His Leu Gly Ile Leu Gly Pro Gln Leu His Ala Asp Val
                805                    810                815

Gly Asp Lys Val Lys Ile Ile Phe Lys Asn Met Ala Thr Arg Pro Tyr
                820                    825                830

Ser Ile His Ala His Gly Val Gln Thr Glu Ser Ser Thr Val Thr Pro
            835                    840                845

Thr Leu Pro Gly Glu Thr Leu Thr Tyr Val Trp Lys Ile Pro Glu Arg
        850                    855                860

Ser Gly Ala Gly Thr Glu Asp Ser Ala Cys Ile Pro Trp Ala Tyr Tyr
865                    870                875                880

Ser Thr Val Asp Gln Val Lys Asp Leu Tyr Ser Gly Leu Ile Gly Pro
                885                    890                895

Leu Ile Val Cys Arg Arg Pro Tyr Leu Lys Val Phe Asn Pro Arg Arg
            900                    905                910

Lys Leu Glu Phe Ala Leu Leu Phe Leu Val Phe Asp Glu Asn Glu Ser
        915                    920                925

Trp Tyr Leu Asp Asp Asn Ile Lys Thr Tyr Ser Asp His Pro Glu Lys
    930                    935                940

Val Asn Lys Asp Asp Glu Glu Phe Ile Glu Ser Asn Lys Met His Ala
945                    950                955                960

Ile Asn Gly Arg Met Phe Gly Asn Leu Gln Gly Leu Thr Met His Val
                965                    970                975

Gly Asp Glu Val Asn Trp Tyr Leu Met Gly Met Gly Asn Glu Ile Asp
            980                    985                990

Leu His Thr Val His Phe His Gly  His Ser Phe Gln Tyr  Lys

995                    1000                    1005

<210> 6
<211> 1246
<212> PRT
<213> Homo sapiens

<400> 6

```
Met Arg Glu Trp Val Leu Leu Met Ser Val Leu Leu Cys Gly Leu Ala
1                   5                   10                  15

Gly Pro Thr His Leu Phe Gln Pro Ser Leu Val Leu Asp Met Ala Lys
            20                  25                  30

Val Leu Leu Asp Asn Tyr Cys Phe Pro Glu Asn Leu Leu Gly Met Gln
        35                  40                  45

Glu Ala Ile Gln Gln Ala Ile Lys Ser His Glu Ile Leu Ser Ile Ser
    50                  55                  60

Asp Pro Gln Thr Leu Ala Ser Val Leu Thr Ala Gly Val Gln Ser Ser
65                  70                  75                  80

Leu Asn Asp Pro Arg Leu Val Ile Ser Tyr Glu Pro Ser Thr Pro Glu
                85                  90                  95

Pro Pro Pro Gln Val Pro Ala Leu Thr Ser Leu Ser Glu Glu Glu Leu
            100                 105                 110

Leu Ala Trp Leu Gln Arg Gly Leu Arg His Glu Val Leu Glu Gly Asn
            115                 120                 125

Val Gly Tyr Leu Arg Val Asp Ser Val Pro Gly Gln Glu Val Leu Ser
    130                 135                 140

Met Met Gly Glu Phe Leu Val Ala His Val Trp Gly Asn Leu Met Gly
145                 150                 155                 160

Thr Ser Ala Leu Val Leu Asp Leu Arg His Cys Thr Gly Gly Gln Val
                165                 170                 175

Ser Gly Ile Pro Tyr Ile Ile Ser Tyr Leu His Pro Gly Asn Thr Ile
            180                 185                 190

Leu His Val Asp Thr Ile Tyr Asn Arg Pro Ser Asn Thr Thr Thr Glu
        195                 200                 205
```

Ile Trp Thr Leu Pro Gln Val Leu Gly Glu Arg Tyr Gly Ala Asp Lys
    210                 215                 220

Asp Val Val Val Leu Thr Ser Ser Gln Thr Arg Gly Val Ala Glu Asp
225                 230                 235                 240

Ile Ala His Ile Leu Lys Gln Met Arg Arg Ala Ile Val Val Gly Glu
                245                 250                 255

Arg Thr Gly Gly Gly Ala Leu Asp Leu Arg Lys Leu Arg Ile Gly Glu
                260                 265                 270

Ser Asp Phe Phe Phe Thr Val Pro Val Ser Arg Ser Leu Gly Pro Leu
            275                 280                 285

Gly Gly Gly Ser Gln Thr Trp Glu Gly Ser Gly Val Leu Pro Cys Val
    290                 295                 300

Gly Thr Pro Ala Glu Gln Ala Leu Glu Lys Ala Leu Ala Ile Leu Thr
305                 310                 315                 320

Leu Arg Ser Ala Leu Pro Gly Val Val His Cys Leu Gln Glu Val Leu
                325                 330                 335

Lys Asp Tyr Tyr Thr Leu Val Asp Arg Val Pro Thr Leu Leu Gln His
                340                 345                 350

Leu Ala Ser Met Asp Phe Ser Thr Val Val Ser Glu Glu Asp Leu Val
                355                 360                 365

Thr Lys Leu Asn Ala Gly Leu Gln Ala Ala Ser Glu Asp Pro Arg Leu
    370                 375                 380

Leu Val Arg Ala Ile Gly Pro Thr Glu Thr Pro Ser Trp Pro Ala Pro
385                 390                 395                 400

Asp Ala Ala Ala Glu Asp Ser Pro Gly Val Ala Pro Glu Leu Pro Glu
                405                 410                 415

Asp Glu Ala Ile Arg Gln Ala Leu Val Asp Ser Val Phe Gln Val Ser
                420                 425                 430

Val Leu Pro Gly Asn Val Gly Tyr Leu Arg Phe Asp Ser Phe Ala Asp
    435                 440                 445

```
Ala Ser Val Leu Gly Val Leu Ala Pro Tyr Val Leu Arg Gln Val Trp
    450             455             460

Glu Pro Leu Gln Asp Thr Glu His Leu Ile Met Asp Leu Arg His Asn
465             470             475             480

Pro Gly Gly Pro Ser Ser Ala Val Pro Leu Leu Leu Ser Tyr Phe Gln
            485             490             495

Gly Pro Glu Ala Gly Pro Val His Leu Phe Thr Thr Tyr Asp Arg Arg
        500             505             510

Thr Asn Ile Thr Gln Glu His Phe Ser His Met Glu Leu Pro Gly Pro
    515             520             525

Arg Tyr Ser Thr Gln Arg Gly Val Tyr Leu Leu Thr Ser His Arg Thr
    530             535             540

Ala Thr Ala Ala Glu Glu Phe Ala Phe Leu Met Gln Ser Leu Gly Trp
545             550             555             560

Ala Thr Leu Val Gly Glu Ile Thr Ala Gly Asn Leu Leu His Thr Arg
            565             570             575

Thr Val Pro Leu Leu Asp Thr Pro Glu Gly Ser Leu Ala Leu Thr Val
            580             585             590

Pro Val Leu Thr Phe Ile Asp Asn His Gly Glu Ala Trp Leu Gly Gly
        595             600             605

Gly Val Val Pro Asp Ala Ile Val Leu Ala Glu Glu Ala Leu Asp Lys
    610             615             620

Ala Gln Glu Val Leu Glu Phe His Gln Ser Leu Gly Ala Leu Val Glu
625             630             635             640

Gly Thr Gly His Leu Leu Glu Ala His Tyr Ala Arg Pro Glu Val Val
            645             650             655

Gly Gln Thr Ser Ala Leu Leu Arg Ala Lys Leu Ala Gln Gly Ala Tyr
            660             665             670

Arg Thr Ala Val Asp Leu Glu Ser Leu Ala Ser Gln Leu Thr Ala Asp
        675             680             685

Leu Gln Glu Val Ser Gly Asp His Arg Leu Leu Val Phe His Ser Pro
```

690 695 700

Gly Glu Leu Val Val Glu Glu Ala Pro Pro Pro Pro Pro Ala Val Pro
705 710 715 720

Ser Pro Glu Glu Leu Thr Tyr Leu Ile Glu Ala Leu Phe Lys Thr Glu
725 730 735

Val Leu Pro Gly Gln Leu Gly Tyr Leu Arg Phe Asp Ala Met Ala Glu
740 745 750

Leu Glu Thr Val Lys Ala Val Gly Pro Gln Leu Val Arg Leu Val Trp
755 760 765

Gln Gln Leu Val Asp Thr Ala Ala Leu Val Ile Asp Leu Arg Tyr Asn
770 775 780

Pro Gly Ser Tyr Ser Thr Ala Ile Pro Leu Leu Cys Ser Tyr Phe Phe
785 790 795 800

Glu Ala Glu Pro Arg Gln His Leu Tyr Ser Val Phe Asp Arg Ala Thr
805 810 815

Ser Lys Val Thr Glu Val Trp Thr Leu Pro Gln Val Ala Gly Gln Arg
820 825 830

Tyr Gly Ser His Lys Asp Leu Tyr Ile Leu Met Ser His Thr Ser Gly
835 840 845

Ser Ala Ala Glu Ala Phe Ala His Thr Met Gln Asp Leu Gln Arg Ala
850 855 860

Thr Val Ile Gly Glu Pro Thr Ala Gly Gly Ala Leu Ser Val Gly Ile
865 870 875 880

Tyr Gln Val Gly Ser Ser Pro Leu Tyr Ala Ser Met Pro Thr Gln Met
885 890 895

Ala Met Ser Ala Thr Thr Gly Lys Ala Trp Asp Leu Ala Gly Val Glu
900 905 910

Pro Asp Ile Thr Val Pro Met Ser Glu Ala Leu Ser Ile Ala Gln Asp
915 920 925

Ile Val Ala Leu Arg Ala Lys Val Pro Thr Val Leu Gln Thr Ala Gly
930 935 940

Lys Leu Val Ala Asp Asn Tyr Ala Ser Ala Glu Leu Gly Ala Lys Met
945                     950                     955                     960

Ala Thr Lys Leu Ser Gly Leu Gln Ser Arg Tyr Ser Arg Val Thr Ser
                965                     970                     975

Glu Val Ala Leu Ala Glu Ile Leu Gly Ala Asp Leu Gln Met Leu Ser
                980                     985                     990

Gly Asp Pro His Leu Lys Ala Ala His Ile Pro Glu Asn Ala Lys Asp
                995                     1000                    1005

Arg Ile Pro Gly Ile Val Pro Met Gln Ile Pro Ser Pro Glu Val
        1010                    1015                    1020

Phe Glu Glu Leu Ile Lys Phe Ser Phe His Thr Asn Val Leu Glu
        1025                    1030                    1035

Asp Asn Ile Gly Tyr Leu Arg Phe Asp Met Phe Gly Asp Gly Glu
        1040                    1045                    1050

Leu Leu Thr Gln Val Ser Arg Leu Leu Val Glu His Ile Trp Lys
        1055                    1060                    1065

Lys Ile Met His Thr Asp Ala Met Ile Ile Asp Met Arg Phe Asn
        1070                    1075                    1080

Ile Gly Gly Pro Thr Ser Ser Ile Pro Ile Leu Cys Ser Tyr Phe
        1085                    1090                    1095

Phe Asp Glu Gly Pro Pro Val Leu Leu Asp Lys Ile Tyr Ser Arg
        1100                    1105                    1110

Pro Asp Asp Ser Val Ser Glu Leu Trp Thr His Ala Gln Val Val
        1115                    1120                    1125

Gly Glu Arg Tyr Gly Ser Lys Lys Ser Met Val Ile Leu Thr Ser
        1130                    1135                    1140

Ser Val Thr Ala Gly Thr Ala Glu Glu Phe Thr Tyr Ile Met Lys
        1145                    1150                    1155

Arg Leu Gly Arg Ala Leu Val Ile Gly Glu Val Thr Ser Gly Gly
        1160                    1165                    1170

```
Cys Gln Pro Pro Gln Thr Tyr  His Val Asp Asp Thr  Asn Leu Tyr
    1175             1180             1185

Leu Thr Ile Pro Thr Ala Arg  Ser Val Gly Ala Ser  Asp Gly Ser
    1190             1195             1200

Ser Trp Glu Gly Val Gly Val  Thr Pro His Val Val  Val Pro Ala
    1205             1210             1215

Glu Glu Ala Leu Ala Arg Ala  Lys Glu Met Leu Gln  His Asn Gln
    1220             1225             1230

Leu Arg Val Lys Arg Ser Pro  Gly Leu Gln Asp His  Leu
    1235             1240             1245
```

<210> 7
<211> 110
<212> PRT
<213> Homo sapiens

<400> 7

```
Met Ala Leu Trp Met Arg Leu Leu Pro Leu Leu Ala Leu Leu Ala Leu
1               5               10                  15

Trp Gly Pro Asp Pro Ala Ala Ala Phe Val Asn Gln His Leu Cys Gly
            20              25              30

Ser His Leu Val Glu Ala Leu Tyr Leu Val Cys Gly Glu Arg Gly Phe
        35              40              45

Phe Tyr Thr Pro Lys Thr Arg Arg Glu Ala Glu Asp Leu Gln Val Gly
    50              55              60

Gln Val Glu Leu Gly Gly Gly Pro Gly Ala Gly Ser Leu Gln Pro Leu
65              70              75              80

Ala Leu Glu Gly Ser Leu Gln Lys Arg Gly Ile Val Glu Gln Cys Cys
                85              90              95

Thr Ser Ile Cys Ser Leu Tyr Gln Leu Glu Asn Tyr Cys Asn
            100             105             110
```

<210> 8
<211> 142
<212> PRT
<213> Homo sapiens

<400> 8

```
Met Val Leu Ser Pro Ala Asp Lys Thr Asn Val Lys Ala Ala Trp Gly
1               5               10              15

Lys Val Gly Ala His Ala Gly Glu Tyr Gly Ala Glu Ala Leu Glu Arg
            20              25              30

Met Phe Leu Ser Phe Pro Thr Thr Lys Thr Tyr Phe Pro His Phe Asp
        35              40              45

Leu Ser His Gly Ser Ala Gln Val Lys Gly His Gly Lys Lys Val Ala
    50              55              60

Asp Ala Leu Thr Asn Ala Val Ala His Val Asp Asp Met Pro Asn Ala
65              70              75              80

Leu Ser Ala Leu Ser Asp Leu His Ala His Lys Leu Arg Val Asp Pro
            85              90              95

Val Asn Phe Lys Leu Leu Ser His Cys Leu Leu Val Thr Leu Ala Ala
        100             105             110

His Leu Pro Ala Glu Phe Thr Pro Ala Val His Ala Ser Leu Asp Lys
    115             120             125

Phe Leu Ala Ser Val Ser Thr Val Leu Thr Ser Lys Tyr Arg
    130             135             140
```

<210> 9
<211> 147
<212> PRT
<213> Homo sapiens

<400> 9

```
Met Val His Leu Thr Pro Glu Glu Lys Ser Ala Val Thr Ala Leu Trp
1               5               10              15

Gly Lys Val Asn Val Asp Glu Val Gly Gly Glu Ala Leu Gly Arg Leu
            20              25              30

Leu Val Val Tyr Pro Trp Thr Gln Arg Phe Phe Glu Ser Phe Gly Asp
        35              40              45

Leu Ser Thr Pro Asp Ala Val Met Gly Asn Pro Lys Val Lys Ala His
    50              55              60
```

53

```
Gly Lys Lys Val Leu Gly Ala Phe Ser Asp Gly Leu Ala His Leu Asp
65              70              75              80

Asn Leu Lys Gly Thr Phe Ala Thr Leu Ser Glu Leu His Cys Asp Lys
            85              90              95

Leu His Val Asp Pro Glu Asn Phe Arg Leu Leu Gly Asn Val Leu Val
            100             105             110

Cys Val Leu Ala His His Phe Gly Lys Glu Phe Thr Pro Pro Val Gln
        115             120             125

Ala Ala Tyr Gln Lys Val Val Ala Gly Val Ala Asn Ala Leu Ala His
    130             135             140

Lys Tyr His
145
```

<210> 10
<211> 16
<212> PRT
<213> séquence artificielle

<220>
<221> MISC_FEATURE
<222> (9)..(9)
<223> tyrosine nitrée (Tyr-NO2)

<400> 10

```
Cys Glu Glu Thr Phe Leu Lys Lys Xaa Leu Tyr Glu Ile Ala Arg Arg
1               5               10              15
```

<210> 11
<211> 9
<212> PRT
<213> séquence artificielle

<220>
<221> MISC_FEATURE
<222> (4)..(4)
<223> tyrosine nitrée (Tyr-NO2)

<400> 11

```
Leu Val Arg Xaa Thr Lys Lys Val Cys
1               5
```